(19) Europäisches Patentamt
European Patent Office
Office européen des brevets

(11) EP 1 789 398 B1

(12) **EUROPEAN PATENT SPECIFICATION**

(45) Date of publication and mention of the grant of the patent:
**29.09.2010 Bulletin 2010/39**

(21) Application number: **05780525.1**

(22) Date of filing: **08.08.2005**

(51) Int Cl.:
***C07D 239/84*** *(2006.01)* ***A61K 31/517*** *(2006.01)*
***A61P 25/28*** *(2006.01)*

(86) International application number:
**PCT/IB2005/002595**

(87) International publication number:
**WO 2006/024932 (09.03.2006 Gazette 2006/10)**

(54) **2-AMINO-QUINAZOLINE DERIVATIVES USEFUL AS INHIBITORS OF B-SECRETASE (BACE)**

ALS B-SEKRETASE (BACE)-HEMMER NÜTZLICHE 2-AMINO-CHINAZOLINDERIVATE

DERIVES DE 2-AMINO-QUINAZOLINE UTILES EN TANT QU'INHIBITEURS DE B-SECRETASE (BACE)

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(30) Priority: **06.08.2004 US 599810 P**

(43) Date of publication of application:
**30.05.2007 Bulletin 2007/22**

(73) Proprietor: **Janssen Pharmaceutica NV**
**2340 Beerse (BE)**

(72) Inventors:
- **BISHOFF, François, Paul**
  **B-2350 Vopsselar (BE)**
- **BRAEKEN, Mirielle**
  **B-2340 Vimmeren (BE)**
- **PIETERS, Serge, Maria, Aloysius**
  **NL-4561 LL Jost (NL)**
- **MERCKEN, Marc, Hubert**
  **B-2300 Turnhout (BE)**
- **DE WINTER, Hans, Louis, Jos**
  **BE-2970 Schilde (BE)**
- **BERTHELOT, Dieder, Jean-Claude**
  **B-2000 Antwerp (BE)**

(74) Representative: **Williams, Paul Edwin et al**
**Ablett & Stebbing**
**Caparo House**
**101-103 Baker Street**
**London W1U 6FQ (GB)**

(56) References cited:
**WO-A-2004/022523**

- **VENUTI M C ET AL: "INHIBITORS OF CYCLIC AMP PHOSPHODIESTERASE. 2. STRUCTURAL VARIATIONS OF N-CYCLOHEXYL-N-METHYL-4-(1,2,3,5-TETRAHYDR O-2-OXOIMIDAZO2,1-B QUINAZOLIN-7-YL)-OXYBUTYRAMIDE (RS-82856)" JOURNAL OF MEDICINAL CHEMISTRY, AMERICAN CHEMICAL SOCIETY. WASHINGTON, US, vol. 30, no. 2, 1987, pages 303-318, XP000941985 ISSN: 0022-2623**
- **PATENT ABSTRACTS OF JAPAN vol. 016, no. 160 (P-1340), 20 April 1992 (1992-04-20) -& JP 04 011255 A (FUJI PHOTO FILM CO LTD), 16 January 1992 (1992-01-16)**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 789 398 B1

## Description

The present invention is directed to novel 2-amino-3,4-dihydroquinazoline derivatives, pharmaceutical compositions containing them and their use in the treatment of Alzheimer's disease (AD), mild cognitive impairment, senility and / or dementia. The compounds of the present invention are inhibitors of β-secretase, also known as β-site amyloid cleaving enzyme, BACE, BACE1, Asp2, or memapsin2.

## BACKGROUND OF THE INVENTION

Alzheimer's Disease (AD) is a neurodegenerative disease associated with aging. AD patients suffer from cognition deficits and memory loss as well as behavioral problems such as anxiety. Over 90% of those afflicted with AD have a sporadic form of the disorder while less than 10% of the cases are familial or hereditary. In the United States, about 1 in 10 people at age 65 have AD while at age 85, 1 out of every two individuals are affected with AD. The average life expectancy from the initial diagnosis is 7-10 years, and AD patients require extensive care either in an assisted living facility which is very costly or by family members. With the increasing number of elderly in the population, AD is a growing medical concern. Currently available therapies for AD merely treat the symptoms of the disease and include acetylcholinesterase inhibitors to improve cognitive properties as well as anxiolytics and antipsychotics to control the behavioral problems associated with this ailment.

The hallmark pathological features in the brain of AD patients are neurofibillary tangles which are generated by hyperphosphorylation of tau protein and amyloid plaques which form by aggregation of β-arnyloid$_{1-42}$ (A$\beta_{1-42}$) peptide. A$\beta_{1-42}$ forms oligomers and then fibrils, and ultimately amyloid plaques. The fibrils are believed to be especially neurotoxic and may cause most of the neurological damage associated with AD. Agents that prevent the formation of A$\beta_{1-42}$ have the potential to be disease-modifying agents for the treatment of AD. A$\beta_{1-42}$ is generated from the amyloid precursor protein (APP), comprised of 770 amino acids. The N-terminus of A$\beta_{1-42}$ is cleaved by β-secretase (BACE), and then γ-secretase cleaves the C-terminal end. In addition to A$\beta_{1-42,}$ γ-secretase also liberates A$\beta_{1-40}$ which is the predominant cleavage product as along with A$\beta_{1-38}$ and A$\beta_{1-43.}$ Thus, inhibitors of BACE would be expected to prevent the formation of A$\beta_{1-42}$ and would be potential therapeutic agents in the treatment of AD.

Beta-secretase inhibitors are known from WO 2004/22523.

## SUMMARY OF THE INVENTION

The present Invention is directed to compounds of formula (III)

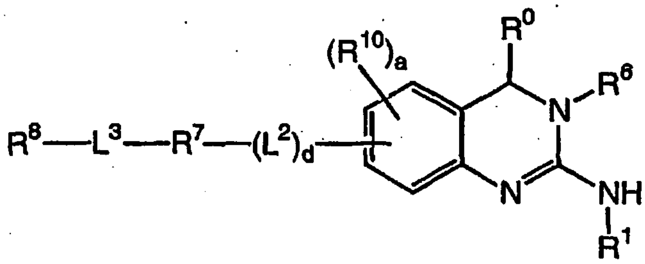

(III)

wherein

$R^0$ is selected from the group consisting of hydrogen, methyl and $CF_3$;
$R^1$ Is selected from the group consisting of hydrogen, hydroxy, methyl, ethyl, trifluoromethyl, 2,2,2-trifluoroethyl, methoxy, ethoxy and methyl-carbonyl;
$R^6$ is selected from the group consisting of $C_{1-6}$alkyl and hydroxy substituted $C_{1-8}$alkyl;
d is an integer from 0 to 1;
$L^2$ is selected from the group consisting of -O-, -S(O)$_{0-2}$-, -NR$^Q$-;
wherein $R^Q$ is selected from the group consisting of hydrogen and $C_{1-4}$alkyl;
$R^7$ is selected from the group consisting of cycloalkyl, cycloalkyl-$C_{1-4}$alkyl, aryl, $C_{1-4}$alkyl-aryl, aryl-$C_{1-4}$alkyl, partially unsaturated carbocyclyl, partially unsaturated carbocyclyl-$C_{1-4}$alkyl, heteroaryl, heteroaryl-$C_{1-4}$alkyl, heterocycloalkyl and heterocycloalkyl-$C_{1-4}$alkyl;
wherein the aryl, cycloalkyl, partially unsaturated carbocyclyl, heteroaryl or heterocycloalkyl group, whether alone or as part of a substituent group, is optionally substituted with one or more substituents independently selected from

the group consisting of $C_{1-4}$alkyl, $C_{1-4}$alkoxy, halogen substituted $C_{1-4}$alkyl, halogen substituted $C_{1-4}$alkoxy, hydroxy substituted $C_{1-4}$alkyl, hydroxy, carboxy, cyano, nitro, amino, $C_{1-4}$alkylamino, di($C_{1-4}$alkyl)amino, 5-tetrazolyl and 1-(1,4-dihydro-tetrazol-5-orie);

$L^3$ is selected from the group consisting of -O-, -S-, -C(O)-, -C(S)-,-C(O)O-, -OC(O)-, -$NR^A$-, -N(CN)-, -$NR^A$-C(O)-, -C(O)-$NR^A$-, -$NR^A$-C(S)-, -C(S)-$NR^A$-, -$NR^A$-$SO_2$-, -$SO_2$-$NR^A$-, -$NR^A$-SO-, -SO-$NR^A$, -$NR^A$-C(O)O-, -OC(O)-$NR^A$-, -O-$SO_2$-$NR^A$-, -$NR^A$-$SO_2$-O-, -$NR^A$-C(O)-$NR^B$-, -$NR^A$-C(S)-$NR^B$- and -$NR^A$-$SO_2$-$NR^B$-;

wherein each $R^A$ and $R^B$ is independently selected from the group consisting of hydrogen, $C_{1-8}$alkyl, hydroxy substituted $c_{1-4}$alkyl, $C_{1-4}$aralkyloxy substituted $C_{1-4}$alkyl, cycloalkyl, aryl, heteroaryl, heterocycloalkyl, cycloalkyl-$C_{1-4}$alkyl-, $C_{1-4}$aralkyl, heteroaryl-$C_{1-4}$alkyl-, heterocycloalkyl-$C_{1-4}$alkyl- and spiro-heterocyclyl;

wherein the cycloalkyl, aryl, heteroaryl, heterocycloalkyl or spiroheterocyclyl, whether alone or as part of a substituent group is optionally substituted with one or more substituents independently selected from the group consisting of halogen, hydroxy, oxo, carboxy, $C_{1-4}$alkyl, $C_{1-4}$alkoxy, $C_{1-4}$alkoxy-carbonyl, nitro, cyano, amino, $C_{1-4}$alkylamino, di ($C_{1-4}$alkyl)amino, -$SO_2$-N($R^CR^D$), 5-tetrazolyl and 1-(1,4-dihydro-tetrazol-5-one);

wherein each $R^C$ and $R^D$ is independently selected from the group consisting of hydrogen and $C_{1-4}$alkyl;

$R^8$ is selected from the group consisting of $C_{1-10}$alkyl, cycloalkyl, aryl, biphenyl, partially unsaturated carbocyclyl, heteroaryl, heterocycloalkyl, cycloalkyl-$C_{1-4}$alkyl-, $C_{1-4}$aralkyl, partially unsaturated carbocyclyl-$C_{1-4}$alkyl-, heteroaryl-$C_{1-4}$alkyl-, heterocycloalkyl-$C_{1-4}$alkyl- and spiro-heterocyclyl;

wherein the $C_{1-10}$alkyl, cycloalkyl, aryl, partially unsaturated carbocyclyl, heteroaryl, heterocycloalkyl or spiro-heterocyclyl, whether alone or as part of a substituent group is optionally substituted with one or more substituents independently selected from the group consisting of halogen, hydroxy, oxo, carboxy, -C(O)-$C_{1-4}$alkyl, -C (O)-$C_{1-4}$aralkyl, -C(O)O-$C_{1-4}$alkyl, -C(O)O-$C_{1-4}$aralkyl, -$C_{1-4}$alkyl-C(O)O-$C_{1-4}$alkyl, -$C_{1-4}$alkyl-S-$C_{1-4}$alkyl, -C(O)-N ($R^LR^M$), -$C_{1-4}$alkyl-C(O)-N($R^LR^M$), -$NR^L$-C(O)-$C_{1-4}$alkyl, -$SO_2$-$C_{1-4}$alkyl, -$SO_2$-aryl, -$SO_2$-N($R^LR^M$), -$C_{1-4}$alkyl-$SO_2$-N($R^LR^M$), $C_{1-4}$alkyl, fluoro substituted $C_{1-4}$alkyl, hydroxy substituted $C_{1-4}$alkyl, carboxy substituted $C_{1-4}$a)ky), $C_{1-4}$alkoxy, fluoro substituted $C_{1-4}$alkoxy, nitro, cyano, amino, $C_{1-4}$alkylamino, di($C_{1-4}$alkyl)amino, phenyl and heteroaryl;

wherein the phenyl or heteroaryl is optionally substituted with one or more substituent independently selected from the group consisting of halogen, hydroxy, oxo, carboxy, -C(O)O-$C_{1-4}$alkyl, -C(O)-$C_{1-4}$alkyl, $C_{1-4}$alkyl, fluoro substituted $C_{1-4}$alkyl, $C_{1-4}$alkoxy, nitro, cyano, amino, $C_{1-4}$alkylamino and di($C_{1-4}$alkyl)amino;

wherein each $R^L$ and $R^M$ is independently selected from the group consisting of hydrogen, $C_{1-4}$alkyl and cycloalkyl;

a is an integer from 0 to 3;

each $R^{10}$ is independently selected from the group consisting of hydroxy, halogen, $C_{1-4}$alkyl, $C_{1-4}$alkoxy, halogen substituted $C_{1-4}$alkyl, halogen substituted $C_{1-4}$alkoxy, -C(O)-$NR^VR^W$, -$SO_2$-$NR^VR^W$, -C(O)-$C_{1-4}$alkyl and -$SO_2$-$C_{1-4}$alkyl;

wherein each $R^V$ and $R^W$ is independently selected from the group consisting of hydrogen and $C_{1-4}$alkyl; alternatively $R^V$ and $R^W$ are taken together with the N atom to which they are bound to form a 5 to 6 membered saturated, partially unsaturated or aromatic ring structure;

provided that the halogens on the halogen substituted $C_{1-4}$alkyl and the halogen substituted $C_{1-4}$alkoxy are selected from the group consisting of chloro and fluoro;
or a pharmaceutically acceptable salt thereof.

Optionally the compounds of formula (III) may be chosen wherein:

$R^0$ is selected from the group consisting of hydrogen; methyl and $CF_3$;

$R^1$ is selected from the group consisting of hydrogen, hydroxy, methyl, trifluoromethyl, methoxy and methyl-carbonyl;

$R^6$ is selected from the group consisting of $C_{1-6}$alkyl and hydroxy substituted $C_{1-6}$alkyl;

d is an integer selected from 0 to 1;

$L^2$ is selected from the group consisting of -O-, -$S(O)_{0-2}$- and -NH-;

$R^7$ is selected from the group consisting of cycloalkyl, cycloalkyl-$C_{1-4}$alkyl, aryl, $C_{1-4}$alkyl-aryl, aryl-$C_{1-4}$alkyl, heteroaryl, heteroaryl-$C_{1-4}$alkyl, heterocycloalkyl and heterocycloalkyl-$C_{1-4}$alkyl;

wherein the aryl, cycloalkyl, partially unsaturated carbocyclyl, heteroaryl or heterocycloalkyl group, whether alone or as part of a substituent group, is optionally substituted with one to two substituents independently selected from the group consisting of $C_{1-4}$alkyl, $C_{1-4}$alkoxy, halogen substituted $C_{1-4}$alkyl, halogen substituted $C_{1-4}$alkoxy, hydroxy substituted $C_{1-4}$alkyl, hydroxy, carboxy, cyano, nitro, amino, $C_{1-4}$alkylamino and dl($C_{1-4}$alkyl)amino;

$L^3$ is selected from the group consisting of -C(O)-, -C(O)O-, -OC(O)-,-$NR^A$-, -N(CN)-, -$NR^A$-C(O)-, -C(O)-$NR^A$-; -$NR^A$-$SO_2$-, -$SO_2$-$NR^A$-, -$NR^A$-C(O)O-and -OC(O)-$NR^A$;

wherein $R^A$ is selected from the group consisting of hydrogen, $C_{1-4}$alkyl, hydroxy substituted $C_{1-4}$alkyl, $C_{1-4}$aralkyloxy substituted $C_{1-4}$alkyl, cycloalkyl, aryl, heteroaryl, heterocycloalkyl, cycloalkyl-$C_{1-4}$alkyf-, $C_{1-4}$aralkyl, heteroaryl-$C_{1-4}$alkyl- and heterocycloalkyl-$C_{1-4}$alkyl-;

wherein the cycloalkyl, aryl, heteroaryl or heterocycloalkyl, whether alone or as part of a substituent group is optionally substituted with one to two substituents independently selected from the group consisting of halogen, hydroxy, oxo, carboxy, $C_{1-4}$alkyl, $C_{1-4}$alkoxy, $C_{1-4}$alkoxy-carbonyl, nitro, cyano, amino, $C_{1-4}$alkylamino and di($C_{1-4}$alkyl)amino;

$R^8$ is selected from the group consisting of $C_{1-6}$alkyl, cycloalkyl, aryl; heteroaryl, heterocycloalkyl, cycloalkyl-$C_{1-4}$alkyl-, $C_{1-4}$aralkyl, heteroaryl-$C_{1-4}$alkyl- and heterocycloalkyl-$C_{1-4}$alkyl-;

whereon the $C_{1-6}$alkyl, cycloalkyl, aryl, heteroaryl or heterocycloalkyl; whether alone or as part of a substituent group is optionally substituted with one to two substituents independently selected from the group consisting of halogen, hydroxy, carboxy, -C(O)-$C_{1-4}$alkyl, -C(O)-$C_{1-4}$aralkyl, -C(O)O-$C_{1-4}$alkyl, -C(O)O-$C_{1-4}$aralkyl, -C(O)-N($R^LR^M$), -$NR^L$-C(O)-$C_{1-4}$alkyl, -$SO_2$-$C_{1-4}$alkyl, -$SO_2$-aryl, -$SO_2$-N($R^LR^M$), $C_{1-4}$alkyl, fluoro substituted $C_{1-4}$alkyl, hydroxy substituted $C_{1-4}$alkyl, carboxy substituted $C_{1-4}$alkyl, $C_{1-4}$alkoxy, fluoro substituted $C_{1-4}$alkoxy, nitro, cyano, amino, $C_{1-4}$alkylamlno, di($C_{1-4}$alkyl)amino, phenyl and heteroaryl;

wherein the phenyl or heteroaryl is optionally substituted with one or more substituent independently selected from the group consisting of halogen, hydroxy, carboxy, $C_{1-4}$alkyl, fluoro substituted $C_{1-4}$alkyl, $C_{1-4}$alkoxy, nitro, cyano, amino, $C_{1-4}$alkylamlno and dl($C_{1-4}$alkyl)amino;

wherein each $R^L$ and $R^M$ is independently selected from the group consisting of hydrogen, $C_{1-4}$alkyl and $C_{5-8}$cycloalkyl;

a is an integer from 0 to 1;

$R^{10}$ is selected from the group consisting of hydroxy, halogen, $C_{1-4}$alkyl, $C_{1-4}$alkoxy, halogen substituted $C_{1-4}$alkyl and halogen substituted $C_{1-4}$alkoxy;

provided that the halogens on the halogen substituted $C_{1-4}$alkyl or the halogen substituted $C_{1-4}$alkoxy are selected from the group consisting of chloro and fluoro;
or a pharmaceutically acceptable salt thereof.

Optionally the compounds of formula (III) may be chosen wherein

$R^0$ is hydrogen;

$R^1$ is hydrogen;

$R^6$ is selected from the group consisting of $C_{1-6}$alkyl and hydroxy substituted $C_{1-6}$alkyl;

d is an integer selected from 0 to 1;

$L^2$ is selected from the group consisting of -0-, -S-, -SO- and -SO-;

$R^7$ is selected from the group consisting of aryl, $C_{1-4}$alkyl-aryl and aryl-$C_{1-4}$alkyl;

$L^3$ is selected from the group consisting of -NH-, -N(CN)-, -N($C_{1-4}$alkyl)-, -NH-C(O)-; -C(O)-NH-, -NH-$SO_2$-, -N($C_{1-4}$alkyl)-C(O)O- and -N(cycloalkyl)-C(O)O-;

$R^8$ is selected from the group consisting of $C_{1-4}$alkyl, cycloalkyl, cycloalkyl-$C_{1-4}$alkyl, aryl, $C_{1-4}$aralkyl, heteroaryl and heterocycloalkyl;

wherein the aryl or heteroaryl, whether alone or as part of a substituent group is optionally substituted with one to three substituents independently selected from the group consisting of halogen, hydroxy, carboxy, $C_{1-4}$alkyl, $C_{1-4}$alkoxy, fluoro substituted $C_{1-4}$alkyl, fluoro substituted$C_{1-4}$alkoxy, cyano, amino, $C_{1-4}$alkylamino, di($C_{1-4}$alkyl)amino, -C(O)O-$C_{1-4}$alkyl, -C(O)-N($R^LR^M$)-$SO_2$-$C_{1-4}$alkyl, -$SO_2$-aryl, -NH-C(O)-$C_{1-4}$alkyl, phenyl and heteroaryl;

wherein the phenyl or heteroaryl is optionally substituted with a substituent selected from the group consisting of fluoro substituted $C_{1-4}$alkyl;

wherein each $R^L$ and $R^M$ is independently selected from the group consisting of hydrogen, $C_{1-4}$alkyl and $C_{5-6}$cycloalkyl;

a is 0;

or a pharmaceutically acceptable salt thereof.

$R^0$ is hydrogen;

$R^1$ is hydrogen;

$R^6$ is selected from the group consisting of n-propyl, 4-hydroxy-n-butyl and 5-hydroxy-n-pentyl;

d is an integer from 0 to 1;

$L^2$ is selected from the group consisting of -O-, -S- and -SO-;

$R^7$ is selected from the group consisting of phenyl, -$CH_2$-phenyl-, phenyl-3-$CH_2$- and -phenyl-2-$CH_2$-$CH_2$-;

$L^3$ is selected from the group consisting of -NH-, -N(CN)-, -N($CH_3$)-, - NH-C(O)-, -C(O)-NH-, -NH-$SO_2$- and -N(cyclohexyl)-C(O)O-;

wherein the $L^3$ group is bound at the 3-position of the $R^7$ group;

$R^8$ is selected from the group consisting of methyl, isopropyl, n-butyl, cyclohexyl, cyclohexyl-methyl, phenyl, phenyl-ethyl, phenyl-n-propyl, 3-(N-methyl-N-cyclohexyl-amino-carbonyl)-n-propyl, 3-bromo-phenyl, 3-methoxy-phenyl, 4-methomy-phenyl, 2-methoxy-4-methyl-phenyl, 2,4,6-trimethyl-phenyl, 2,5-dimethoxy-phenyl, 3,4-dimethoxy-phenyl, 3-trifluoromethoxy-phenyl, 4-trifluoromethoxy-phenyl, 3-cyano-phenyl, 2-(methyl-sulfonyl)-phenyl, 4-(methyl-carbonyl-amino)-phenyl, 5-carboxy-2-methoxy-phenyl, benzyl, 3-hydroxy-benzyl, 4-methyl-benzyl, 2-methoxy-benzyl, 4-methoxy-benzyl, 2,6-dimethoxy-benzyl, 2,4,6-trimethyl-benzyl, 1-nahthyl, 2-naphthyl, 1-naphthyl-methyl, 1-(5-dimethylamino)-naphthyl, 4-biphenyl, 2-thienyl, 3-thienyl, 4-(3,5-dimethyl-isoxazolyl), 3-benzothienyl, 4-benzo[2,3,1]thiadiazolyl, 2-(5-(2-pyridyl)-thienyl), 2-(5-(3-(2-methyl-thiazolyl)-thienyl)), 2-(5-(3-(5-trifluoromethyl)-isoxazolyl)-thienyl), 6-(2,3-dihydro-benzo[1,4]dioxanyl), 3-(2-methoxy-carbonyl)-thienyl, 2-(5-(5-isoxazolyl)-thienyl)), 2-(5-bromo-thienyl) and 2-(4-phenyl-sulfonyl)-thionyl;
a is 0;

or a pharmaceutically acceptable salt thereof.

Optionally the compounds of formula (III) may be chosen wherein

$R^0$ is hydrogen;
$R^1$ is hydrogen;
$R^6$ is selected from the group consisting of n-propyl, 4-hydroxy-n-butyl and 5-hydroxy-n-pentyl;
d is an integer from 0 to 1;
$L^2$ is selected from the group consisting of -O- and -S-;
$R^7$ is selected from the group consisting of -phenyl-, -phenyl-3-$CH_2$- and phenyl-2-$CH_2$-$CH_2$-;
$L^3$ is selected from the group consisting of -NH-, -N(CN)-, -NH-C(O)-,-NH-C(O)O-, -N(cyclohexyl)-C(O)P- and -NH-$SO_2$-; wherein the $L^3$ is bound to the $R^7$ phenyl at the 3-position;
$R^8$ is selected from the group consisting of n-butyl, 3-(N-methyl-N-cyclohexyl-amino-carbonyl)-n-propyl, cyclohexyl, cyclohexyl-methyl-, phenyl, 2-trifluoromethylphenyl, 3-trifluoromethoxyphenyl, 3-cyanophenyl, 3-methoxyphenyl, 4-methoxyphenyl, 2-methoxy-4-methylphenyl, 2,4,6-trimethylphenyl, benzyl, 2-methoxybenzyl, 4-methoxbenzyl, 2,4,6-trimethylbenzyl, phenylethyl-, 2-thienyl, 3-thienyl, 2-(5-bromo-thienyl) and 3-benzothienyl;
a is 0;

or a pharmaceutically acceptable salt thereof.

Optionally the compounds of formula (III) may be chosen wherein

$R^0$ is hydrogen;
$R^1$ is hydrogen;
$R^6$ is selected from the group consisting of n-propyl and 4-hydroxy-n-butyl;
d is 1;
$L^2$ is selected from the group consisting of -0- and -S-;
$R^7$ is selected from the group consisting of -phenyl- and -phenyl-3-$CH_2$-;
$L^3$ is selected from the group consisting of NH-, -NH-C(O)- and -NH-$SO_2$-; wherein the $L^3$ is bound to the $R^7$ phenyl at the 3-position;
$R^8$ is selected from the group consisting of 3-(N-methyl-N-cyclohexyl-amino-carbonyl)-n-propyl, phenyl, 2-methoxy-4-methylphenyl, 2,4,6-trimethylphenyl, benzyl, 2-methoxybenzyl, 4-methoxybenzyl, 2,4,6-trimethylbenzyl and 3-benzothienyl;
a Is 0;

or a pharmaceutically acceptable salt thereof.

$R^7$ is selected from the group consisting of cycloalkyl, cycloalkyl-$C_{1-4}$alkyl, aryl, $C_{1-4}$alkyl-aryl, aryl-$C_{1-4}$alkyl, partially unsaturated carbocyclyl, partially unsaturated carbocyclyl-$C_{1-4}$alkyl, heteroaryl, heteroaryl-$C_{1-4}$alkyl, heterocycloalkyl and heterocycloalkyl-$C_{1-4}$alkyl;
wherein the aryl, cycloalkyl, partially unsaturated carbocyclyl, heteroaryl or heterocycloalkyl group, whether alone or as part of a substituent group, is optionally substituted with one or more substituents independently selected from the group consisting of $C_{1-4}$alkyl, $C_{1-4}$alkoxy, halogen substituted $C_{1-4}$alkyl, halogen substituted $C_{1-4}$alkoxy, hydroxy substituted $C_{1-4}$alkyl, hydroxy, carboxy, cyano, nitro, amino, $C_{1-4}$alkylamino, di($C_{1-4}$alkyl)amino, 5-tetrazolyl and 1-(1,4-dihydro-tetrazol-5-one);
$L^3$ is selected from the group consisting of -O-, -S-, -C(O)-, -C(S)-, - C(O)O-, -OC(O)-, -$NR^A$-, -N(CN)-, -$NR^A$-C(O)-, -C(O)-$NR^A$-, -$NR^A$-C(S)-, -C(S)-$NF^A$-, -$NR^A$-$SO_2$-, -$SO_2$-$NR^A$-, -$NR^A$-SO-,-SO-$NR^A$, -$NR^A$-C(O)O-, -OC(O)-$NR^A$--

$O-SO_2-NR^A-$, $-NR^A-SO_2-O-$, $-NR^A-C(O)-NR^B-$, $-NR^A-C(S)-NR^B-$ and $-NR^A-SO_2-NR^B-$;

wherein each $R^A$ and $R^B$ is independently selected from the group consisting of hydrogen, $C_{1-8}$alkyl, hydroxy substituted $C_{1-4}$alkyl, $C_{1-4}$aralkyloxy substituted $C_{1-4}$alkyl, cycloalkyl, aryl, heteroaryl, heterocycloalkyl, cycloalkyl-$C_{1-4}$alkyl-, $C_{1-4}$aralkyl, heteroaryl-$C_{1-4}$alkyl-, heterocycloalkyl-$C_{1-4}$alkyl- and spiro-heterocyclyl;

wherein the cycloalkyl, aryl, heteroaryl, heterocycloalkyl or spiroheterocyclyl, whether alone or as part of a substituent group is optionally substituted with one or more substituents independently selected from the group consisting of halogen, hydroxy, oxo, carboxy; $C_{1-4}$alkyl, $C_{1-4}$alkoxy, $C_{1-4}$alkoxy-carbonyl, nitro, cyano, amino, $C_{1-4}$aikylamino, di($C_{1-4}$alkyl)amino,-$SO_2$-N($R^CR^D$), 5-tetrazolyl and 1-(1,4-dihydro-tetrazol-5-one)

wherein each $R^C$ and $R^D$ is independently selected from the group consisting of hydrogen and $C_{1-4}$alkyl;

$R^8$ is selected from the group consisting of $C_{1-10}$alkyl, cycloalkyl, aryl, biphenyl, partially unsaturated carbocyclyl, heteroaryl, heterocycloalkyl, cycloalkyl-$C_{1-4}$alkyl-, $C_{1-4}$aralkyl, partially unsaturated carbocyclyl-$C_{1-4}$alkyl-, heteroaryl-$C_{1-4}$alkyl-, heterocycloalkyl-$C_{1-4}$alkyl- and spiro-heterocyclyl;

wherein the $C_{1-10}$alkyl, cycloalkyl, aryl, partially unsaturated carbocyclyl, heteroaryl, heterocycloalkyl or spiro-heterocyclyl, whether alone or as part of a substituent group is optionally substituted with one or more substituents independently selected from the group consisting of halogen, hydroxy, oxo, carboxy, -C(O)-$C_{1-4}$alkyl, -C(O)-$C_{1-4}$aralkyl, -C(O)O-$C_{1-4}$alkyl, -C(O)O-$C_{1-4}$aralkyl, -$C_{1-4}$alkyl-C(O)O-$C_{1-4}$alkyl, -$C_{1-4}$alkyl-S-$C_{1-4}$alkyl, -C(O)-N($R^LR^M$), -$C_{1-4}$alkyl-C(O)-N($R^LR^M$), -$NR^L$-C(O)-$C_{1-4}$alkyl, -$SO_2$-$C_{1-4}$alkyl, -$SO_2$-aryl, -$SO_2$-N($R^LR^M$), -$C_{1-4}$alkyl-$SO_2$-N($R^LR^M$), $C_{1-4}$alkyl, fluoro substituted $C_{1-4}$alkyl, hydroxy substituted $C_{1-4}$alkyl, carboxy substituted $C_{1-4}$alkyl, $C_{1-4}$alkoxy, fluoro substituted $C_{1-4}$alkoxy, nitro, cyano, amino, $C_{1-4}$alkylamino, di($C_{1-4}$alkyl)amino, phenyl and heteroaryl;

wherein the phenyl or heteroaryl is optionally substituted with one or more substituent independently selected from the group consisting of halogen, hydroxy, oxo, carboxy, C(O)O-$C_{1-4}$alkyl, C(O)-$C_{1-4}$alkyl, $C_{1-4}$alkyl, fluoro substituted $C_{1-4}$alkyl, $C_{1-4}$alkoxy, nitro, cyano, amino, $C_{1-4}$alkylamino and di($C_{1-4}$alkyl)amino;

wherein each $R^L$ and $R^M$ is independently selected from the group consisting of hydrogen and $C_{1-4}$alkyl;

a is an integer from 0 to 3;

each $R^{10}$ Is independently selected from the group consisting of hydroxy, halogen, $C_{1-4}$alkyl, $C_{1-4}$alkoxy, halogen substituted $C_{1-4}$alkyl, halogen substituted $C_{1-4}$alkoxy, -C(O)-$NR^VR^W$; -$SO_2$-$NR^VR^W$, -C(O)-$C_{1-4}$alkyl and -$SO_2$-$C_{1-4}$alkyl;

wherein each $R^V$ and $R^W$ is independently selected from the group consisting of hydrogen and $C_{1-4}$alkyl; alternatively $R^V$ and $R^W$ are taken together with the N atom to which they are bound to form a 5 to 6 membered saturated, partially unsaturated or aromatic ring structure;

provided that the halogens on the halogen substituted $C_{1-4}$alkyl or the halogen substituted $C_{1-4}$alkoxy are selected from the group consisting of chloro and fluoro;
or a pharmaceutically acceptable salt thereof.

Optionally the compounds of formula (III) may be chosen wherein
$R^0$ is hydrogen; $R^1$ is hydrogen; $R^6$ is n-propyl; d is 1; $L^2$ is selected from the group consisting of -0- and -S-; $R^7$ is -phenyl-; $L^3$ is -NH-$SO_2$-; wherein the $L^3$ is bound to the $R^7$ phenyl at the 3-position; $R^8$ is 2,4,6-trimethylphenyl; a is 0; or a pharmaceutically acceptable salt thereof.

Optionally the compounds of formula (III) may be chosen wherein
$R^0$ is hydrogen; $R^1$ is hydrogen; $R^6$ is n-propyl; d is 1; $L^2$ is -O-; $R^7$ is phenyl; $L^3$ is selected from the group consisting of -NH-C(O)O-, -N(cyclohexyl)-C(O)O-, -NH-, -N(CN)- and -NH-$SO_2$; wherein the $L^3$ is bound to $R^7$ phenyl at the 3-position; $R^8$ is selected from the group consisting of 3-(N-methyl-N-cyclohexyl-amino-carbonyl)-n-propyl, cyclohexyl, cyclohexyl-methyl-, phenyl and benzyl; a is 0; or a pharmaceutically acceptable salt thereof.

A further aspect of the present invention concerns a pharmaceutical composition comprising a pharmaceutically acceptable carrier and a compound of the present invention.

Optionally the pharmaceutical composition made by mixing a compound of the present invention and a pharmaceutically acceptable carrier.

A further aspect of the present invention concerns a process for making a pharmaceutical composition comprising mixing a compound of the present invention and a pharmaceutically acceptable carrier.

A further aspect of the invention concerns a compound of the present invention for treating a disorder mediated by β-secretase by administering to a subject in need thereof a therapeutically effective amount of the compound.

Optionally the disorder mediated by β-secretase may be selected from the group consisting of Alzheimer's disease (AD), mild cognitive impairment, senility, dementia, dementia with Lewy bodies, Down's syndrome, dementia associated with Parkinson's disease and dementia associated with beta-amyloid.

Optionally a composition of the present invention may be for treating a disorder mediated by β-secretase by administering to a subject in need thereof a therapeutically effective amount of the composition.

Optionally a compound of the present invention may be for treating a condition selected from the group consisting

of Alzheimer's disease (AD), mild cognitive impairment, senility, dementia, dementia with Lewy bodies, Down's syndrome, dementia associated with Parkinson's disease and dementia associated with beta-amyloid by administering to a subject in need thereof a therapeutically effective amount of the compound.

A further aspect of the present invention concerns the use of a compound of the present invention for the preparation of a medicament for treating: (a) Alzheimer's disease (AD), (b) mild cognitive impairment, (c) senility, (d) dementia, (e) dementia with Lewy bodies, (f) Down's syndrome, (g) dementia associated with Parkinson's disease or (h) dementia associated with beta-amyloid, in a subject in need thereof.

A further aspect of the present invention concerns a compound of formula (CIII)

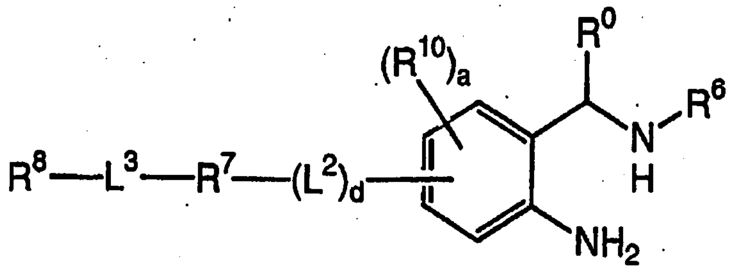

(CIII)

wherein

R° is selected from the group consisting of hydrogen, methyl and $CF_3$;
$R^6$ is selected from the group consisting of $C_{1-6}$alkyl and hydroxy substituted $C_{1-8}$alkyl;
d is an integer from 0 to 1;
$L^2$ is selected from the group consisting of -O-, $-S(O)_{0-2}-$ and $-NR^Q-$;
wherein $R^Q$ is selected from the group consisting of hydrogen and $C_{1-4}$alkyl;
$R^7$ is selected from the group consisting of cycloalkyl, cycloalkyl-$C_{1-4}$alkyl, aryl, $C_{1-4}$alkyl-aryl, aryl-$C_{1-4}$alkyl, partially unsaturated carbocyclyl, partially unsaturated carbocyclyl-$C_{1-4}$alkyl, heteroaryl, heteroaryl-$C_{1-4}$alkyl, heterocycloalkyl and heterocycloalkyl-$C_{1-4}$alkyl;
wherein the aryl, cycloalkyl, partially unsaturated carbocyclyl, heteroaryl or heterocycloalkyl group, whether alone or as part of a substituent group, is optionally substituted with one or more substituents Independently selected from the group consisting of $C_{1-4}$alkyl, $C_{1-4}$alkoxy, halogen substituted $C_{1-4}$alkyl, halogen substituted $C_{1-4}$alkoxy, hydroxy substituted $C_{1-4}$alkyl, hydroxy, carboxy, cyano, nitro, amino, $C_{1-4}$alkylamino, di($C_{1-4}$alkyl)amino, 5-tetrazolyl and 1-(1,4-dihydro-tetrazol-5-one);
$L^3$ is selected from the group consisting of -0-, -S-, -C(O)-, -C(S)-, - C(O)O-, -OC(O)-, $-NR^A-$, -N(CN)-, $-NR^A-C(O)-$, $-C(O)-NR^A-$, $-NR^A-C(S)-$, $-C(S)-NR^A-$, $-NR^A-SO_2-$, $-SO_2-NR^A-$, $-NR^A-SO-$, $-SO-NR^A$, $-NR^A-C(O)O-$, $-OC(O)-NR^A-$, $-O-SO_2-NR^A-$, $-NR^A-SO_2-O-$, $-NR^A-C(O)-NR^B-$, $-NR^A-C(S)-NR^B-$ and $-NR^A-SO_2-NR^B-$;
wherein each $R^A$ and $R^B$ is independently selected from the group consisting of hydrogen, $C_{1-8}$alkyl, hydroxy substituted $C_{1-4}$alkyl, $C_{1-4}$aralkyloxy substituted $C_{1-4}$alkyl, cycloalkyl, aryl, heteroaryl, heterocycloalkyl, cycloalkyl-$C_{1-4}$alkyl-, $C_{1-4}$aralkyl, heteroaryl-$C_{1-4}$alkyl-, heterocycloalkyl-$C_{1-4}$alkyl- and spiro-heterocyclyl;
wherein the cycloalkyl, aryl, heteroaryl, heterocycloalkyl or spiroheterocyclyl, whether alone or as part of a substituent group is optionally substituted with one or more substituents independently selected from the group consisting of halogen, hydroxy, oxo, carboxy, $C_{1-4}$alkyl, $C_{1-4}$alkoxy, $C_{1-4}$alkoxy-carbonyl, nitro, cyano, amino, $C_{1-4}$alkylamino, di ($C_{1-4}$alkyl)amino,$-SO_2-N(R^CR^D)$, 5-tetrazolyl and 1-(1,4-dihydro-tetrazol-5-one)
wherein each $R^C$ and $R^D$ is independently selected from the group consisting of hydrogen and $C_{1-4}$alkyl;
$R^8$ is selected from the group consisting of $C_{1-10}$alkyl, cycloalkyl, aryl, biphenyl; partially unsaturated carbocyclyl, heteroaryl, heterocycloalkyl, cycloalkyl-$C_{1-4}$alkyl-, $C_{1-4}$aralkyl, partially unsaturated carbocyclyl-$C_{1-4}$alkyl-, heteroaryl-$C_{1-4}$alkyl-, heterocycloalkyl-$C_{1-4}$alkyl- and spiro-heterocyclyl;
wherein the $C_{1-10}$alkyl, cycloalkyl, aryl, partially unsaturated carbocyclyl, heteroaryl, heterocycloalkyl or spiro-heterocyclyl, whether alone or as part of a substituent group is optionally substituted with one or more substituents independently selected from the group consisting of halogen, hydroxy, oxo, carboxy, -C(O)-$C_{1-4}$alkyl, -C (O)-$C_{1-4}$aralkyl, -C(O)O-$C_{1-4}$alkyl, -C(O)O-$C_{1-4}$aralkyl, -$C_{1-4}$alkyl-C(O)O-$C_{1-4}$alkyl, -$C_{1-4}$alkyl-S-$C_{1-4}$alkyl, -C(O)-N ($R^L$-$R^M$), -$C_{1-4}$alkyl-C(O)-N($R^LR^M$), $-NR^L-C(O)-$$C_{1-4}$alkyl, $-SO_2-$$C_{1-4}$alkyl, $-SO_2-$aryl, $-SO_2-N(R^LR^M)$, -$C_{1-4}$alkyl-$SO_2-N(R^LR^M)$, $C_{1-4}$alkyl, fluoro substituted $C_{1-4}$alkyl, hydroxy substituted $C_{1-4}$alkyl, carboxy substituted $C_{1-4}$alkyl, $C_{1-4}$alkoxy, fluoro substituted $C_{1-4}$alkoxy, nitro, cyano, amino, $C_{1-4}$alkylamino, di($C_{1-4}$alkyl)amino, phenyl and heteroaryl;
wherein the phenyl or heteroaryl is optionally substituted with one or more substituent independently selected from

the group consisting of halogen, hydroxy, oxo, carboxy, $C(O)O-C_{1-4}$alkyl, $C(O)-C_{1-4}$alkyl, $C_{1-4}$alkyl, fluoro substitute $C_{1-4}$alkyl, $C_{1-4}$alkoxy, nitro, cyano, amino, $C_{1-4}$alkylamino and di($C_{1-4}$alkyl)amino;

wherein each $R^L$ and $R^M$ is independently selected from the group consisting of hydrogen and $C_{1-4}$alkyl;

a is an integer from 0 to 3;

each $R^{10}$ is independently selected from the group consisting of hydroxy, halogen, $C_{1-4}$alkyl, $C_{1-4}$alkoxy, halogen substituted $C_{1-4}$alkyl, halogen substituted $C_{1-4}$alkoxy, $-C(O)-NR^VR^W$, $-SO_2-NR^VR^W$, $-C(O)-C_{1-4}$alkyl and $-SO_2-C_{1-4}$alkyl;

wherein each $R^V$ and $R^W$ is independently selected from the group consisting of hydrogen and $C_{1-4}$alkyl; alternatively $R^V$ and $R^W$ are taken together with the N atom to which they are bound to form a 5 to 6 membered saturated, partially unsaturated or aromatic ring structure;

provided that the halogens on the halogen substituted $C_{1-4}$alkyl or the halogen substituted $C_{1-4}$alkoxy are selected from the group consisting of chloro and fluoro;

or a pharmaceutically acceptable salt thereof.

Illustrative of the invention is a pharmaceutical composition comprising a pharmaceutically acceptable carrier and any of the compounds described above. An illustration of the invention is a pharmaceutical composition made by mixing any of the compounds described above and a pharmaceutically acceptable carrier. Illustrating the invention is a process for making a pharmaceutical composition comprising mixing any of the compounds described above and a pharmaceutically acceptable carrier.

In a method of treating a disorder mediated by the β-secretase enzyme, a therapeutically effective amount of any of the compounds or pharmaceutical compositions described above can be administered to a subject in need thereof.

For example; a disorder selected from the group consisting of Alzheimer's disease (AD), mild cognitive impairment, senility, dementia, dementia with Lewy bodies, Down's syndrome, dementia associated with Parkinson's disease and dementia associated with beta-amyloid, may be treated by administering to a subject in need thereof, a therapeutically effective amount of any of the compounds or pharmaceutical compositions described above.

Another example of the invention is the use of any of the compounds described above in the preparation of a medicament for treating: (a) Alzheimer's Disease (AD), (b) mild cognitive impairment, (c) senility, (d) dementia, (e) dementia with Lewy bodies, (f) Down's syndrome, (g) dementia associated with Parkinson's disease and (h) dementia associated with beta-amyloid, in a subject in need thereof.

## DETAILED DESCRIPTION OF THE INVENTION

The present invention is directed to compounds of formula (III)

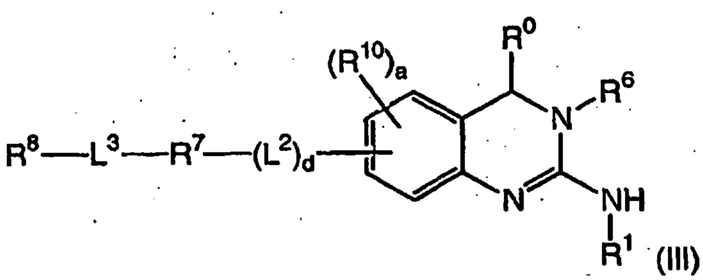

(III)

wherein $R^0$, $R^1$, $R^6$, $R^7$, $R^8$, $R^{10}$, $L^2$, $L^3$, a, d,

are as herein defined. The compounds of formula (III) are inhibitors of the β-secretase enzyme (also known as β-site cleaving enzyme, BACE, BACE1, Asp2 or memapsin2), and are useful in the treatment of Alzheimer's disease (AD), mild cognitive impairment (MCI), senility, dementia, dementia with Lewy bodies, Down's syndrome, dementia associated with Parkinson's disease and dementia associated with beta-amyloid, preferably Alzheimer's disease.

In an embodiment of the present invention is a compound of formula (III) wherein the $-(L^2)_d-R^7-L^3-R^8$ substituent group is bound at the 6 or 7 position, preferably at the 6-position. In another embodiment of the present invention, is a compound of formula (III) wherein the $-L^3-R^8$ substituent group is bound at the 2-, 3- or 4- position of the $R^7$ substituent, preferably, at the 3-position of the $R^7$ substituent.

In an embodiment of the present invention, $R^6$ is selected from the group consisting of hydrogen and methyl. In another embodiment of the present invention, $R^0$ is hydrogen.

In an embodiment of the present invention, $R^1$ is selected from the group consisting of hydrogen, hydroxy, methyl, trifluoromethyl, methoxy and methylcarbonyl. In another embodiment of the present invention $R^1$ is selected

from the group consisting of hydrogen, hydroxy and methoxy. In yet another embodiment of the present invention, $R^1$ is hydrogen.

In an embodiment of the present invention, $R^{10}$ is selected from the group consisting of hydroxy, halogen, $C_{1-4}$alkyl, $C_{1-4}$alkoxy, halogen substituted $C_{1-4}$alkyl, halogen substituted $C_{1-4}$alkoxy; provided that the halogens on the halogen substituted $C_{1-4}$alkyl and the halogen substituted $C_{1-4}$alkoxy are selected from fluoro or chloro. In another embodiment of the present invention, $R^{10}$ is selected from the group consisting of halogen. In another embodiment of the present invention, $R^{10}$ is fluoro.

In an embodiment of the present invention, $R^1$ is selected from the group consisting of hydrogen, methyl and methylcarbonyl. In another embodiment of the present invention, $R^1$ is hydrogen. In another embodiment of the present invention, $R^1$ is selected from the group consisting of hydrogen and methoxy.

In an embodiment of the present invention, $R^6$ is selected from the group consisting of $C_{1-6}$alkyl and hydroxy substituted $C_{1-6}$alkyl. In another embodiment of the present invention, $R^6$ is selected from the group consisting of n-propyl, 4-hydroxy-n-butyl and 5-hydroxy-n-pentyl.

In an embodiment of the present invention, $R^6$ is selected from the group consisting of n-propyl, 4-hydroxy-n-butyl and 5-hydroxy-n-pentyl. In another embodiment of the present invention, $R^6$ is selected from the group consisting of n-propyl and 4-hydroxy-n-butyl. In another embodiment of the present invention, $R^6$ is n-propyl.

In an embodiment of the present invention d is an integer from 0 to 1. In another embodiment of the present invention, d is 1.

In an embodiment of the present invention, $L^2$ is selected from the group consisting of -0-, $-S(O)_{0-2}-$ and -NH-. In another embodiment of the present invention, $L^2$ is selected from the group consisting of -O-, -S-, -SO- and -SO-. In yet another embodiment of the present invention, $L^2$ is selected from the group consisting of -O-, -S- and -SO-. In another embodiment of the present invention, $L^2$ is selected from the group consisting of -O- and -S-. In yet another embodiment of the present invention, $L^2$ is -O-.

In an embodiment of the present invention, $R^7$ is selected from the group consisting of cycloalkyl, cycloalkyl-$C_{1-4}$alkyl, aryl, $C_{1-4}$alkyl-aryl, aryl-$C_{1-4}$alkyl, heteroaryl, heteroaryl-$C_{1-4}$alkyl, heterocycloalkyl and heterocycloalkyl-$C_{1-4}$alkyl; wherein the aryl, cycloalkyl, partially unsaturated carbocyclyl, heteroaryl or heterocycloalkyl group is optionally substituted with one to two substituent independently selected from $C_{1-4}$alkyl, $C_{1-4}$alkoxy, halogen substituted $C_{1-4}$alkyl, halogen substituted $C_{1-4}$alkoxy, hydroxy substituted $C_{1-4}$alkyl, hydroxy, carboxy, cyano, nitro, amino, $C_{1-4}$alkylamino or di($C_{1-4}$alkyl)amino.

In another embodiment of the present invention, $R^7$ is selected from the group consisting of aryl, $C_{1-4}$alkyl-aryl and aryl-$C_{1-4}$alkyl. In another embodiment of the present invention, $R^7$ is selected from the group consisting of phenyl, $-CH_2$-phenyl-, -phenyl-3-$CH_2$- and -phenyl-2-$CH_2$-$CH_2$-.

In an embodiment of the present invention, $R^7$ is selected from the group consisting of -phenyl-, -phenyl-3-$CH_2$- and phenyl-2-$CH_2$-$CH_2$-. In another embodiment of the present invention, $R^7$ is selected from the group consisting of -phenyl- and -phenyl-3-$CH_2$-. In another embodiment of the present invention, $R^7$ is phenyl.

In an embodiment of the present invention, $L^3$ is selected from the group consisting of -C(O)-, -C(O)O-, -OC (O)-, $-NR^A-$, -N(CN)-, $-NR^A$-C(O)-, -C(O)-$NR^A$-, $-NR^A-SO_2-$, $-SO_2-NR^A-$, $-NR^A$-C(O)O- and -OC(O)-$NR^A$.

In another embodiment of the present invention, $L^3$ is selected from the group consisting of -NH-, -N(CN)-, -N ($C_{1-4}$alkyl)-, -NH-C(O)-, -C(O)-NH-, $-NH-SO_2-$, -N($C_{1-4}$alkyl)-C(O)O- and -N(cycloalkyl)-C(O)O-. In yet another embodiment of the present invention, $L^3$ is selected from the group consisting of -NH-, -N(CN)-, $-N(CH_3)-$, -NH-C(O)-, -C (O)-NH-, $-NH-SO_2-$ and - N(cyclohexyl)-C(O)O-; wherein the $L^3$ group is bound at the 3-position of the $R^7$ group.

In an embodiment of the present invention, $L^3$ is selected from the group consisting of -NH-, -N(CN)-, -NH-C (O)-, -NH-C(O)O-, -N(cyclohexyl)-C(O)O- and $-NH-SO_2-$; wherein the $L^3$ is bound to the $R^7$ phenyl at the 3-position. In another embodiment of the present invention, $L^3$ is selected from the group consisting of -NH-, -NH-C(O)- and $-NH-SO_2-$; wherein the $L^3$ is bound to the $R^7$ phenyl at the 3-position. In another embodiment of the present invention, $L^3$ is $-NH-SO_2-$; wherein the $L^3$ is bound to the $R^7$ phenyl at the 3-position.

In an embodiment of the present invention, $L^3$ is selected from the group consisting of -NH-C(O)O-, -N(cyclohexyl)-C(O)O-, -NH-, -N(CN)- and $-NH-SO_2$; wherein the $L^3$ is bound to $R^7$ phenyl at the 3-position.

In an embodiment of the present invention, $R^A$ is selected from the group consisting of hydrogen, $C_{1-4}$alkyl, hydroxy substituted $C_{1-4}$alkyl, $C_{1-4}$aralkyloxy substituted $C_{1-4}$alkyl, cycloalkyl, aryl, heteroaryl, heterocycloalkyl, cycloalkyl-$C_{1-4}$alkyl-, $C_{1-4}$aralkyl, heteroaryl-$C_{1-4}$alkyl- and heterocycloalkyl-$C_{1-4}$alkyl-; wherein the cycloalkyl, aryl, heteroaryl or heterocycloalkyl, whether alone or as part of a substituent group is optionally substituted with one to two substituents independently selected from halogen, hydroxy, oxo, carboxy, $C_{1-4}$alkyl, $C_{1-4}$alkoxy, $C_{1-4}$alkoxy-carbonyl, nitro, cyano, amino, $C_{1-4}$alkylamino or di($C_{1-4}$alkyl)amino.

In an embodiment of the present invention, $R^8$ is selected from the group consisting of $C_{1-6}$alkyl, cycloalkyl, aryl, heteroaryl, heterocycloalkyl, cycloalkyl-$C_{1-4}$alkyl-, $C_{1-4}$aralkyl, heteroaryl-$C_{1-4}$alkyl- and heterocycloalkyl-$C_{1-4}$alkyl-; wherein the $C_{1-6}$alkyl, cycloalkyl, aryl, heteroaryl or heterocycloalkyl, whether alone or as part of a substituent group is optionally substituted with one to two substituents independently selected from halogen, hydroxy, carboxy, -C

(O)-$C_{1-4}$alkyl, -C(O)-$C_{1-4}$aralkyl, -C(O)O-$C_{1-4}$alkyl, -C(O)O-$C_{1-4}$aralkyl, -C(O)-N($R^LR^M$), - $NR^L$-C(O)-$C_{1-4}$alkyl, -$SO_2$-$C_{1-4}$alkyl, -$SO_2$-aryl, -SO2-N($R^LR^M$), $C_{1-4}$alkyl, fluoro substituted $C_{1-4}$alkyl, hydroxy substituted $C_{1-4}$alkyl, carboxy substituted $C_{1-4}$alkyl, $C_{1-4}$alkoxy, fluoro substituted $C_{1-4}$alkoxy, nitro, cyano, amino, $C_{1-4}$alkylamino, di($C_{1-4}$alkyl)amino, phenyl or heteroaryl; wherein the phenyl or heteroaryl substituent is optionally substituted with one or more substituent independently selected from halogen, hydroxy, carboxy, $C_{1-4}$alkyl, fluoro substituted $C_{1-4}$alkyl, $C_{1-4}$alkoxy, nitro, cyano, amino, $C_{1-4}$alkylamino or di($C_{1-4}$alkyl)amino; and wherein each $R^L$ and $R^M$ is independently selected from the group consisting of hydrogen, $C_{1-4}$alkyl and $C_{5-8}$cydoalkyl.

In another embodiment of the present invention, $R^8$ is selected from the group consisting of $C_{1-4}$alkyl, cycloalkyl, cycloatkyl-$C_{1-4}$alkyl, aryl, $C_{1-4}$aralkyl, heteroaryl and heterocycloalkyl; wherein the aryl or heteroaryl, whether alone or as part of a substituent group is optionally substituted with one to three substituents independently selected from halogen, hydroxy, carboxy, $C_{1-4}$alkyl, $C_{1-4}$alkoxy, fluoro substituted $C_{1-4}$alkyl, fluoro substituted $C_{1-4}$alkoxy, cyano, amino, $C_{1-4}$alkylamino, di($C_{1-4}$alkyl)amino, -C(O)O-$C_{1-4}$alkyl, -C(O)-N($R^LR^M$)-$SO_2$-$C_{1-4}$alkyl, -$SO_2$-aryl, -NH-C(O)-$C_{1-4}$alkyl, phenyl or heteroaryl; wherein the phenyl or heteroaryl substituent is optionally substituted with a substituent selected from fluoro substituted $C_{1-4}$alkyl; and wherein each $R^L$ and $R^M$ is independently selected from hydrogen, $C_{1-4}$alkyl or $C_{5-6}$cycloalkyl.

In yet another embodiment of the present invention, $R^8$ is selected from the group consisting of methyl, isopropyl, n-butyl, cyclohexyl, cyclohexyl-methyl, phenyl, phenyl-ethyl, phenyl-n-propyl, 3-(N-methyl-N-cyclohexyl-aminocarbonyl)-n-propyl, 3-bromo-phenyl, 3-methoxy-phenyl, 4-methoxy-phenyl, 2-methoxy-4-methyl-phenyl, 2,4,6-trimethyl-phenyl, 2,5-dimethoxy-phenyl, 3,4-dimethoxy-phenyl, 3-trifluoromethoxy-phenyl, 4-trifluoromethoxy-phenyl, 3-cyano-phenyl, 2-(methyl-sulfonyl)-phenyl, 4-(methyl-carbonyl-amino)-phenyl, 5-carboxy-2-methoxy-phenyl, benzyl, 3-hydroxy-benzyl, 4-methyl-benzyl, 2-methoxy-benzyl, 4-methoxy-benzyl, 2,6-dimethoxy-benzyl, 2,4,6-trimethyl-benzyl, 1-nahthyl, 2-naphthyl, 1-naphthyl-methyl, 1-(5-dimethylamino)-naphthyl, 4-biphenyl, 2-thienyl, 3-thienyl, 4-(3,5-dimethyl-isoxazolyl), 3-benzothienyl, 4-benzo[2,3,1]thiadiazolyl, 2-(5-(2-pyridyl)-thienyl), 2-(5-(3-(2-methyl-thiazolyl)-thienyl)), 2-(5-(3-(5-trifluoromethyl)-isoxazolyl)-thienyl), 6-(2,3-dihydro-benzo[1,4]dioxanyl), 3-(2-methoxy-carbonyl)-thienyl, 2-(5-(5-isoxazolyl)-thienyl)), 2-(5-bromo-thienyl) and 2-(4-phenyl-sulfonyl)-thienyl.

In an embodiment of the present invention, $R^8$ is selected from the group consisting of n-butyl, 3-(N-methyl-N-cyclohexyl-amino-carbonyl)-n-propyl, cyclohexyl, cyclohexyl-methyl-, phenyl, 2-trifluoromethylphenyl, 3-trifluoromethoxyphenyl, 3-cyanophenyl, 3-methoxyphenyl, 4-methoxyphenyl, 2-methoxy-4-methylphenyl, 2,4,6-trimethylphenyl, benzyl, 2-methoxybenzyl, 4-methoxbenzyl, 2,4,6-trimethylbenzyl, phenylethyl-, 2-thieriyl, 3-thienyl, 2-(5-bromo-thienyl) and 3-benzothienyl. In another embodiment of the present In an embodiment of the present invention are compounds of formula (III) selected from the group consisting of the compounds listed in Tables 7-8 below.

In another embodiment of the present invention are compounds of formula (III) whose Ki, as measured according to the procedure described In Example 159, is less than or equal to about 1μM, preferably, less than or equal to about 250 nM, more preferably, less than or equal to about 100nM, more preferably still, less than or equal to about 50nM.

In another embodiment of the present invention are compounds of formula (III), whose $IC_{50}$, as measured according to the procedure described in Example 158, is less than or equal to about 1μM, preferably, less than or equal to about 250nM, more preferably, less than or equal to about 50nM.

Additional embodiments of the present invention, include those wherein the substituents selected for one or more of the variables defined herein (e. g.

$R^0$, $R^1$, $R^6$, $R^7$, $R^8$, $R^{10}$, $L^2$, $L^3$, a, d, etc.) are independently selected to be any individual substituent or any subset of substituents selected from the complete list as defined herein.

Representative compounds of the present invention are as listed in Tables 7 and 8, below. Unless otherwise noted, all compounds were prepared as mixtures of stereo-isomers. Representative compounds of formula (III) are as listed in Tables 7 and 8; below.

For substituent groups bound through two points within the structures in the Tables below, for example $R^7$, $L^2$, $L^3$, etc., the substituent group is identified as it would be incorporated into the structure heading the table. Thus, for example in Table 7, wherein $R^7$ is -$CH_2$-phenyl-, the carbon of the $CH_2$- is bound to the $L^3$ group and the phenyl is bound to the $(L^2)_d$ group whereas when $R^7$ is -phenyl-3-$CH_2$-, the phenyl is bound to the $L^3$ group and the carbon of the $CH_2$- is bound to the $(L^2)_d$ group. Further, unless otherwise noted, any terminal substituent group is bound at the 1-position. Thus for example, 4-fluorophenyl corresponds to a phenyl group bound at the 1-position and substituted with a fluoro group at the 4-position, and could alternatively be defined as 1-(4-fluorophenyl).

Representative compounds of formula (III) are as listed in Tables 7-8, below.

**Table 7: Compounds of formula (III)**

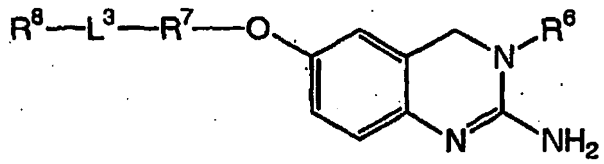

| ID No | $R^6$ | $R^7$ | $L^3$ | $R^8$ |
|---|---|---|---|---|
| 44 | n-propyl | -phenyl- | -3-NH-C(O)- | cyclohexyl |
| 49 | n-propyl | -phenyl- | -3-N(cyclohexyl)-C(O)O- | benzyl |
| 53 | n-propyl | -phenyl- | -3-NH- | cyclohexyl |
| 55 | n-propyl | -phenyl- | -3-NH- | cyclohexyl-methyl- |
| 56 | n-propyl | -phenyl- | -3-N(CN)- | cyclohexyl-methyl- |
| 60 | n-propyl | -phenyl- | -3-NH-C(O)- | phenyl |
| 61 | n-propyl | -phenyl- | $-3-NH-SO_2-$ | phenyl |
| 67 | n-propyl | -phenyl- | -3-NH-C(O)O- | benzyl |
| 95 | 4-hydroxy-n-butyl | -phenyl- | -3-NH- | cyclohexyl-methyl- |
| 126 | 4-hydroxy-n-butyl | -phenyl- | $-3-NH-SO_2-$ | phenyl |
| 203 | n-propyl | $-CH_2-phenyl-$ | $-3-NH-SO_2-$ | phenyl |
| 225 | n-propyl | $-phenyl-3-CH_2-$ | $-NH-SO_2-$ | phenyl |
| 237 | n-propyl | -phenyl- | $-3-NH-SO_2-$ | methyl |
| 238 | n-propyl | -phenyl- | $-3-NH-SO_2-$ | isopropyl |
| 239 | n-propyl | -phenyl- | $-3-NH-SO_2-$ | n-butyl |
| 240 | n-propyl | -phenyl- | $-3-NH-SO_2-$ | 3-thienyl |
| 241 | n-propyl | -phenyl- | $-3-NH-SO_2-$ | 2-thienyl |
| 242 | n-propyl | -phenyl- | $-3-NH-SO_2-$ | 4-(3,5-dimethyl-isoxazolyl) |
| 243 | n-propyl | -phenyl- | $-3-NH-SO_2-$ | 2,4,6-trimethyl-phenyl |
| 244 | n-propyl | -phenyl- | -3-NH-S02- | 2-naphthyl |
| 245 | n-propyl | -phenyl- | $-3-NH-SO_2-$ | 3-benzothienyl |
| 246 | n-propyl | -phenyl- | $-3-NH-SO_2-$ | 4-(methyl-carbonyl-amino)-phenyl |
| 247 | n-propyl | -phenyl- | $-3-NH-SO_2-$ | 4-benzo[2,3,1]-thiadiazolyl |
| 248 | n-propyl | -phenyl- | $-3-NH-SO_2-$ | 2,5-dimethoxy-phenyl |
| 249 | n-propyl | -phenyl- | $-3-NH-SO_2-.$ | 3,4-dimethoxy-phenyl |
| 250 | n-propyl | -phenyl- | $-3-NH-SO_2-$ | 2-(methyl-sulfonyl)-phenyl |
| 251 | n-propyl | -phenyl- | $-3-NH-SO_2-$ | 2-(5-(2-pyridyl)-thienyl) |
| 252 | n-propyl | -phenyl- | $-3-NH-SO_2-$ | 3-trifluoromethoxy-phenyl |
| 253 | n-propyl | -phenyl- | $-3-NH-SO_2-$ | 1-(5-(dimethyl-amino)-naphthyl) |
| 254 | n-propyl | -phenyl- | $-3-NH-SO_2-$ | 2-(5-(3-(2-methyl-thiazolyl)-thienyl)) |

(continued)

| ID No | $R^6$ | $R^7$ | $L^3$ | $R^8$ |
|---|---|---|---|---|
| 255 | n-propyl | -phenyl- | -3-NH-$SO_2$- | 2-(5-(3-(5-trifluoro-methyl)-isoxazolyl)-thienyl) |
| 269 | n-propyl | -phenyl- | -3-NH-$SO_2$- | benzyl |
| 270 | n-propyl | -phenyl- | -3-NH-$SO_2$- | 3-cyano-phenyl |
| 271 | n-propyl | -phenyl- | -3-NH-$SO_2$- | 3-methoxy-phenyl |
| 272 | n-propyl | -phenyl- | -3-NH-$SO_2$- | 4-methoxy-phenyl |
| 273 | n-propyl | -phenyl- | -3-NH-$SO_2$- | 2-methoxy-4-methyl-phenyl |
| 274 | n-propyl | -phenyl- | -3-NH-$SO_2$- | 1-naphthyl |
| 275 | n-propyl | -phenyl- | -3-NH-$SO_2$- | 6-(2,3-dihydro-benzo[1,4] dioxanyl) |
| 276 | n-propyl | -phenyl- | -3-NH-$SO_2$- | 3-(2-methoxy-carbonyl)-thienyl) |
| 277 | n-propyl | -phenyl- | -3-NH-$SO_2$- | 2-trifluoromethyl-phenyl |
| 278 | n-propyl | -phenyl- | -3-NH-$SO_2$- | 2-(5-(5-isoxazolyl)-thienyl)) |
| 279 | n-propyl | -phenyl- | -3-NH-$SO_2$- | 5-carboxy-2-methoxy-phenyl |
| 280 | n-propyl | -phenyl- | -3-NH-$SO_2$- | 4-biphenyl |
| 281 | n-propyl | -phenyl- | -3-NH-$SO_2$- | 1-naphthyl-ethyl- |
| 282 | n-propyl | -phenyl- | -3-NH-$SO_2$- | 3-bromo-phenyl |
| 283 | n-propyl | -phenyl- | -3-NH-$SO_2$- | 4-trifluoromethoxy-phenyl |
| 284 | n-propyl | -phenyl- | -3-NH-$SO_2$- | 2-(5-bromo-thienyl) |
| 285 | n-propyl | -phenyl- | -3-NH-$SO_2$- | 2-(4-phenyl-sulfonyl)-thienyl |
| 296 | n-propyl | -phenyl-3-$CH_2$- | -NH- | benzyl |
| 305 | n-propyl | -phenyl-3-$CH_2$- | -NH- | 3-hydroxy-benzyl |
| 335 | n-propyl | -phenyl-3-$CH_2$- | -NH- | 2-methoxy-benzyl |
| 340 | n-propyl | -phenyl-3-$CH_2$- | -NH- | 2,6-dimethoxy-benzyl |
| 344 | n-propyl | -phenyl-3-$CH_2$- | -NH- | 2,4,6-trimethyl-benzyl |
| 355 | 5-hydroxy-n-pentyl | -phenyl- | -3-NH-$SO_2$- | 2-methoxy-4-methyl-phenyl |
| 380 | 5-hydroxy-n-pentyl | -phenyl- | -3-NH-$SO_2$- | phenyl |
| 383 | 5-hydroxy-n-pentyl | -phenyl- | -3-NH-$SO_2$- | 2,4,6-trimethyl-phenyl |
| 423 | n-propyl | -phenyl- | -3-C(O)-NH- | 2,4,6-trimethyl-benzyl |
| 430 | n-propyl | -phenyl-3-$CH_2$- | -NH- | phenyl-n-propyl- |
| 431 | n-propyl | -phenyl-3-$CH_2$- | -NH- | phenylethyl- |
| 464 | n-propyl | -phenyl-3-$CH_2$- | -N($CH_3$)- | 2,4,6-trimethyl-benzyl |
| 500 | n-propyl | -phenyl- | -3-NH- | 2,4,6-trimethyl-benzyl |
| 501 | n-propyl | -phenyl- | -3-NH- | 2,6-dimethoxy-benzyl |
| 502 | n-propyl | -phenyl- | -3-NH- | 4-methoxy-benzyl |
| 507 | n-propyl | -phenyl- | -3-NH- | benzyl |
| 533 | n-propyl | -phenyl-3-$CH_2$- | -NH-C(O)- | 2,4,6-trimethyl-phenyl |

(continued)

| ID No | $R^6$ | $R^7$ | $L^3$ | $R^8$ |
|---|---|---|---|---|
| 729 | n-propyl | -phenyl- | -3-NH- | 4-methyl-benzyl |
| 755 | n-propyl | -phenyl- | -3-NH-C(O)- | 3-(N-methyl-N-cyclohexyl-amino-carbonyl)-n-propyl |

**Table 8: Compounds of formula (III)**

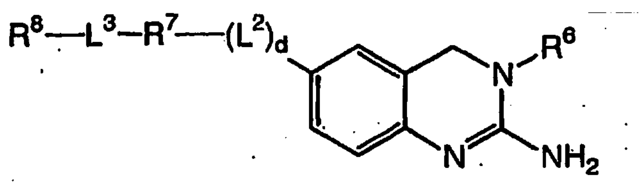

| ID No | $R^6$ | $(L^2)_d$ | $R^7$ | $L^3$ | $R^8$ |
|---|---|---|---|---|---|
| 345 | n-propyl | -S- | -phenyl- | -3-NH-$SO_2$- | phenyl |
| 385 | n-propyl | absent | -phenyl-2-$CH_2$-$CH_2$- | -NH-$SO_2$- | phenyl |
| 387 | n-propyl | -S(O)- | -phenyl- | -3-NH-$SO_2$- | phenyl |
| 397 | n-propyl | absent | -phenyl- | -3-NH-$SO_2$- | phenyl |
| 497 | n-propyl | -S- | -phenyl- | -3-NH-$SO_2$- | 2,4,6-trimethyl-phenyl |

Table 14 below lists representative intermediates and by-products in the preparation of the compounds of formula (III) of the present Invention.

**Table 14: Intermediates or By-Products**

| | |
|---|---|
| 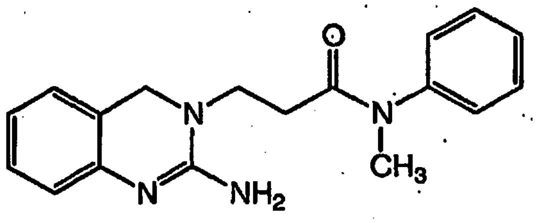 Cmpd #800 | 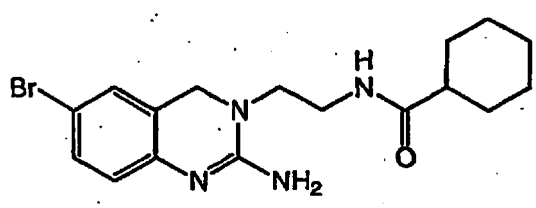 Cmpd #806 |
| 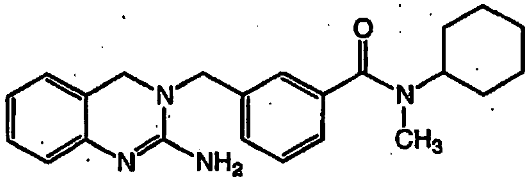 Cmpd #801 | 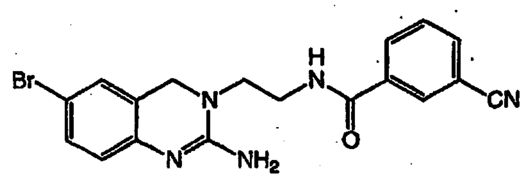 Cmpd #807 |
| 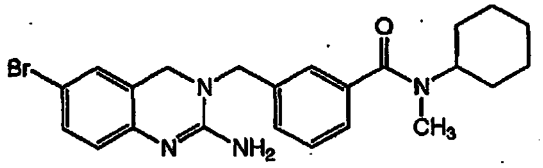 Cmpd #802 | 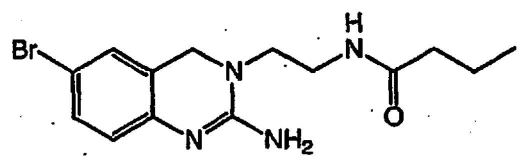 Cmpd #808 |

(continued)

| | |
|---|---|
| 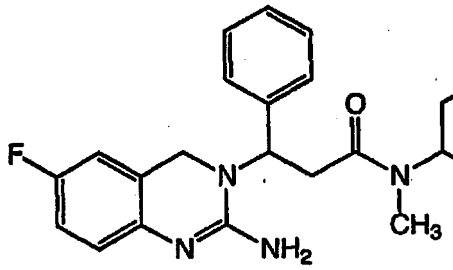 Cmpd #803 | 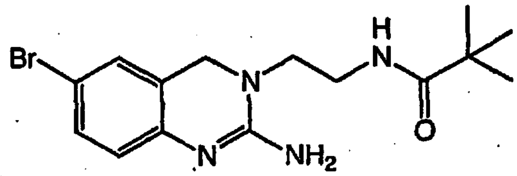 Cmpd #809 |
| 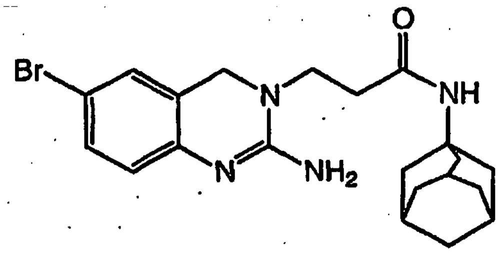 Cmpd #804 | 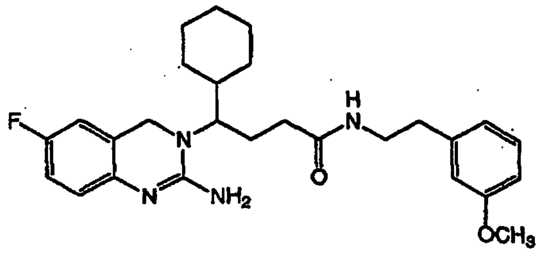 Cmpd #810 |
| 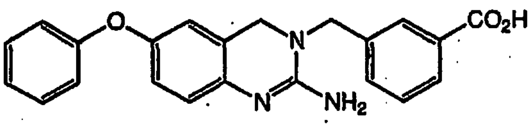 Cmpd #805 | 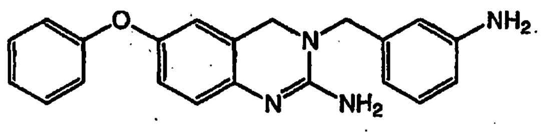 Cmpd #811 |

The present invention is further directed to compounds of formula (CIII) and

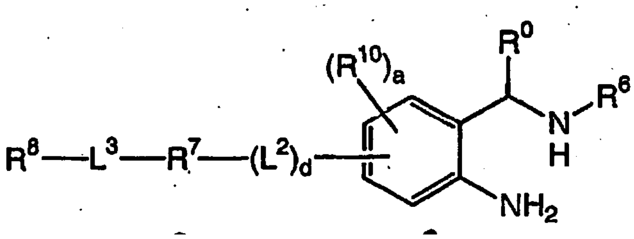

(CIII)

wherein $R^0$, $R^6$, $R^7$, $R^8$, $R^{10}$, $L^2$, $L^3$, a, d,
are as herein defined. The compounds of formula (CIII) are useful as intermediates in the preparation of the compounds of formula (III) of the present invention.

As used herein, unless otherwise noted, the term "**halogen**" shall mean chlorine, bromine, fluorine and iodine.

As used herein, unless otherwise noted, the term **"alkyl"** whether used alone or as part of a substituent group, includes straight and branched chains. For example, alkyl radicals include methyl, ethyl, n-propyl, isopropyl, n-butyl, isobutyl, sec-butyl, t-butyl, n-pentyl, isopentyl, hexyl, heptyl, octyl, nonyl, decyl, and the like. Similarly, the term "$C_{1-8}$alkl" shall include straight and branched chains comprising one to eight carbon atoms. The term "alkyl" may also encompass multivalent radicals where indicated in the specification and claims.

As used herein, unless otherwise noted, "**alkoxy**" shall denote an oxygen ether radical of the above described straight or branched chain alkyl groups. For example, methoxy, ethoxy, n-propoxy, sec-butoxy, t-butoxy, n-hexyloxy and the like.

As used herein, unless otherwise noted, the terms **"halogen substituted $C_{1-4}$alkyl"** and **"halogenated $C_{1-4}$alkyl"**, shall mean a straight or branched chain alkyl group comprising one to four carbon atoms, wherein the alkyl is substituted with one or more, preferably one to five, more preferably one to three halogen atoms. Preferably, the halogen is selected from the group consisting of chloro and fluoro.

Similarly, the terms **"halogen substituted $C_{1-4}$alkoxy"** and **"halogenated $C_{1-4}$alkoxy"** shall mean a straight or branched chain alkoxy group comprising one to four carbon atoms, wherein the alkoxy is substituted with one or more, preferably one to five, more preferably one to three halogen atoms. Preferably, the halogen is selected from the group consisting of chloro and fluoro.

As used herein, unless otherwise noted, the term **"hydroxy substituted $C_{1-4}$alkyl"** shall mean a straight or branched chain $C_{1-4}$alkyl, wherein the $C_{1-4}$alkyl is substituted with one or more, preferably one to three hydroxy groups, more preferably one to two hydroxy groups. Most preferably, the $C_{1-4}$alkyl group is substituted with one hydroxy group. Preferably, wherein the $C_{1-4}$alkyl group has a terminal carbon atom, the hydroxy group is bound at said terminal carbon atom.

As used herein, unless otherwise noted, the term "**carboxy substituted $C_{1-4}$alkyl**" shall mean a straight or branched chain $C_{1-4}$alkyl, wherein the $C_{1-4}$alkyl is substituted with one or more, preferably one to three carboxy groups, more preferably one to two carboxy groups. Most preferably, the $C_{1-4}$alkyl group is substituted with one carboxy group. Preferably, wherein the $C_{1-4}$alkyl group has a terminal carbon atom, the carboxy group is bound at said terminal carbon atom.

As used herein, unless otherwise noted, **"aryl"** shall refer to fully conjugated aromatic ring structures such as phenyl, naphthyl, and the like.

As used herein, unless otherwise noted, **"$C_{1-4}$aralkyl"** shall mean any $C_{1-4}$alkyl group substituted with an aryl group such as phenyl, naphthyl and the like. For example, benzyl, phenylethyl, phenylpropyl, naphthylmethyl, and the like. Unless otherwise noted, the **"$C_{1-4}$aralkyl"** group is bound through the alkyl portion. For example, phenylethyl- is bound through the terminal carbon atom of the ethyl group (i.e. phenyl-$CH_2$-$CH_2$-).

As used herein, unless otherwise noted, the term **"cycloalkyl"** shall mean any stable monocyclic, bicyclic, polycyclic, bridged or spiro-bound, saturated ring system. Suitable examples include, but are not limited to, cyclopropyl, cyclobutyl, cyclopentyl, Cyclohexyl, cycloheptyl, cyclooctyl, norboranyl, adamantyl, spiropentane, 2,2,2-bicyclooctyl, and the like. Unless otherwise noted, **"cycloalkyl"** groups do not contain N, O or S heteroatoms.

As used herein, unless otherwise noted, the term **"partially unsaturated carbocyclyl"** shall mean any stable monocyclic, bicyclic, polycyclic, bridge or spiro-bound ring system containing at least one carbon atom which is not part of an unsaturated bond (i.e. a double or triple bond) or any bicyclic, polycyclic, bridged or spiro-bound, partially aromatic (e.g. benzo-fused) rings system. Suitable examples include, but are not limited to 1,2,3,4-tetrahydro-naphthyl, fluorenyl, 9,10-dihydroanthracenyl, indanyl, and the like. Unless otherwise noted, **"partially unsaturated carbocyclyl"** groups do not contain N, O or S heteroatoms.

As used herein, unless otherwise noted, **"heteroaryl"** shall denote any five or six membered monocyclic aromatic ring structure containing at least one heteroatom selected from the group consisting of O, N and S, optionally containing one to three additional heteroatoms independently selected from the group consisting of O, N and S; or a nine or ten membered bicyclic aromatic ring structure containing at least one heteroatom selected from the group consisting of O, N and S, optionally containing one to four additional heteroatoms independently selected from the group consisting of O, N and S. The heteroaryl group may be attached at any heteroatom or carbon atom of the ring such that the result is a stable structure.

Examples of suitable heteroaryl groups include, but are not limited to, pyrrolyl, furyl, thienyl, oxazolyl, imidazolyl, pyrazolyl, isoxazolyl, isothiazolyl, triazolyl, thiadiazolyl, pyridyl, pyridazinyl, pyrimidinyl, pyrazinyl, pyranyl, furazanyl, indolizinyl, indolyl, isoindolinyl, indazolyl, benzofuryl, benzothienyl, benzimidazolyl, benzthiazolyl, purinyl, quinolizinyl, quinolinyl, isoquinolinyl, isothiazolyl, cinnolinyl, phthalazinyl, quinazolinyl, quinoxalinyl, naphthyridinyl, pteridinyl, tetrazolyl, triazolyl, and the like. Preferred heteroaryl groups include furyl, thienyl, imidazolyl, pyridyl, triazolyl, benzimidazolyl and tetrazolyl.

As used herein, the term **"heterocycloalkyl"** shall denote any five to seven membered monocyclic, saturated or partially unsaturated ring structure containing at least one heteroatom selected from the group consisting of O, N and S, optionally containing one to three additional heteroatoms independently selected from the group consisting of O, N and S; or a nine to ten membered saturated, partially unsaturated or partially aromatic (e.g. benzo-fused) bicyclic ring system containing at least one heteroatom selected from the group consisting of O, N and S, optionally containing one to four additional heteroatoms independently selected from the group consisting of O, N and S. The heterocycloalkyl group may be attached at any heteroatom or carbon atom of the ring such that the result is a stable structure.

Examples of suitable heterocycloalkyl groups include, but are not limited to, pyrrolinyl, pyrrolidinyl, dioxalanyl, imidazolinyl, imidazolidinyl, pyrazolinyl, pyrazolidinyl, piperidinyl, dioxanyl, morpholinyl, dithianyl, thiomorpholinyl, piperazinyl, trithianyl, indolinyl, chromenyl, 3,4-methylenedioxyphenyl, 2,3-dihydrobenzofuryl, tetrahydropyranyl, azepinyl, 2,3-dihydro-1,4-benzodioxanyl, and the like. Preferred heterocycloalkyl groups include piperidinyl, morpholinyl, tetrahydropyranyl (preferably tetrahydropyran-2-yl or tetrahydropyran-6-yl) and azepinyl.

As used herein, unless otherwise noted, the term **"spiro-heterocyclyl"** shall mean any spiro-bound ring structure wherein the spiro-bound ring structure contains at least one heteroartom selected from O, S or N. Suitable examples include, but are not limited to 1,4-dioxaspiro[4.5]decyl, 1-oxa-4-azaspiro[4.5]decyl, 1-thia-4azaspiro[4.5]decyl,

1,4-diazaspiro[4.5]decyl, 1,3-diazaspiro[4.5]dec-2-2nyl and 1-oxa-azaspiro[4.5]dec-2-enyl. Preferred spiro-heterocyclyl groups include

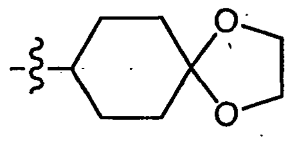

(i.e. 1,4-dioxaspiro[4.5]decyl).

As used herein, the notation "*" shall denote the presence of a stereogenic center.

When a particular group is **"substituted"** (e.g., cycloalkyl, aryl, heterocycloalkyl, heteroaryl, etc.), that group may have one or more substituents, preferably from one to five substituents, more preferably from one to three substituents, most preferably from one to two substituents, independently selected from the list of substituents.

With reference to substituents, the term **"independently"** means that when more than one of such substituents is possible, such substituents may be the same or different from each other.

To provide a more concise description, some of the quantitative expressions given herein are not qualified with the term **"about"**. It is understood that whether the term "about" is used explicitly or not, every quantity given herein is meant to refer to the actual given value, and it is also meant to refer to the approximation to such given value that would reasonably be inferred based on the ordinary skill in the art, including approximations due to the experimental and/or measurement conditions for such given value.

As used herein, unless otherwise noted, the term **"aprotic solvent"** shall mean any solvent that does not yield a proton. Suitable examples include, but are not limited to DMF, dioxane, THF, acetonitrile, pyridine, dichloroethane, dichloromethane, MTBE, toluene, and the like.

As used herein, unless otherwise noted, the term **"leaving group"** shall mean a charged or uncharged atom or group which departs during a substitution or displacement reaction. Suitable examples include, but are not limited to, Br, Cl, I, mesylate, tosylate, and the like.

As used herein, unless otherwise noted, the term **"nitrogen protecting group"** shall mean a group which may be attached to a nitrogen atom to protect said nitrogen atom from participating in a reaction and which may be readily removed following the reaction. Suitable nitrogen protecting groups include, but are not limited to carbamates - groups of the formula -C(O)O-R wherein R is for example methyl, ethyl, t-butyl, benzyl, phenylethyl, $CH_2$=CH-$CH_2$-, and the like; amides - groups of the formula -C(O)-R' wherein R' is for example methyl, phenyl, trifluoromethyl, and the like; N-sulfonyl derivatives-groups of the formula -$SO_2$-R" wherein R" is for example tolyl, phenyl, trifluoromethyl, 2,2,5,7,8-pentamethylchroman-6-yl-, 2,3,6-trimethyl-4-methoxybenzene, and the like. Other suitable nitrogen protecting groups may be found in texts such as T.W. Greene & P.G.M. Wuts, Protective Groups in Organic Synthesis, John Wiley & Sons, 1999.

As used herein, unless otherwise noted, the term **"oxygen protecting group"** shall mean a group which may be attached to an oxygen atom to protect said oxygen atom from participating in a reaction and which may be readily removed following the reaction. Suitable examples include, but are not limited to methyl, benzyl, trimethylsilyl, *tert*-butyldimethylsilyl, acetate, 1-ethoxyethyl, and the like. Other suitable nitrogen protecting groups may be found in texts such as T.W. Greene & P.G.M. Wuts, Protective Groups in Organic Synthesis, John Wiley & Sons, 1991.

Under standard nomenclature used throughout this disclosure, the terminal portion of the designated side chain is described first, followed by the adjacent functionality toward the point of attachment. Thus, for example, a "phenyl-($C_1$-$C_6$alkyl)-aminocarbonyl-($C_1$-$C_6$alkyl)-" substituent refers to a group of the formula

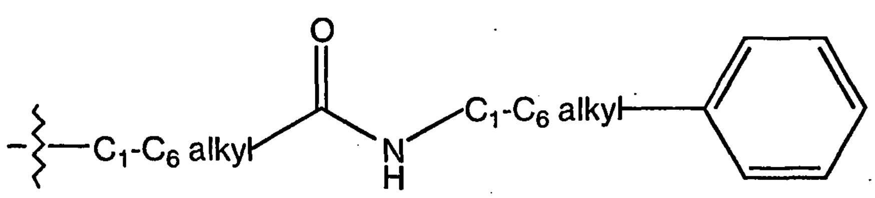

Unless otherwise noted, the position at which substituent groups on the compounds of formula (I), formula (II) and formula (III) are bound to the 2-amino-quinazoline core shall be denoted as follows:

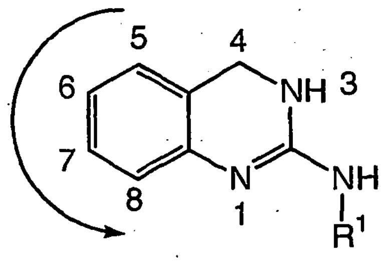

Abbreviations used in the specification, particularly the Schemes and Examples, are as follows:

| | |
|---|---|
| Ac = | Acetyl (i.e. -C(O)-$CH_3$) |
| ACN = | Acetonitrile |
| AD = | Alzheimer's Disease |
| AgOAc = | Silver Acetate |
| BACE = | β-secretase |
| $BH(OAc)_3$ = | Triacetoxy Borohydride |
| BOC or Boc = | *t*-Butoxycarbonyl |
| $(Boc)_2O$ = | Boc Anhydride |
| Cbz = | Carbobenzyloxy |
| DBU = | 1,8-iazabicyclo[5.4.0]undec-7-ene |
| DCC = | N,N'-Dicyclohexylcarbodiimide |
| DCE = | 1,2-Dichloroethane |
| DCM = | Dichloromethane |
| DEA = | diethylamine |
| DEAD = | Diethylazodicarboxylate |
| DIAD = | Diisopropylazodicarboxylate |
| DIPE = | Diisopropyl Ether |
| DIPCDI = | 1,3-Diisopropylcarbodiimide |
| DIPEA or DIEA = | Diisopropylethylamine |
| DMA = | N,N-Dimethylacetamide |
| DMAP = | 4-N,N-Dimethylaminopyridine |
| DME = | Dimethoxyethane |
| DMF = | N,N-Dimethylformamide |
| DMSO. = | Dimethylsulfoxide |
| dppf = | 1,1'-Bis(diphenylphosphino)ferrocene |
| EDC = | 1-(3-Dimethylaminoproyl)-3-ethylcarbodiimide hydrochoride |
| EDCI = | 1-(3-Dimethylaminopropyl)3-ethylcarbodiimide hydrochloride |
| Et = | Ethyl(-$CH_2CH_3$) |
| $Et_3N$ = | Triethylamine |
| $Et_2O$ = | Diethyl Ether |
| EtOAc = | Ethyl acetate |
| EtOH = | Ethanol |
| HOAc = | Acetic acid |
| HATU = | O-(7-Azabenzotriazol-1-yl)-N,N,N",N"- Tetramethyl Uronium Hexafluorophosphate |
| HBTU = | *O*-Benzotriazol-1-yl-*N, N, N', N'*- tetramethyluronium hexafluorophosphate |
| HEPES = | 4-(2-Hydroxyethyl)-1-Piperizine Ethane Sulfonic Acid |
| HOBT or HOBt = | 1-Hydroxybenzotriazole |
| HPLC = | High Pressure Liquid Chromatography |
| LAH = | Lithium Aluminum Hydride |
| μwave = | Microwave |
| MCPBA = | 2-(4-Chloro-2-methylphenoxy)acetic acid |
| Me = | Methyl |
| MeCN = | Acetonitrile |
| MeOH = | Methanol |
| $MeONH_2$ HCl = | O-methylhydroxylamine hydrochloride |
| MTBE = | Methyl-*tert*-Butyl Ether |

| | |
|---|---|
| $Na(OAC)_3BH$ = | Sodium triacetoxyborohydride |
| $NH_4OAc$ = | Ammonium Acetate |
| NMR = | Nuclear Magnetic Resonance |
| OXONE® = | Potassium Monopersulfaphate Triple Salt |
| Pd-C or Pd/C = | Palladium on Carbon Catalyst |
| Pt-C or Pt/C = | Platinum on Carbon Catalyst |
| $Pd_2(OAc)_2$ = | Palladium(II)acetate |
| $Pd_2(dba)_3$ = | Tris(dibenzylidene acetone)dipalladium(0) |
| $Pd(dppf)Cl_2$ = | Dichloro[1,1'- bis(diphenylphosphino)ferrocene]palladium |
| $Pd(PPh_3)_4$ = | tetrakistriphenylphosphine palladium (0) |
| $Pd(PCy_3)_2Cl_2$ = | Dichlorobis(tricyclohexylphosphine)palladium |
| PTSA or *p*-TsOH = | *p*-Toluenesulfonic acid Monohydride |
| q. s. = | Quantum Sufficiat (Quantity Sufficient) |
| RP-HPLC = | Reverse Phase High Pressure Liquid Chromatography |
| RT or rt = | Room temperature |
| SPE = | Solid phase extraction |
| t-BOC or Boc = | Tert-Butoxycarbonyl |
| TDA-1 = | Tris(3,6-Dioxaheptyl)amine |
| TEA = | Triethylamine |
| TEMPO = | 2,2,6,6-Tetramethyl-1-piperidinyloxy Free Radical |
| TFA = | Trifluoroacetic Acid |
| THF = | Tetrahydrofuran |
| TLC = | Thin Layer Chromatography |

The term **"subject"** as used herein, refers to an animal, preferably a mammal, most preferably a human, who is or has been the object of treatment, observation or experiment.

The term **"therapeutically effective amount"** as used herein, means that amount of active compound or pharmaceutical agent that elicits the biological or medicinal response in a tissue system, animal or human that is being sought by a researcher, veterinarian, medical doctor or other clinician, which includes alleviation of the symptoms of the disease or disorder being treated.

As used herein, the term **"composition"** is intended to encompass a product comprising the specified ingredients in the specified amounts, as well as any product which results, directly or indirectly, from combinations of the specified ingredients in the specified amounts.

Where the compounds according to this invention have at least one **chiral center,** they may accordingly exist as enantiomers. Where the compounds possess two or more chiral centers, they may additionally exist as **diastereomers.** It is to be understood that all such isomers and mixtures thereof are encompassed within the scope of the present invention. Preferably, wherein a compound of the present invention is present an enantiomer, the enantiomer is present at an enantiomeric excess of greater than or equal to about 80%, more preferably, at an enantiomeric excess of greater than or equal to about 90%, more preferably still, at an enantiomeric excess of greater than or equal to about 95%, more preferably still, at an enantiomeric excess of greater than or equal to about 98%, most preferably, at an enantiomeric excess of greater than or equal to about 99%. Similarly, wherein a compound of the present invention is a diastereomer, the diastereomer is present at an diastereomeric excess of greater than or equal to about 80%, more preferably, at an diastereomeric excess of greater than or equal to about 90%, more preferably still, at an diastereomeric excess of greater than or equal to about 95%, more preferably still, at an diastereomeric excess of greater than or equal to about 98%, most preferably, at an diastereomeric excess of greater than or equal to about 99%.

Some of the crystalline forms for the compounds may exist as polymorphs and as such are intended to be included in the present invention. In addition, some of the compounds may form solvates with water (i.e., hydrates) or common organic solvents, and such solvates are also intended to be encompassed within the scope of this invention.

One skilled in the art will recognize that wherein a reaction step of the present invention may be carried out in a variety of solvents or solvent systems, said reaction step may also be carried out in a mixture of the suitable solvents or solvent systems.

Where the processes for the preparation of the compounds according to the invention give rise to mixture of stereoisomers, these isomers may be separated by conventional techniques such as preparative chromatography or recrystallization. The compounds may be prepared in racemic form, or individual enantiomers may be prepared either by enantiospecific synthesis or by resolution. The compounds may, for example, be resolved into their component enantiomers by standard techniques, such as the formation of diastereomeric pairs by salt formation with an optically active acid, such as (-)-di-p-toluoyl-D-tartaric acid and/or (+)-di-p-toluoyl-L-tartaric acid followed by fractional crystallization and regeneration of the free base. The compounds may also be resolved by formation of diastereomeric esters

or amides, followed by chromatographic separation and removal of the chiral auxiliary. Alternatively, the compounds may be resolved using a chiral HPLC column.

During any of the processes for preparation of the compounds of the present invention, it may be necessary and/or desirable to protect sensitive or reactive groups on any of the molecules concerned. This may be achieved by means of conventional protecting groups, such as those described in Protective Groups in Organic Chemistry, ed. J.F.W. McOmie, Plenum Press, 1973; and T.W. Greene & P.G.M. Wuts, Protective Groups in Organic Synthesis, John Wiley & Sons, 1999. The protecting groups may be removed at a convenient subsequent stage using methods known from the art.

The present invention includes within its scope **prodrugs** of the compounds of this invention. In general, such prodrugs will be functional derivatives of the compounds which are readily convertible *in vivo* into the required compound. Thus, in the methods of treatment of the present invention, the term "administering" shall encompass the treatment of the various disorders described with the compound specifically disclosed or with a compound which may not be specifically disclosed, but which converts to the specified compound *in vivo* after administration to the patient. Conventional procedures for the selection and preparation of suitable prodrug derivatives are described, for example, in Design of Prodrugs, ed. H. Bundgaard, Elsevier, 1985.

For use in medicine, the salts of the compounds of this invention refer to non-toxic **"pharmaceutically acceptable salts."** Other salts may, however, be useful in the preparation of compounds according to this invention or of their pharmaceutically acceptable salts. Suitable pharmaceutically acceptable salts of the compounds include acid addition salts which may, for example, be formed by mixing a solution of the compound with a solution of a pharmaceutically acceptable acid such as hydrochloric acid, sulfuric acid, fumaric acid, maleic acid, succinic acid, acetic acid, benzoic acid, citric acid, tartaric acid, carbonic acid or phosphoric acid. Furthermore, where the compounds of the invention carry an acidic moiety, suitable pharmaceutically acceptable salts thereof may include alkali metal salts, e.g., sodium or potassium salts; alkaline earth metal salts, e.g., calcium or magnesium salts; and salts formed with suitable organic ligands, e.g., quaternary ammonium salts. Thus, representative pharmaceutically acceptable salts include the following:

acetate, benzenesulfonate, benzoate, bicarbonate, bisulfate, bitartrate, borate, bromide, calcium edetate, camsylate, carbonate, chloride, clavulanate, citrate, dihydrochloride, edetate, edisylate, estolate, esylate, fumarate, gluceptate, gluconate, glutamate, glycollylarsanilate, hexylresorcinate, hydrabamine, hydrobromide, hydrochloride, hydroxynaphthoate, iodide, isothionate, lactate, lactobionate, laurate, malate, maleate, mandelate, mesylate, methylbromide, methylnitrate, methylsulfate, mucate, napsylate, nitrate, N-methylglucamine ammonium salt, oleate, pamoate (embonate), palmitate, pantothenate, phosphate/diphosphate, polygalacturonate, salicylate, stearate, sulfate, subacetate, succinate, tannate, tartrate, teoclate, tosylate, triethiodide and valerate.

Representative acids and bases which may be used in the preparation of pharmaceutically acceptable salts include the following:

acids including acetic acid, 2,2-dichloroactic acid, acylated amino acids, adipic acid, alginic acid, ascorbic acid, L-aspartic acid, benzenesulfonic acid, benzoic acid, 4-acetamidobenzoic acid, (+)-camphoric acid, camphorsulfonic acid, (+)-(1S)-camphor-10-sulfonic acid, capric acid, caproic acid, caprylic acid, cinnamic acid, citric acid, cyclamic acid, dodecylsulfuric acid, ethane-1,2-disulfonic acid, ethanesulfonic acid, 2-hydroxy-ethanesulfonic acid, formic acid, fumaric acid, galactaric acid, gentisic acid, glucoheptonic acid, D-gluconic acid, D-glucoronic acid, L-glutamic acid, $\alpha$-oxo-glutaric acid, glycolic acid, hipuric acid, hydrobromic acid, hydrochloric acid, (+)-L-lactic acid, ($\pm$)-DL-lactic acid, lactobionic acid, maleic acid, (-)-L-malic acid, malonic acid, ($\pm$)-DL-mandelic acid, methanesulfonic acid, naphthalene-2-sulfonic acid, naphthalene-1,5-disulfonic acid, 1-hydroxy-2-naphthoic acid, nicotinc acid, nitric acid, oleic acid, orotic acid, oxalic acid, palmitric acid, pamoic acid, phosphoric acid, L-pyroglutamic acid, salicyllc acid, 4-amino-salicylic acid, sebaic acid, stearic acid, succinic acid, sulfuric acid, tannic acid, (+)-L-tartaric acid, thiocyanic acid, p-toluenesulfonic acid and undedylenic acid; and

bases including ammonia, L-arginine, benethamine, benzathine, calcium hydroxide, choline, deanol, diethanolamine, diethylamine, 2-(diethylamino)-ethanol, ethanolamine, ethylenediamine, N-methyl-glucamine, hydrabamine, 1H-imidazole, L-lysine, magnesium hydroxide, 4-(2-hydroxyethyl)-morpholine, piperazine, potassium hydroxide, 1-(2-hydroxyethyl)-pyrrolidine, secondary amine, sodium hydroxide, triethanolamine, tromethamine and zinc hydroxide.

The present invention further comprises pharmaceutical compositions containing one or more compounds of formula (III) with a pharmaceutically acceptable carrier. Pharmaceutical compositions containing one or more of the compounds of the invention described herein as the active ingredient can be prepared by intimately mixing the compound or compounds with a pharmaceutical carrier according to conventional pharmaceutical compounding techniques. The

carrier may take a wide variety of forms depending upon the desired route of administration (e.g., oral, parenteral). Thus for liquid oral preparations such as suspensions, elixirs and solutions, suitable carriers and additives include water, glycols, oils, alcohols, flavoring agents, preservatives, stabilizers, coloring agents and the like; for solid oral preparations, such as powders, capsules and tablets, suitable carriers and additives include starches, sugars, diluents, granulating agents, lubricants, binders, disintegrating agents and the like. Solid oral preparations may also be coated with substances such as sugars or be enteric-coated so as to modulate major site of absorption. For parenteral administration, the carrier will usually consist of sterile water and other ingredients may be added to increase solubility or preservation. Injectable suspensions or solutions may also be prepared utilizing aqueous carriers along with appropriate additives.

To prepare the pharmaceutical compositions of this invention, one or more compounds of the present invention as the active ingredient is intimately admixed with a pharmaceutical carrier according to conventional pharmaceutical compounding techniques, which carrier may take a wide variety of forms depending of the form of preparation desired for administration, e.g., oral or parenteral such as intramuscular. In preparing the compositions in oral dosage form, any of the usual pharmaceutical media may be employed. Thus, for liquid oral preparations, such as for example, suspensions, elixirs and solutions, suitable carriers and additives include water, glycols, oils, alcohols, flavoring agents, preservatives, coloring agents and the like; for solid oral preparations such as, for example, powders, capsules, caplets, gelcaps and tablets, suitable carriers and additives include starches, sugars, diluents, granulating agents, lubricants, binders, disintegrating agents and the like. Because of their ease in administration, tablets and capsules represent the most advantageous oral dosage unit form, in which case solid pharmaceutical carriers are obviously employed. If desired, tablets may be sugar coated or enteric coated by standard techniques. For parenterals, the carrier will usually comprise sterile water, through other ingredients, for example, for purposes such as aiding solubility or for preservation, may be included. Injectable suspensions may also be prepared, in which case appropriate liquid carriers, suspending agents and the like may be employed. The pharmaceutical compositions herein will contain, per dosage unit, e.g., tablet, capsule, powder, injection, teaspoonful and the like, an amount of the active ingredient necessary to deliver an effective dose as described above. The pharmaceutical compositions herein will contain, per unit dosage unit, e.g., tablet, capsule, powder, injection, suppository, teaspoonful and the like, of from about 0.1-1000 mg and may be given at a dosage of from about 0.1-500 mg/kg/day. The dosages, however, may be varied depending upon the requirement of the patients, the severity of the condition being treated and the compound being employed. The use of either daily administration or post-periodic dosing may be employed.

Preferably these compositions are in unit dosage forms from such as tablets, pills, capsules, powders, granules, sterile parenteral solutions or suspensions, metered aerosol or liquid sprays, drops, ampoules, autoinjector devices or suppositories; for oral parenteral, intranasal, sublingual or rectal administration, or for administration by inhalation or insufflation. Alternatively, the composition may be presented in a form suitable for once-weekly or once-monthly administration; for example, an insoluble salt of the active compound, such as the decanoate salt, may be adapted to provide a depot preparation for intramuscular injection. For preparing solid compositions such as tablets, the principal active ingredient is mixed with a pharmaceutical carrier, e.g. conventional tableting ingredients such as corn starch, lactose, sucrose, sorbitol, talc, stearic acid, magnesium stearate, dicalcium phosphate or gums, and other pharmaceutical diluents, e.g. water, to form a solid preformulation composition containing a homogeneous mixture of a compound of the present invention, or a pharmaceutically acceptable salt thereof. When referring to these preformulation compositions as homogeneous, it is meant that the active ingredient is dispersed evenly throughout the composition so that the composition may be readily subdivided into equally effective dosage forms such as tablets, pills and capsules. This solid preformulation composition is then subdivided into unit dosage forms of the type described above containing from 0.1 to about 1000 mg, preferably, from about 0.1 to about 500 mg, of the active ingredient of the present invention. The tablets or pills of the novel composition can be coated or otherwise compounded to provide a dosage form affording the advantage of prolonged action. For example, the tablet or pill can comprise an inner dosage and an outer dosage component, the latter being in the form of an envelope over the former. The two components can be separated by an enteric layer which serves to resist disintegration in the stomach and permits the inner component to pass intact into the duodenum or to be delayed in release. A variety of material can be used for such enteric layers or coatings, such materials including a number of polymeric acids with such materials as shellac, cetyl alcohol and cellulose acetate.

The liquid forms in which the novel compositions of the present invention may be incorporated for administration orally or by injection include, aqueous solutions, suitably flavoured syrups, aqueous or oil suspensions, and flavoured emulsions with edible oils such as cottonseed oil, sesame oil, coconut oil or peanut oil, as well as elixirs and similar pharmaceutical vehicles. Suitable dispersing or suspending agents for aqueous suspensions, include synthetic and natural gums such as tragacanth, acacia, alginate, dextran, sodium carboxymethylcellulose, methylcellulose, polyvinylpyrrolidone or gelatin.

The method of treating central nervous system disorders described in the present invention may also be carried out using a pharmaceutical composition comprising any of the compounds as defined herein and a pharmaceutically acceptable carrier. The pharmaceutical composition may contain between about 0.1 mg and 1000 mg, preferably about 50 to 500 mg, of the compound, and may be constituted into any form suitable for the mode of administration selected.

Carriers include necessary and inert pharmaceutical excipients, including, but not limited to, binders, suspending agents, lubricants, flavorants, sweeteners, preservatives, dyes, and coatings. Compositions suitable for oral administration include solid forms, such as pills, tablets, caplets, capsules (each including immediate release, timed release and sustained release formulations), granules, and powders, and liquid forms, such as solutions, syrups, elixirs, emulsions, and suspensions. Forms useful for parenteral administration include sterile solutions, emulsions and suspensions.

Advantageously, one or more of the compounds of the present invention may be administered in a single daily dose, or the total daily dosage may be administered in divided doses of two, three or four times daily. Furthermore, compounds for the present invention can be administered in intranasal form via topical use of suitable intranasal vehicles, or via transdermal skin patches well known to those of ordinary skill in that art. To be administered in the form of a transdermal delivery system, the dosage administration will, of course, be continuous rather than intermittent throughout the dosage regimen.

For instance, for oral administration in the form of a tablet or capsule, the active drug component can be combined with an oral, non-toxic pharmaceutically acceptable inert carrier such as ethanol, glycerol, water and the like. Moreover, when desired or necessary, suitable binders; lubricants, disintegrating agents and coloring agents can also be incorporated into the mixture. Suitable binders include, without limitation, starch, gelatin, natural sugars such as glucose or betalactose, corn sweeteners, natural and synthetic gums such as acacia, tragacanth or sodium oleate, sodium stearate, magnesium stearate, sodium benzoate, sodium acetate, sodium chloride and the like. Disintegrators include, without limitation, starch, methyl cellulose, agar, bentonite, xanthan gum and the like.

The liquid forms in suitably flavored suspending or dispersing agents such as the synthetic and natural gums, for example, tragacanth, acacia, methylcellulose and the like. For parenteral administration, sterile suspensions and solutions are desired. Isotonic preparations which generally contain suitable preservatives are employed when intravenous administration is desired.

The compounds of the present invention can also be administered in the form of liposome delivery systems, such as small unilamellar vesicles, large unilamellar vesicles, and multilamellar vesicles. Liposomes can be formed from a variety of phospholipids, such as cholesterol, stearylamine or phophatidylcholines.

Compounds of the present invention may also be delivered by the use of monoclonal antibodies as individual carriers to which the compound molecules are coupled. The compounds of the present invention may also be coupled with soluble polymers as targetable drug carriers. Such polymers can include polyvinylpyrrolidone, pyran copolymer, polyhydroxypropylmethacrylamidephenol, polyhydroxy-ethylaspartamidephenol, or polyethyl eneoxidepolylysine substituted with palmitoyl residue. Furthermore, the compounds of the present invention may be coupled to a class of biodegradable polymers useful in achieving controlled release of a drug, for example, polylactic acid, polyepsilon caprolactone, polyhydroxy butyeric acid, polyorthoesters, polyacetals, polydihydropyrans, polycyanoacrylates and cross-linked or amphipathic block copolymers of hydrogels.

Compounds of this invention may be administered in any of the foregoing compositions and according to dosage regimens established in the art whenever treatment of disorders of the central nervous system is required.

The daily dosage of the products may be varied over a wide range from 0.01 to 1,000 mg per adult human per day. For oral administration, the compositions are preferably provided in the form of tablets containing, 0.01, 0.05, 0.1, 0.5, 1.0, 2.5, 5.0, 10.0, 15.0, 25.0, 50.0, 100, 150, 200, 250, 500 and 1000 milligrams of the active ingredient for the symptomatic adjustment of the dosage to the patient to be treated. An effective amount of the drug is ordinarily supplied at a dosage level of from about 0.01 mg/kg to about 1000 mg/kg of body weight per day. Preferably, the range is from about 0.5 to about 500 mg/kg of body weight per day, most preferably, from about 1.0 to about 250 mg/kg of body weight per day. The compounds may be administered on a regimen of 1 to 4 times per day.

Optimal dosages to be administered may be readily determined by those skilled in the art, and will vary with the particular compound used, the mode of administration, the strength of the preparation, the mode of administration, and the advancement of the disease condition. In addition, factors associated with the particular patient being treated, including patient age, weight, diet and time of administration, will result in the need to adjust dosages.

Compounds of formula (III) wherein d is 1, may be prepared according to the process outlined in Scheme 5.

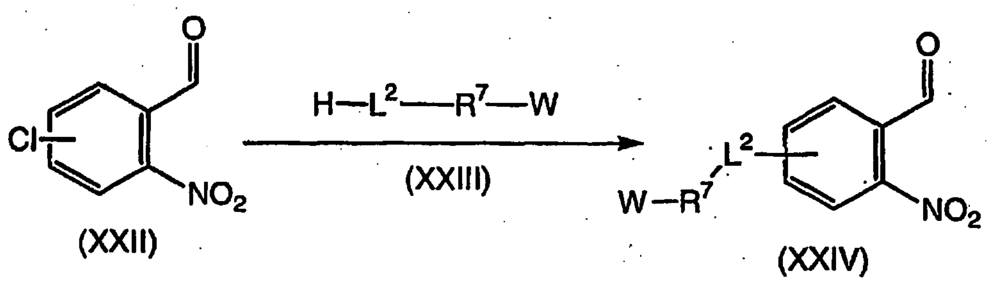


(XXII) (XXIV)

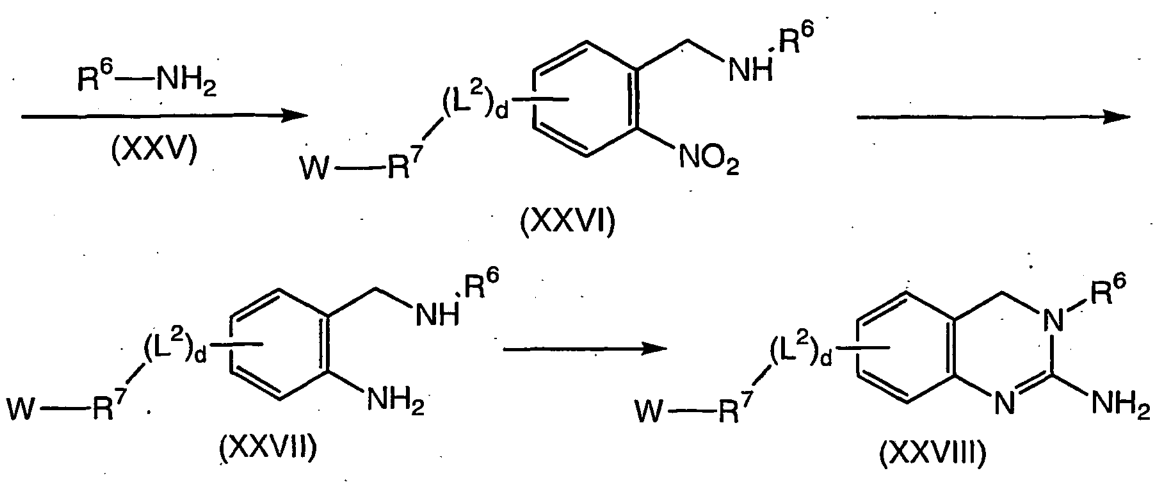


(XXVI)

(XXVII)

(XXVIII)

Scheme 5

Accordingly, a suitably substituted compound of formula (XXII), a known compound or compound prepared by known methods, is reacted with a suitably substituted compound of formula (XXIII), a known compound or compound prepared by known methods, wherein W is selected from $-L^3-R^8$ or a reactive group such as $NH_2$, CN, Br, OH and the like; in the presence of a base such as $K_2CO_3$, $Na_2CO_3$, $Cs_2CO_3$, TEA, DIPEA, pyridine, and the like, in a polar organic solvent such as DMF, DMA, and the like, at an elevated temperature in the range of from about 70°C to about 150°C, preferably at an elevated temperature in the range of about 80°C to about 100°C, to yield the corresponding compound of formula (XXIV).

One skilled in the art will recognize that in the process step described above, in the compound of formula (XXII), the Cl may be replaced with F and reacted as decribed above, to yield the compound of formula (XXIV).

The compound of formula (XXIV) is reacted with a suitably substituted compound of formula (XXV), a known compound or compound prepared by known methods, in an organic solvent such as DCE, DCM, THF, and the like, in the presence of a reducing agent such as $BH(OAc)_3$, $NaCNBH_3$, and the like, to yield the corresponding compound of formula (XXVI).

Alternatively, the compound of formula (XXII) (or the compound of formula (XXII) wherein the Cl is replaced with F) is protected, according to known methods, for example as an acetal of the formula (XXIX)

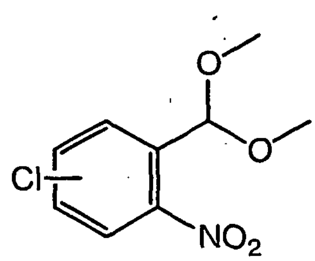

(XXIX)

and then reacted with a suitably substituted compound of formula (XXIII) to yield the corresponding protected version of the compound of formula (XXIV), which is the de-protected and reacted with the compound of formula (XXV), to yield the corresponding compound of formula (XXVI).

The compound of formula (XXVI) is reacted with hydrogen gas, in the presence of a catalyst such as Pd on carbon (Pd/C), and the like, in a protic solvent such as methanol, ethanol, and the like, to yield the corresponding compound of formula (XXVII). Alternatively, the compound of formula (XXVI) is reacted with a reducing agent such as stannous chloride, and the like, in an organic solvent such as methanol, ethanol, ethyl acetate, THF and the like, or in acid such as concentrated HCl, and the like; or with a reducing agent such as zinc, in the presence of an acid source such as ammonium chloride, calcium chloride, HBr, and the like, in an organic solvent such as methanol, ethanol, ethyl acetate, and the like, or in a mixture of an organic solvent and water as a co-solvent, or in aqueous acid such as acetic acid, and the like, to yield the corresponding compound of formula (XXVII).

The compound of formula (XXVII) is reacted with cyanogen bromide, and the like, in an organic solvent such as methanol, ethanol, toluene, and the like, to yield the corresponding compound of formula (XXVIII). Alternatively, the compound of formula (XXVII) is reacted with 2-methyl-2-thiopseudourea, in the presence of an acid such as hydrochloric acid, sulfuric acid, and the like, in an organic solvent such as butanol, and the like, to yield the corresponding compound of formula (XXVIII).

One skilled in the art will recognize that wherein W is $-L^3-R^2$, the compound of formula (XXVIII) is the corresponding compound of formula (III).

Wherein the compound of formula (XXVIII), W is other than $-L^3-R^2$, the compound of formula (XXVIII) is further reacted with according to known methods to yield the corresponding compound of formula (III).

For example, wherein W is $NH_2$, the compound of formula (XXVIII) is reacted with a suitably substituted aldehyde or cyclic ketone, for example an aldehyde, a compound of the formula $R^8$-CHO or a cyclic ketone such as cyclohexanone, to yield the corresponding compound of formula (III) wherein $L^3$ is -NH-.

Alternatively, wherein W is $NH_2$, the compound of formula (XXVIII) is reacted with a suitably substituted acid, a compound of the formula $R^8$-C(O)-OH, according to known methods, to yield the corresponding compound of formula (III) wherein $L^3$ is -NH-C(O)-.

Alternatively still, wherein W is $NH_2$, the compound of formula (XXVIII) is reacted with a suitably substituted sulfonyl chloride, a compound of the formula $R^8-SO_2-Cl$, or a suitably substituted acid chloride, a compound of the formula $R^8$-C(O)-Cl, according to known methods, to yield the corresponding compound of formula (III) wherein $L^3$ is $-NH-SO_2-$ or -NH-C(O)-, respectively.

Alternatively still, wherein W is CN, the cyano group on the compound of formula (XXVIII) is reduced according to known methods, to yield the corresponding amine and then further functionalized according to known methods, for example, as described above.

Compounds of formula (III) wherein d is 1 may alternatively be prepared according to the process outlined in Scheme 6.

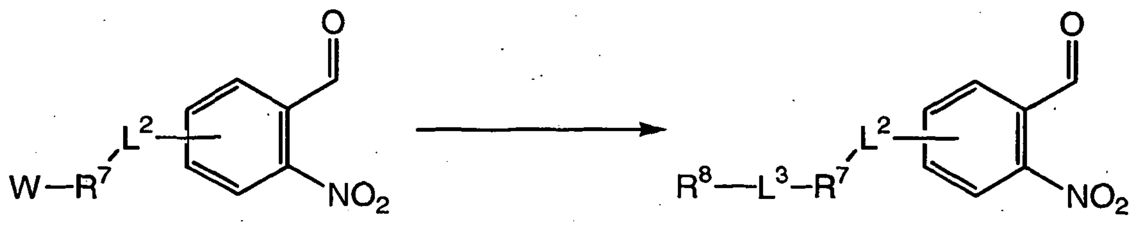

(XXIVa) (XXX)

Scheme 6

Accordingly, a suitably substituted compound of formula (XXIVa), a compound of formula (XXIV) wherein W is a reactive group such as CN, $NH_2$, Br, OH, and the like, is reacted according to known methods, to yield the corresponding compound of formula (XXX).

For example, wherein W is $NH_2$, the compound of formula (XXIVa) is reacted with a suitably substituted aldehyde or cyclic ketone, for example an aldehyde, a compound of the formula $R^8$-CHO or a cyclic ketone such as cyclohexanone, to yield the corresponding compound of formula (XXX) wherein $L^3$ is -NH-.

Alternatively, wherein W is $NH_2$, the compound of formula (XXIVa) is reacted with a suitably substituted acid, a compound of the formula $R^8$-C(O)-OH, according to known methods, to yield the corresponding compound of formula (XXX) wherein $L^3$ is -NH-C(O)-.

Alternatively still, wherein W is $NH_2$, the compound of formula (XXIVa) is reacted with a suitably substituted sulfonyl chloride, a compound of the formula $R^8-SO_2-Cl$, or a suitably substituted acid chloride, a compound of the formula $R^8$-C(O)-Cl, according to known methods, to yield the corresponding compound of formula (XXX) wherein $L^3$ is $-NH-SO_2-$ or -NH-C(O)-, respectively.

Alternatively still, wherein W is CN, the cyano group on the compound of formula (XXIVa) is reduced according to known methods, to yield the corresponding amine and then further functionalized according to known methods, for example, as described above.

Alternatively still, wherein W is Br, the compound of formula (XXIVa) is reacted with a suitably substituted amine, a compound of the formula $R^8-NH_2$, or a compound of the formula $R^8-NH-R^B$, according to known methods, to yield the corresponding compound of formula (XXX) wherein $L^3$ is $-NH-R^8$ or $R^8-(R^B)N-$, respectively.

Alternatively still, wherein W is OH, the compound of formula (XXIVa) is converted to the corresponding triflate and then reacted with a suitably substituted amine, a compound of the formula $R^8-NH_2$, or of a compound of the formula

$R^8$-NH-$R^B$, to yield the corresponding compound of formula (XXX) wherein $L^3$ is -NH-$R^3$ or $R^8$-($R^B$)N-, respectively.

Wherein the compound of formula (XXIV), W is Br or OH, preferably the compound of formula (XXIV) is reacted according to the process outlined in Scheme 6.

The compound of formula (XXX) is then substituted for the compound of formula (XXIV) in the process outlined in Scheme 5 above, and reacted as disclosed in Scheme 5 above, to yield the corresponding compound of formula (III).

Compounds of formula (III) wherein d is 0 may be prepared according to the processes outlined in Schemes 5 and 6 above by substituting a suitably substituted compound of formula (XXXI)

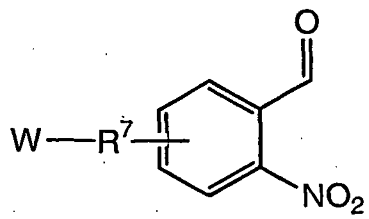

(XXXI)

for the compound of formula (XXIV), a known compound or compound prepared by known methods. For example, the compound of formula (XXXI) may be prepared according to the process outlined in Scheme 7.

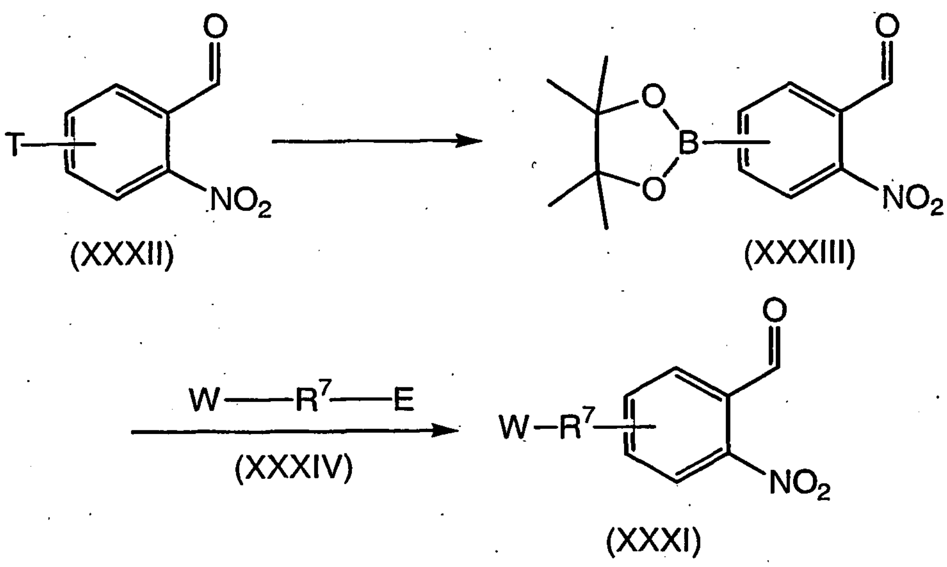


(XXXII) (XXXIII) (XXXI)

Scheme 7

Accordingly, a suitably substituted compound of formula (XXXII), wherein T is selected from Cl, Br or I, a known compound or compound prepared by known methods, is reacted with pinacol diborane (also known as 4,4,5,5,4',4',5',5'-octamethyl-[2,2']bi[[1,3,2]dioxaborolanyl]), a known compound, in the presence of a catalyst such as Pd(dppf)$Cl_2$, $Pd_2(dba)_3$, and the like, with a base such as potassium carbonate, potassium acetate, and the like, in an organic solvent, such as DMSO, DME, and the like, in the absence or presence of an aqueous co-solvent, such as water, at an elevated temperature in the range of from about 50°C to about 130°C, preferably at an elevated temperature in the range of from about 80°C to about 110°C, to yield the corresponding compound of formula (XXXIII).

The compound of formula (XXXIII) is reacted with a suitably substituted compound of formula (XXXIV), wherein E is Br or I, and wherein W is -$L^3$-$R^8$ or a suitable reactive group such as $NH_2$, CN, Br, OH, and the like, a known compound or compound prepared by known methods, (for example as disclosed in Mlyaura, N.; Suzuki, A. Chem. Rev. 1995, 95, 2457; Suzuki, A. J. Organomet. Chem. 1999, 576, 147), in the presence of a catalyst such $Pd(PPh_3)_4$. Pd (dppf)$Cl_2$, and the like, in the presence of a base such as $Na_2CO_3$, $K_2CO_3$, $NaHCO_3$, and the like, in a mixture of an organic solvent such as toluene, DME, THF, MeOH, and the like, and a protic solvent such as water, and the like, at an elevated temperature in the range of from about 60°C to about 150°C) preferably, at an elevated temperature in the range of from about 100°C to about 120°C, optionally in the presence of microwave irradiation, to yield the corresponding

compound of formula (XXXI).

The following Examples are set forth to aid in the understanding of the invention, and are not intended and should not be construed to limit in any way the invention set forth in the claims which follow thereafter.

In the Examples which follow, some synthesis products are listed as having been isolated as a residue. It will be understood by one of ordinary skill in the art that the term "residue" does not limit the physical state in which the product was isolated and may include, for example, a solid, an oil, a foam, a gum, a syrup, and the like.

**Example 1**

**3-(3-Dimethoxymethyl-4-nitro-phenoxy)-phenylamin**

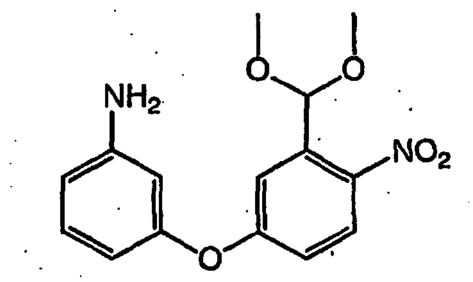

3-Aminophenol (0.050 mol) was dissolved in DMA (100 mL). $K_2CO_3$ (0.060 mol) was added. 4-Chloro-2-dimethoxymethyl-1-nitrobenzene (0.050 mol) was added, and the reaction mixture was stirred overnight at 100˚C and then for 2 hours at 120˚C. The reaction mixture was poured into water and this mixture was extracted with diisopropyl ether. The separated organic layer was dried, filtered and the solvent evaporated. The residue was purified by column chromatography over silica gel (eluent: $CH_2Cl_2$:$CH_3OH$ 100:0 (to remove unreacted 4-chloro-2-dimethoxymethyl-1-nitrobenzene) up to 70:30). The desired fractions were collected and the solvent was evaporated to yield a residue.

**Example 2**

**N-[3-(3-Dimethoxymethyl-4-nitro-phenoxy)-phenyl]-benzenesulfonamide**

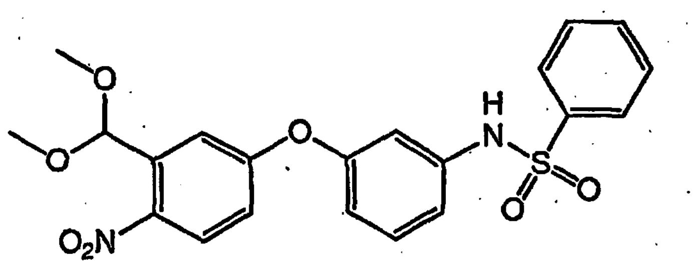

A mixture of 3-(3-dimethoxymethyl-4-nitro-phenoxy)phonylamine (0.013 mol) in THF (100 mL) and TEA (200 mL) was stirred at room temperature and a mixture of benzenesulfonyl chloride (0.013 mol) in THF (50 mL) was added dropwise, then the reaction mixture was stirred overnight at room temperature and again overnight at 60˚C. The solvent was evaporated and the residue was stirred in $H_2O$. After extraction with $CH_2Cl_2$, the organic layer was separated, dried, filtered off and the solvent was evaporated. The residue was purified by Biotage column chromatography (eluent: $CH_2Cl_2$ 100%). The purest product fractions were collected and the solvent was evaporated to yield the title compound as a residue.

**Example 3**

**N-[3-(3-Formyl-4-nitro-phenoxy)-henyl]-benzenesulfonamide**

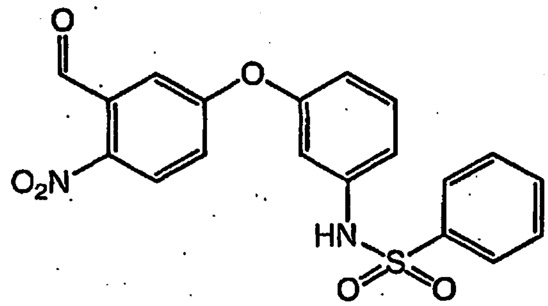

A mixture of N-[3-(3-dimethoxymethyl-4-nitrophenoxy)-phenyl]-benzenesulfonamide (0.0083 mol) in THF (12 mL) was stirred at room temperature, then 12*N* HCl (6 mL) and water (6 mL) were added and the reaction mixture was stirred for 48 hours at room temperature. The mixture was stirred in $H_2O$ and extracted with diisopropyl ether. The organic layer was separated, washed with a 10% $NaHCO_3$ solution, separated again, dried, filtered, and the solvent was evaporated. The obtained residue was stirred in diisopropyl ether, and then the desired product was filtered off and dried to yield the title compound as a solid.

**Example 4**

**N-[3-(4-Nitro-3-propylaminomethyl-phenoxy)-phenyl]-benzenesulfonamide**

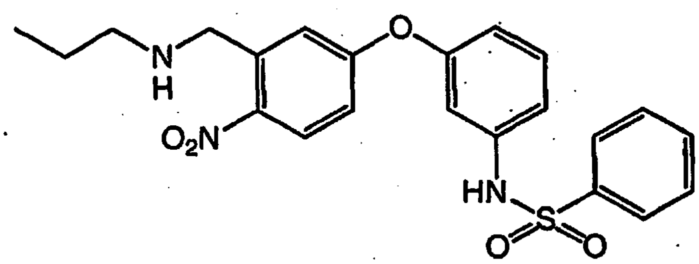

Under $N_2$, a mixture of N-[3-(3-formyl-4-nitrophenoxy)-phenyl]-benzenesulfonamide (0.0060 mol) and propylamine (0.0085 mol) in DCE (50 mL) was stirred at room temperature, then $NaBH(OAc)_3$.(0.0080 mol) was added and the reaction mixture was stirred overnight at room temperature. A saturated $NaHCO_3$ solution (50 mL) was added and the layers were separated. The organic layer was dried, filtered off and the solvent was evaporated. The residue was purified over silica gel filter (eluent: 98:2 $CH_2Cl_2$:$CH_3OH$). The purest product fractions were collected and the solvent was evaporated to yield the title compound as a residue.

**Example 5**

**N-[3-(4-Amino-3-propylaminomethyl-phenoxy)-phenyl]-benzenesulfonamide**

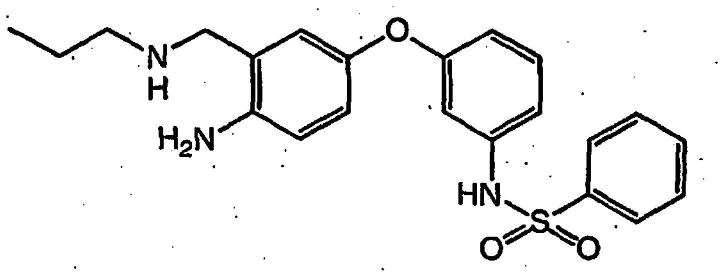

A mixture of N-[3-(4-nitro-3-propylaminomethyl-phenoxy)-phenyl]-benzenesulfonamide (0.0045 mol) in methanol (100 mL) was hydrogenated with 10% Pd/C (0.5 g) as a catalyst in the presence of thiophene solution (1 mL). After uptake of $H_2$ gas (3 equiv.), the reaction mixture was filtered over Dicalite and the filtrate was evaporated to yield the title compound as a residue.

**Example 6**

**N-[3-(2-Amino-3-propyl-3,4-dihydro-quinazolin-6-yloxy)-phenyl]-benzenesulfonamide**

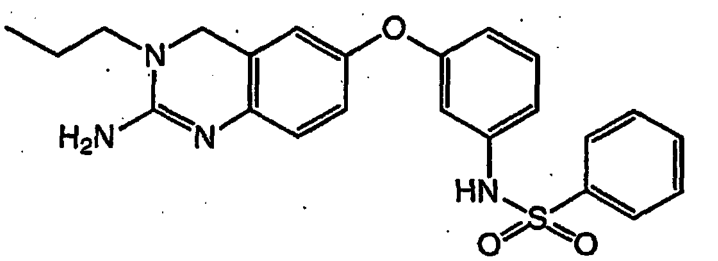

A mixture of N-[3-(4-amino-3-propylaminomethyl-phenoxy)-phenyl]-benzenesulfonamide (0.0044 mol) in ethanol (50 mL) was stirred at room temperature and cyanogen bromide (0.0064 mol) was added, then the reaction mixture was stirred and refluxed for 4 hours. After cooling, the precipitate was filtered off and was stirred in boiling $CH_3CN$ with $CH_3OH$. The resulting solids were filtered off, washed with diisopropyl ether and dried to yield the title compound as a solid.

**Example 7**

**3-(3-Dimethoxymethyl-4-nitrophenoxy)-benzonitrile**

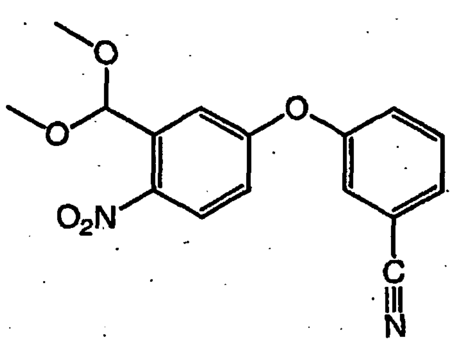

A mixture of 3-hydroxybenzonitrile (0.129 mol) in *N,N*-dimethyl-acetamide (200 mL) was stirred at room temperature and $K_2CO_3$ (0.147 mol) was added, then 4-chloro-2-dimethoxymethyl-1-nitrobenzene (0.086 mol) was added and the reaction mixture was stirred for 24 hours at 130˚C. The solvent was evaporated and the residue was stirred in $H_2O$. After extraction with $CH_2Cl_2$, the organic layer was separated, dried, filtered off and the solvent was evaporated.

The residue was purified over a silica gel filter (eluent: $CH_2Cl_2$ 100 %). The purest product fractions were collected and the solvent was evaporated to yield the title compound as residue.

**Example 8**

**3-(3-Formyl-4-nitrophenoxy)-benzonitrile**

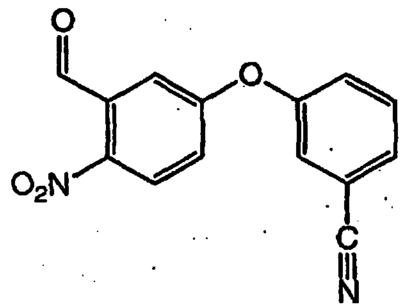

A mixture of 3-(3-dimethoxymethyl-4-nitro-phenoxy)-benzonitrile (0.068 mol) in water (55 mL) was stirred at room temperature, and then 12*N* HCl (55 mL) and THF (140 mL) were added, and the reaction mixture was stirred overnight at room temperature. The mixture was stirred in $H_2O$ and extracted with diisopropyl ether. The organic layer was separated, washed with a 10% $NaHCO_3$ solution, separated again, dried, filtered off and the solvent was evaporated. The obtained residue was stirred in diisopropyl ether, and then the desired product was filtered off and dried to yield the title compound as a solid.

**Example 9**

**3-(4-Nitro-3-propylaminomethyl-phenoxy)-benzonitrile**

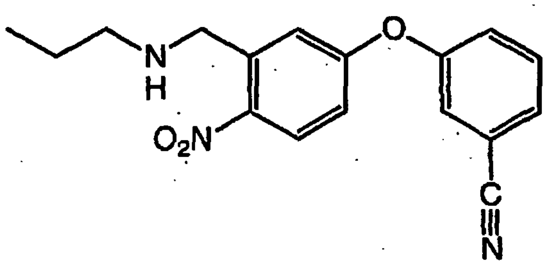

Under $N_2$, a mixture of 3-(3-formyl-4-nitrophenoxy)-benzonitrile (0.056 mol) and propylamine (0.059 mol) in DCE (450 mL) was stirred at room temperature, and then $NaBH(OAc)_3$ (0.063 mol) was added, and the reaction mixture was stirred overnight at room temperature. A saturated $NaHCO_3$ solution was added, and the layers were separated. The organic layer was dried, filtered off and the solvent was evaporated. The residue was purified over silica gel filter (eluent: 99:1 $CH_2Cl_2$:$CH_3OH$). The purest product fractions were collected and the solvent was evaporated to yield the title compound as a residue.

**Example 10**

**3-(4-Amino-3-propylaminomethyl-phenoxy)-benzonitrile**

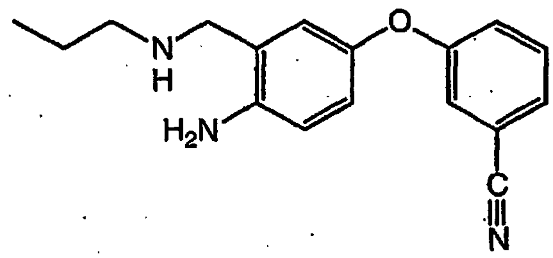

A mixture of 3-(4-nitro-3-propylaminomethyl-phenoxy)-benzonitrile (0.045 mol) in methanol (250 mL) was hydrogenated with 10% Pd/C (2 g) as a catalyst in the presence of thiophene solution (2 mL). After uptake of $H_2$ gas (3 equiv.), the reaction mixture was filtered over Dicalite and the filtrate was evaporated. The residue was purified by column chromatography over silica gel (eluent: 98:2 $CH_2Cl_2$:$CH_3OH$). The purest product fractions were collected and the solvent was evaporated to yield the title compound as a residue.

**Example 11**

**3-(2-Amino-3-propyl-3.4-dihydro-quinazoly-6-yloxy)-benzonitrile**

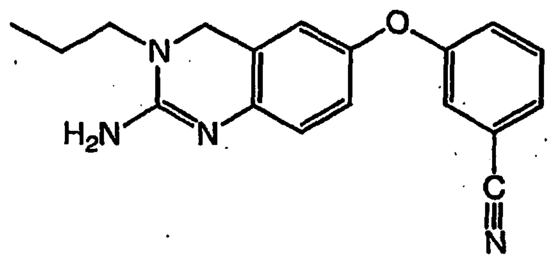

A mixture of 3-(4-amino-3-propylaminomethyl-phenoxy)-benzonitrile (0.032 mol) in ethanol (400 mL) was stirred at room temperature, and cyanogen bromide (0.038 mol) was added, and then the reaction mixture was stirred and refluxed for 2 hours. After cooling, the precipitate was filtered off, washed with diisopropyl ether and dried to yield crude product as a solid.

The filtrate was evaporated and the obtained residue was crystallized from $CH_3CN$:diisopropyl ether, then the precipitate was filtered off, washed with diisopropyl ether, and dried to yield an additional crop of the title compound as a solid.

**Example 12**

**[6-(3-Cyano-phenoxy)-3-aropyl-3,4-dihydro-quinazolin-2-yl]-carbamic acid *tert*-butyl ester**

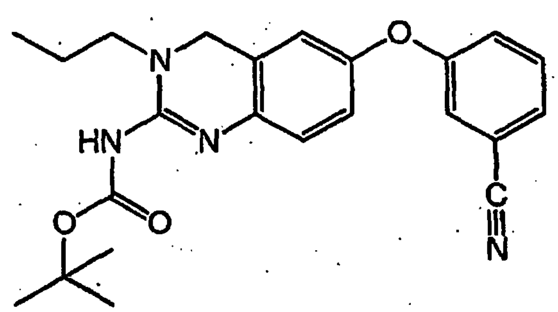

A mixture of 3-(2-amino-3-propyl-3,4-dihydro-quinazolin-6-yloxy)-benzonitrile (0.021 mol) and TEA (2.5 g) in THF (400 mL) was stirred at 5°C, then *t*-butoxycarbonyl anhydride (0.023 mol) was added, and the reaction mixture was stirred over the weekend at room temperature. The solvent was evaporated, then the residue was stirred in $H_2O$ and the mixture was extracted with $CH_2Cl_2$. The organic layer was separated, dried, filtered off and the solvent was evaporated. The residue was purified by column chromatography over silica gel (eluent: 99:1 $CH_2Cl_2$:$CH_3OH$). The purest product fractions were collected and the solvent was evaporated to yield the title compound as residue.

**Example 13**

**[6-(3-Aminomethyl-phenoxy)-3-propyl-3,4-dihydro-quinazolin-2-yl]-carbamic acid *tert*-butyl ester**

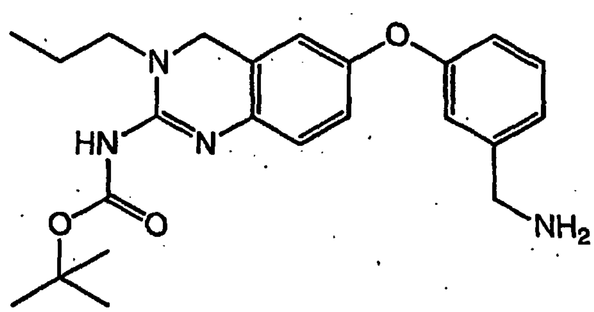

A mixture of [6-(3-cyano-phenoxy)-3-propyl-3,4-dihydro-quinazolin-2-yl]-carbamic acid *tert*-butyl ester (0.0197 mol) in a solution of ammonia in methanol (250 mL) was hydrogenated at 14°C with Raney nickel (1 g) as a catalyst. After uptake of $H_2$ gas (2 equiv.), the reaction mixture was filtered over Dicalite and the filtrate was evaporated to yield the title compound as a residue.

**Example 14**

**(3-Propyl-6-{3-[(2,4,6-trimethyl-benzylamino)-methyl]-phenoxy}-3,4-dihydro-quinazolin-2-yl)-carbamic acid *tert*-butyl ester**

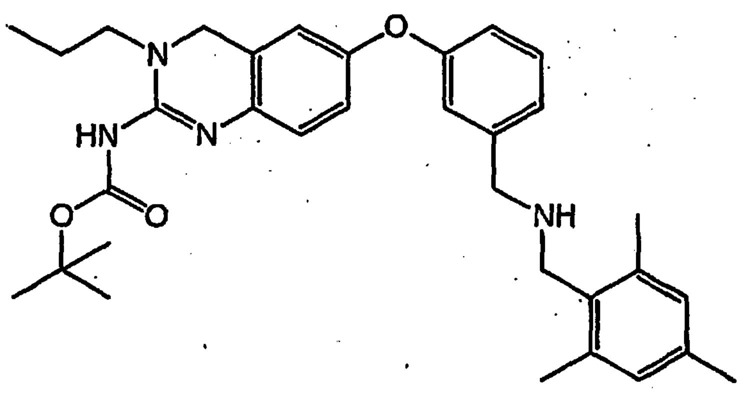

Under $N_2$, a mixture of [6-(3-aminomethyl-phenoxy)-3-propyl-3,4-dihydro-quinazolin-2-yl]-carbamic acid *tert*-butyl ester (0.0007 mol) and 2,4,6-trimethyl-benzaldehyde (0.0007 mol) in DCE (10 mL) was stirred at room temperature, then $NaBH(OAc)_3$ (0.2 g) was added, and the reaction mixture was stirred overnight at room temperature. A saturated $NaHCO_3$ solution was added, and the layers were separated. The organic layer was dried, filtered off and the solvent was evaporated. The residue was purified by Biotage column chromatography (gradient eluent: $CH_2Cl_2$:$CH_3OH$). The purest product fractions were collected and the solvent was evaporated to yield the title compound as a residue.

**Example 15**

**3-Pronyl-6-{3-[(2,4,6-trimethyl-benzylamino)-methyl]-phenoxy}-3,4-dihydro-quinazolin-2-ylamine**

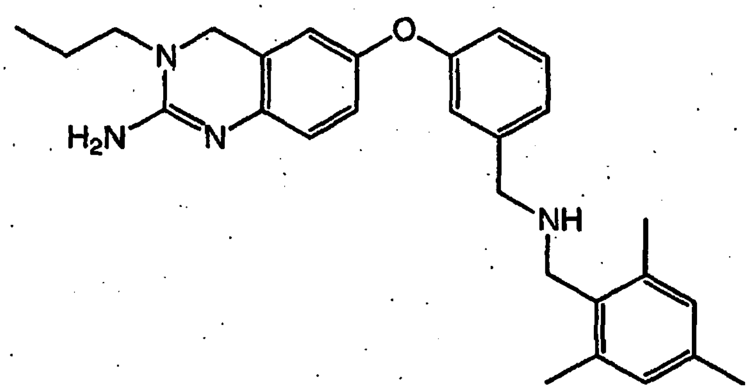

A mixture of (3-propyl-6-{3-[(2,4,6-trimethyl-benzylamino)-methyl]-phenoxy}-3,4-dihydro-quinazolin-2-yl)-carbamic acid *tert*-butyl ester (0.00041 mol) in trifluoroacetic acid (1 mL) and DCM (10 mL) was stirred overnight at room temperature and the solvent was evaporated. The obtained residue was decomposed in diisopropyl ether/$CH_3CN$, then the desired product was filtered off and dried to yield the title compound as a solid.
mp 184.5˚C

**Example 16**

**3-(3-Dimethoxymethyl-4-nitro-phenylsulfanyl)-phenylamine**

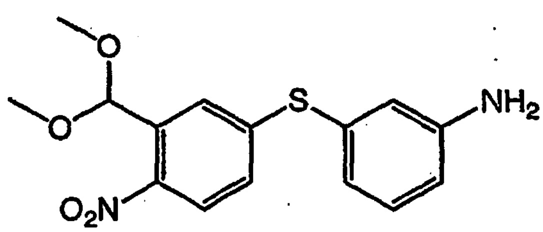

$K_2CO_3$ (0.085 mol) was added to a mixture of 3-aminothlophenol (0.075 mol) in DMA (150 mL), then 4-chloro-2-dimethoxymethyl-1-nitrobenzene (0.050 mol) was added and the reaction mixture was stirred for 2 hours at 80˚C. $H_2O$ was added and the mixture was extracted with diisopropyl ether. The crude product was purified by column chromatography (eluent: 70:30 $CH_2Cl_2$:hexane up to 99:1 $CH_2Cl_2$:$CH_3OH$). The product fractions were collected and the solvent was evaporated to yield the title compound as a residue which contained about 10% of an impurity. (The product was used in subsequent steps without further purification.)

**Example 17**

**N-[3-(3-Dimethoxymethyl-4-nitro-phenylsulfanyl)-phenyl]-benzenesulfonamide**

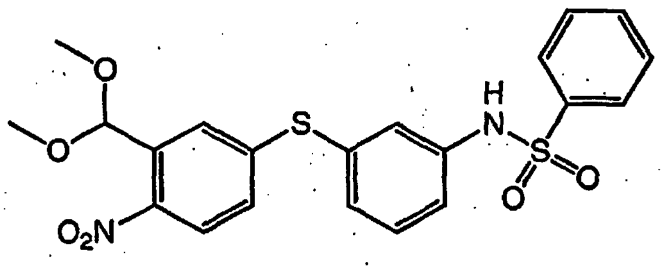

A mixture of 3-(3-dimethoxymethyl-4-nitro-phenylsulfanyl)-phenylamine (0.0384 mol) and TEA (0.0576 mol) In THF (q.s.) was stirred at 5˚C, and benzenesulfonyl chloride (0.0384 mol) was added dropwise at 5˚C, then the reaction mixture was reacted at room temperature and purified by column chromatography over silica gel (eluent: $CH_2Cl_2$). The product fractions were collected and the solvent was evaporated to yield the title compound as a residue. (The product was used In subsequent steps without further purification.)

**Example 18**

**N-[3-(3-Formyl-4-nitro-phenylsulfanyl)-phenyl]-benzenesulfonamide**

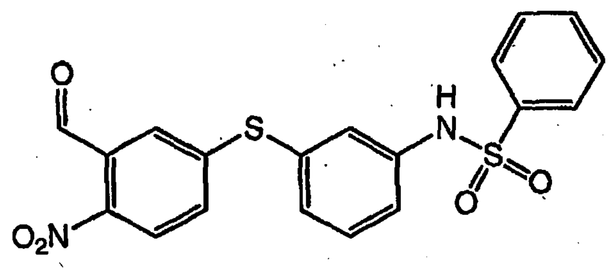

A mixture of N-[3-(3-dimethoxymethyl-4-nitro-phenylsulfanyl)-phenyl]-benzenesulfonamide (0.027 mol) In 12*N* HCl (25 mL); THF (75 mL) and water (50 mL) was stirred overnight at room temperature, and then the mixture was extracted with ethyl acetate. The extract was washed with water and with a saturated aqueous $Na_2CO_3$ solution. The solvent was evaporated and the residue was purified by column chromatography over silica gel (eluent $CH_2Cl_2$). The product fractions were collected and the solvent evaporated to yield the title compound as a residue.

**Example 19**

**N-[3-(4-Nitro-3-propylaminomethyl-phenylsulfanyl)-phenyl]-benzenesulfonamide**

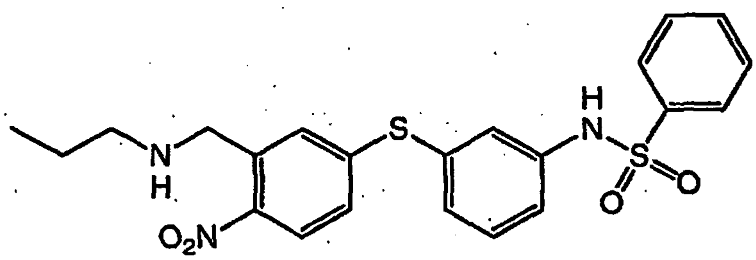

A mixture of N-[3-(3-formyl-4-nitro-phenylsulfanyl)-phenyl]-benzenesulfonamide (0.0027 mol), propylamine (0.003 mol) and $NaBH(OAc)_3$ (0.0041 mol) in DCE (60 mL) was reacted overnight at room temperature. Then a 10% NaOH solution was added and the mixture was extracted with $CH_2Cl_2$. The extract was dried ($MgSO_4$) and then purified by column chromatography over silica gel (eluent: 1:0 to 7:1 $CH_2Cl_2$:$CH_3OH$). The product fractions were collected and

the solvent was evaporated to yield the title compound as a residue.

**Example 20**

**N-[3-(4-Amino-3-propylaminomethyl-phenylsulfanyl)-phenyl]-benzenesulfonamide**

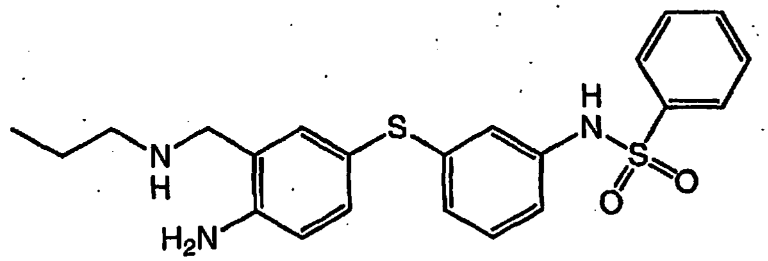

A mixture of N-[3-(4-nitro-3-propylaminomethyl-phenylsulfanyl)-phenyl]-benzenesulfonamide (0.0016 mol) In methanol (50 mL) was hydrogenated with 10% Pd/C (0.1 g) as a catalyst. After uptake of $H_2$ gas (3 equiv.), the catalyst was filtered off and the filtrate was evaporated to yield the title compound as a residue. (The product was used in subsequent steps without further purification.)

**Example 21**

**N-[3-(2-Amino-3-propyl-3,4-dihydro-quinazolin-6-ylsulfanyl)-phenyl]-benzenesulfonamide**

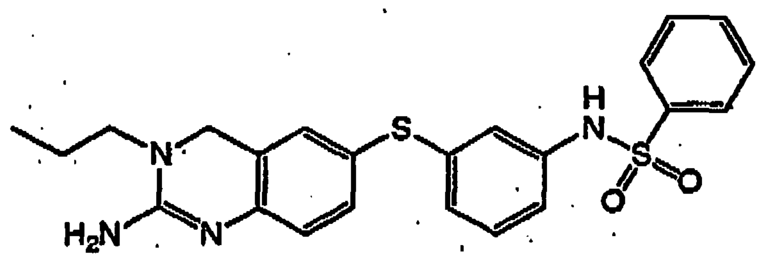

A mixture of N-[3-(4-Amino-3-propylaminomethyl-phenylsulfanyl)-phenyl]-benzonesulfonamide (0.0016 mol) and cyanogen bromide (0.0020 mol) in ethanol (q.s.) was reacted overnight at room temperature and then the organic solvent (EtOH) was evaporated. The obtained concentrate was warmed in EtOH and then cooled. The mixture was filtered and the collected residue was recrystallized from EtOH to yield the title compound as a solid,
mp 196.8-274°C

**Example 21A**

**N-[2-(2-Bromo-phenyl)-ethyl]-benzenesulfonamide**

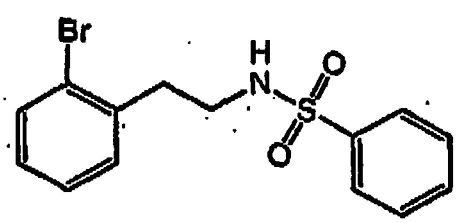

Benzenesulfonyl chloride (0.011 mol) was slowly added dropwise at room temperature to a mixture of 2-bromophenylethylamine (0.01 mol) and TEA (0.013 mol) In THF (50 mL). Then the reaction mixture was stirred overnight at room temperature, and the solvent was evaporated. The obtained residue was washed with $H_2O$ and extracted with $CH_2Cl_2$. The organic layer was separated, dried ($MgSO_4$), filtered off and the solvent was evaporated. The residue was stirred In diethyl ether, then the desired product was filtered off and dried (vac.) to yield the title compound as an oil.

**Example 21B**

**2-Nitro-5-(4,4,5,5-tetramethyl-[1,3,2]dioxaborolan-2-yl)-benzaldehyde**

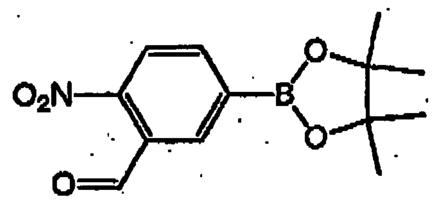

A mixture of $Pd_2(dba)_3$ (0,0007 mol) and tricyclohexylphosphine (0.0029 mol) in dry dioxane (200 mL) was stirred under $N_2$ for 30 min,and then 2-nitro-4-bromobenzaidehyde (0.040 mol), pinacol diborane (4,4,4',4',5,5,5',5'-octamethyl-2,2'-bi-1,3,2-dioxaborolane) (0.044 mol), and potassium acetate (0.060 mol) were added and the reaction mixture was stirred for 16 hours under $N_2$ at 80°C, The mixture was cooled to room temperature and the solvent was evaporated. The obtained residue was washed with $H_2O$ and extracted with $CH_2Cl_2$. The organic layer was separated, dried ($MgSO_4$), filtered off and the solvent was evaporated. The residue was purified by short column chromatography over silica gel (eluent: 100% $CH_2Cl_2$). The purest product fractions were collected and the solvent was evaporated. The residue was stirred in hexane, and then the resulting precipitate was filtered off and dried to yield the title compound as a residue.

**Example 21C**

**N-[2-(3'-Formyl-4'-nitro-biphenyl-2-yl)-ethyl]-benzenesulfonemide**

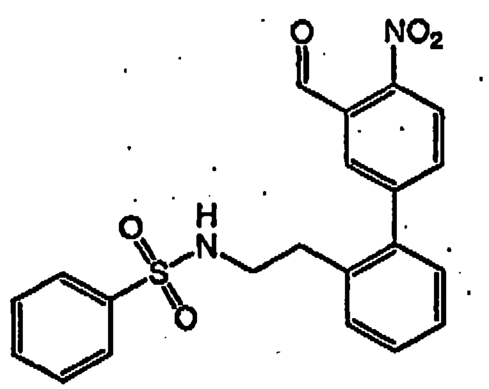

A mixture of 2-nitro-5-(4,4,5,5-tetramethyl-[1,3,2]dioxaborolan-2-yl)-benzaldehyde (0.001 mol), N-[2-(2-bromo-phenyl)-ethyl]-benzenesulfonamide (0.0012 mol) and $Pd(PCy_3)_2Cl_2$ (0.00005 mol) in DME (5 mL) and 1 *M* $Na_2CO_3$ (2 mL) in a reaction vial was stirred for 3 hours at 80°C, then DME was removed under a stream of $N_2$ and the resulting mixture was extracted with $CH_2Cl_2$. The mixture was filtered through Extrelut, and the organic layer was blown dry, then the desired product was isolated by FAST-synthesis to yield the title compound as a residue.

**Example 21D**

**N-[2-(4'-Nitro-3'-propylaminomethyl-biphenyl-2-yl)-ethyl]-benzenesulfonamide**

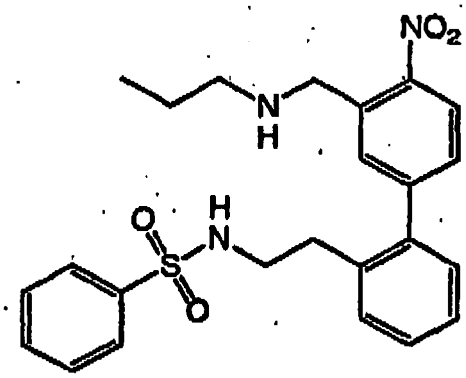

$NaBH(OAc)_3$ (0.0031 mol) was added to a mixture of N-[2-(3'-formyl-4'-nitro-biphenyl-2-yl)-ethyl-benzenesulfonamide (0.0021 mol) and propylamine (0.0023 mol) in DCE (2 mL), and the reaction mixture was stirred overnight under $N_2$. The excess of $NaBH(OAc)_3$ was decomposed with $CH_3OH$, and the solvent was evaporated. The residue was purified by Biotage column chromatography (eluent 95:5 $CH_2Cl_2$:$CH_3OH$). The purest product fractions were collected and then the solvent was evaporated and co-evaporated with toluene to yield the title compound as a residue.

**Example 21E**

**N-[2-(4'-Amino-3'-propylaminomethyl-biphenyl-2-yl)-ethyl]-benzenesulfonamide**

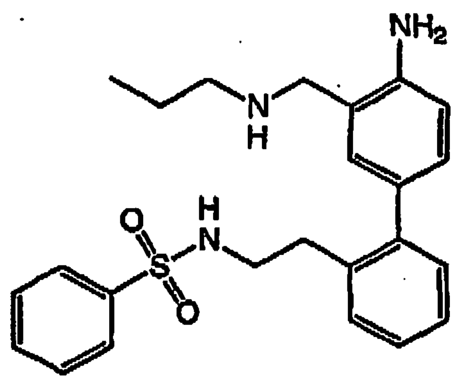

A mixture of N-[2-(4'-nitro-3'-propylaminomethyl-biphenyl-2-yl)-ethyl]-benzenesulfonamide (0.00031 mol) in methanol (50 mL) was hydrogenated with 10% Pd/C (0.100 g) as a catalyst in the presence of thiophene solution (0.5 mL). After uptake of $H_2$ gas (3 equiv.), the reaction mixture was filtered over Dicalite and the filtrate was evaporated to yield the title compound as a residue. (The compound was used without further purification, immediately in the next reaction step.)

**Example 21F**

**N-{2-[2-(2-Amino-3-propyl-3,4-dihydro-quinazolin-6-yl)-phenyl]-ethyl}-benzenesulfonamide**

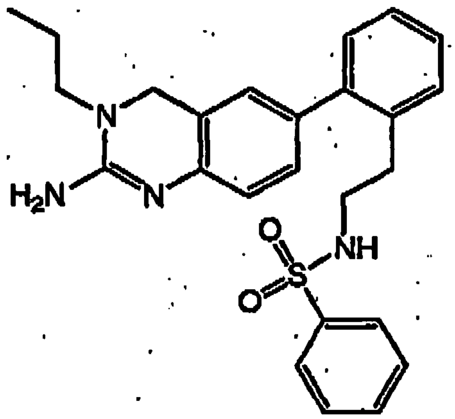

Cyanogen bromide (0.00034 mol) was added to a mixture of N-[2-(4'-amino-3'-propylaminomethyl-biphanyl-2-yl)-ethyl]-benzenesulfonamide (0.00031 mol) in ethanol (1 mL) in a Fast-tube, and then the reaction mixture was stirred overnight at room temperature. The solvent was evaporated and the residue was purified by high-performance liquid chromatography. The purest product fractions were collected and the solvent was evaporated to yield the title compound as a residue.

**Example 22**

**[3-(3-Dimethoxymethyl-4-nitro-phenoxy)-phenyl]-carbamic acid benzyl ester**

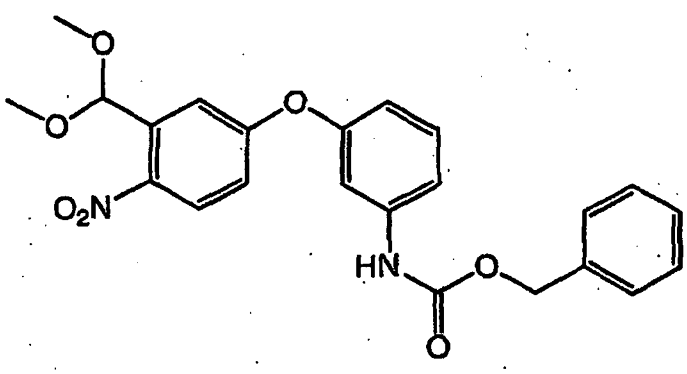

A mixture of 3-(3-dimethoxymethyl-4-nitro-phenoxy)-phenylamine (0.14 mol), prepared as In Example 65, and diisopropyl ether (0.16 mol) in DCM (1000 mL) was cooled to 10˚C, and carbonochloridic acid phenylmethyl ester (also known as benzyl chloroformate) (0.16 mol) was added dropwise, then the reaction mixture was stirred overnight at room temperature and washed with $H_2O$. The organic layer was separated, dried ($MgSO_4$), filtered off and the solvent was evaporated. The residue was purified by column chromatography (eluent: 100% $CH_2Cl_2$). The product fractions were collected and the solvent was evaporated to yield the title compound as a residue.

**Example 23**

**[3-(3-Formyl-4-nitro-phenoxy)-phenyl]-carbamic acid benzyl ester**

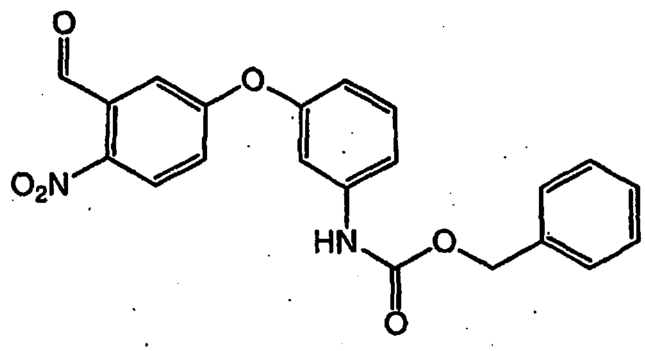

12*N* HCl (33 mL) and water (66 mL) were added to a mixture of [3-(3-dimethoxymethyl-4-nitro-phenoxy)-phenyl]-carbamic acid benzyl ester (0.14 mol) in THF (400 mL) and the reaction mixture was stirred for 48 hours at room temperature. The mixture was diluted with $H_2O$ (500 mL) and extracted 2 times with diisopropyl ether. The organic layer was separated, dried ($MgSO_4$), filtered off and the solvent was evaporated. The resulting product was removed with a spatula and dried under vacuum to yield the title compound as a residue.

**Example 24**

**[3-(4-Nitro-3-propylaminomethyl-phenoxy)phenyl]-carbamic acid benzyl ester**

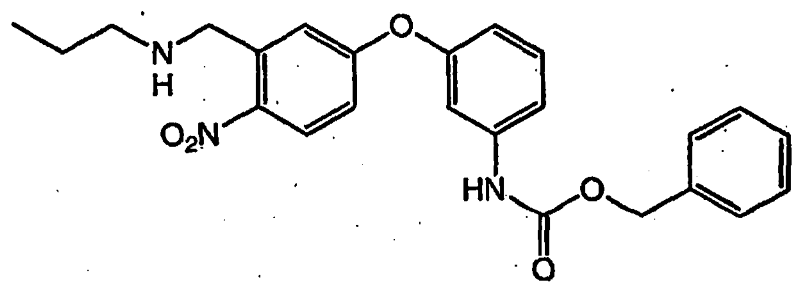

A mixture of [3-(3-formyl-4-nitro-phenoxy)-phenyl]-carbamic acid benzyl ester (0.038 mol) in DCE (300 mL) was cooled in an ice bath to 15°C, then propylamine (0.042 mol), followed by $NaBH(OAc)_3$ (0.057 mol) which was added portionwise. The reaction mixture was stirred overnight at room temperature and the mixture was washed with a saturated $NaHCO_3$ solution. The organic layer was separated, dried ($MgSO_4$), filtered off and the solvent was evaporated. The residue was purified by column chromatography over silica gel (eluent: 90:10 $CH_2Cl_2$:$CH_3OH$). The product fractions were collected and the solvent was evaporated, then co-evaporated with toluene to yield the title compound as a residue.

**Example 25**

**[3-(4-Amino-3-propylaminomethyl-phenoxy)-phenyl]-carbamic acid benzyl ester**

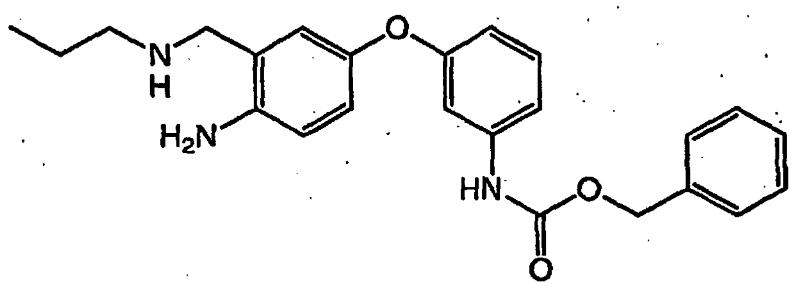

A mixture of [3-(4-nitro-3-propylaminomethyl-phenoxy)-phenyl]-carbamic acid benzyl ester (0.033 mol) in THF (200 mL) was hydrogenated with 10% Pt/C (2 g) as a catalyst in the presence of thiophene solution (2 mL). After uptake of $H_2$ gas (3 equiv.), the reaction mixture was filtered through Dicalite, and the filtrate was evaporated. The residue was purified by column chromatography (eluent: 90:10 $CH_2Cl_2$:($CH_3OH$:$NH_3$)). The product fractions were collected, and the solvent was evaporated to yield the title compound as an oil.

**Example 26**

**[3-(2-Amino-3-propyl-3,4-dihydro-quinazolin-6-yloxy)-phenyl]-carbamic acid benzyl ester**

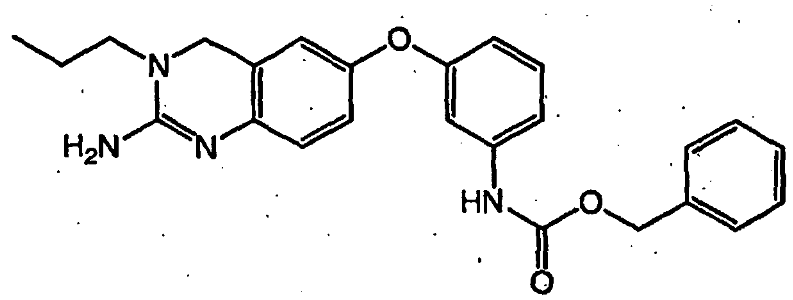

A mixture of [3-(4-Amino-3-propylaminomethyl-phenoxy)-phenyl]-carbamic acid benzyl ester (0.032 mol) and cyanogen bromide (0.035 mol) in ethanol (250 mL) was stirred and refluxed for 2 hours, then the reaction mixture was cooled to room temperature and the solvent was evaporated. The residue was converted into its free base with a NaOH solution and the resulting mixture was extracted with $CH_2Cl_2$. The organic layer was separated and the solvent was evaporated, then the residue was stirred in diisopropyl ether, filtered off and dried under vacuum to yield the title compound as a solid.

**Example 27**

**[6-(3-Benzvloxycarbonylamino-phenoxy)-3-propyl-3,4-dihydro-quinazolin-2-yl]-carbamic acid *tert*-butyl ester**

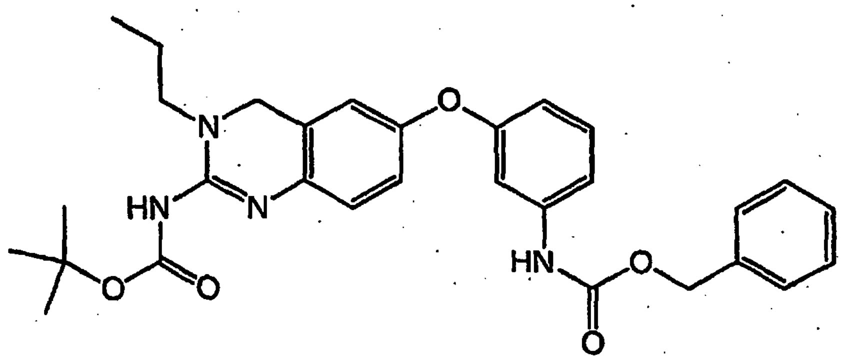

A mixture of [3-(2-amino-3-propyl-3,4-dihydro-quinazolin-6-yloxy)-phenyl]-carbamic acid benzyl ester (0.011 mol) in DCM (100 mL) was cooled to 5°C. Then, a solution of *t*-butoxycarbonyl anhydride (0.013 mol) in DCM (20 mL) was added dropwise, and the reaction mixture was stirred overnight at room temperature. Extra *t*-butoxycarbonyl anhydride (q.s.) was added and the resulting mixture was stirred for 3 hours at 30°C. The solvent was evaporated and the obtained residue was stirred in diisopropyl ether. Finally, the resulting precipitate was filtered off and dried under vacuum to yield the title compound as a solid.

**Example 28**

**[6-(3-Amino-phenoxy)-3-propyl-3,4-dihydro-quinazolin-2-yl]-carbamic acid *tert*-butyl ester**

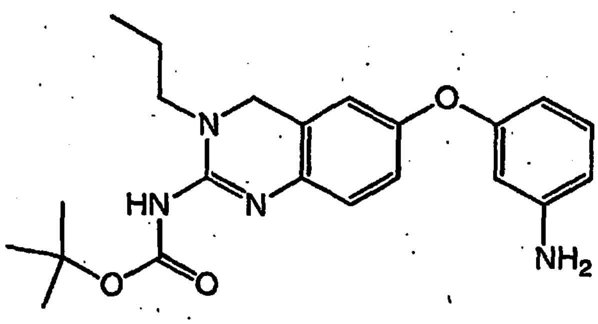

A mixture of [6-(3-benzyloxycarbonylamino-phenoxy)-3-propyl-3,4-dihydro-quinazolin-2-yl]-carbamic acid *tert*-butyl ester (0.008 mol) In methanol (150 mL) was hydrogenated with 10% Pd/C (1 g) as a catalyst. After uptake of $H_2$ gas (1 equiv.), the reaction mixture was filtered over Dicalite and the filtrate was evaporated. The residue was stirred in diisopropyl ether and the resulting precipitate was filtered off and dried to yield the title compound as a solid.

**Example 29**

**{3-Propyl-6-[3-(2,4,6-trimethyl-benzylamino)-phenoxy]-3,4-dihydroquinazolin-2-yl}-carbamic acid *tert*-butyl ester**

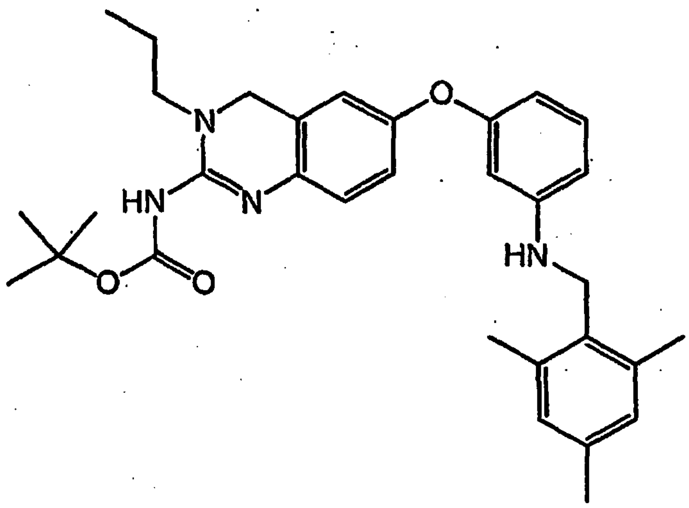

2,4,6-Trimethyl-benzaldehyde (0.0005 mol) was added to a mixture of [6-(3-amino-phenoxy)-3-propyl-3,4-dihydro-quinazolin-2-yl]-carbamic acid *tert*-butyl ester (0.0005 mol) in DCE (3 mL) and the mixture was stirred for 5 min. at room temperature, and then $NaBH(OAc)_3$ (0.0006 mol) was added and the reaction mixture was stirred overnight at room temperature. The excess $NaBH(OAc)_3$ was decomposed with $CH_3OH$, and the solvent was evaporated. The obtained residue was purified by Biotage column chromatography (eluent: 100% $CH_2Cl_2$). The purest product fractions Were collected and then the solvent was evaporated and co-evaporated with toluene to yield the title compound as a residue.

**Example 30**

**3-Propyl-6-[3-(2,4,6-trimethyl-benzylamino)-phenoxy]-3,4-dihydroquinazolin-2-ylamine**

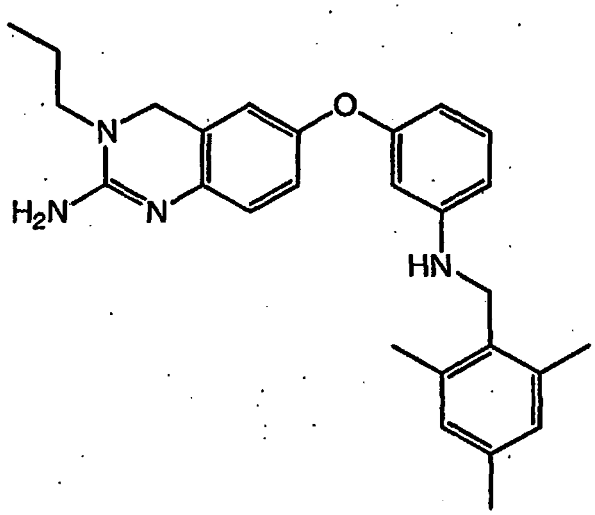

A mixture of {3-propyl-6-[3-(2,4,6-trimethyl-benzylamino)-phenoxy]-3,4-dihydro-quinazolin-2-yl}-carbamic acid *tert*-butyl ester (0.0001 mol) in 10% TFA In DCM (6 mL) was stirred in a reaction vial for 1 hour at room temperature and then the solvent was evaporated. The obtained residue was crystallized from $CH_3CN$, and then the desired product was filtered off, washed with diisopropyl ether and dried under vaccum to yield the title compound as a solid.
mp 238˚C

**Example 31**

**3-(3-Dimethoxymethyl-4-nitro-phenoxy)-phenylamine**

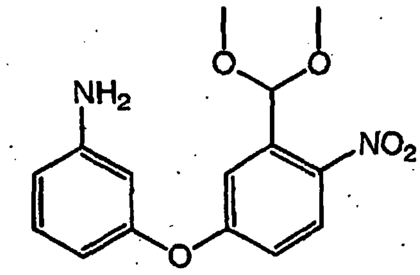

$K_2CO_3$ (0.17 mol) and then 4-chloro-2-dimethoxymethyl-1-nitro-benzene (0.1 mol) was added to a solution of 3-amino-phenol (0.15 mol) in DMA (200 mL) and the reaction mixture was stirred at 130°C for 24 hours. The mixture was poured out into water and extracted with diisopropyl ether. The extract was purified by column chromatography over silica gel (eluent: $CH_2Cl_2$:$CH_3OH$ 100:0 to 65:35). The product fractions were collected and the solvent was evaporated to yield the title compound as a residue.

**Example 32**

**3-(3-Dimethoxymethyl-4-nitro-phenoxy)-phenylamine**

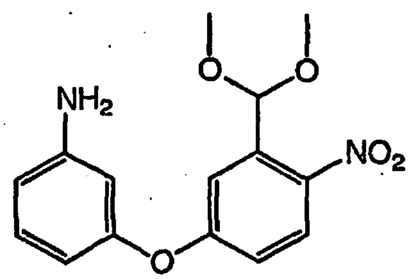

3-Amino-phenol (0.050 mol) was dissolved in DMA (100 ml). $K_2CO_3$ (0.050 mol) was added. 4-Chloro-2-dimethoxymethyl-1-nitro-benzene (0.050 mol) was added and the reaction mixture was stirred overnight at 100°C, then for 2 hours at 120°C. The reaction mixture was poured out into water and this mixture was extracted with diisopropyl ether. The separated organic layer was dried, filtered and the solvent evaporated. The residue was purified by column chromatography over silica gel (eluent: $CH_2Cl_2$:$CH_3OH$ 100:0 (to remove starting material) up to 70:30). The desired fractions were collected and the solvent was evaporated to yield the title compound as a residue.

**Example 33**

**Cyclohexyl-[3-(3-dimethoxymethyl-4-nitro-phenoxy)-phenyl]-amine**

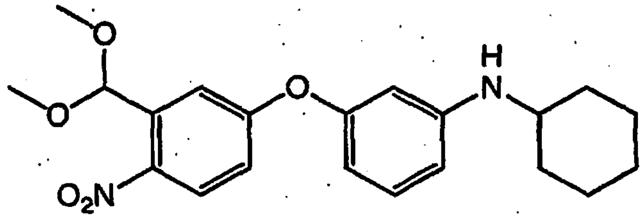

3-(3-Dimethoxymethyl-4-nitro-phenoxy)-phenylamine (0.0125 mol) was dissolved in DCE (150 mL), and cyclohexanone (0.0125 mol) was added, followed by $NaBH(OAc)_3$ (0.0187 mol). The reaction mixture was stirred overnight at room temperature. Some extra $NaBH(OAc)_3$ was added, and the reaction mixture was stirred overnight at room temperature. A 10% NaOH solution (150 mL) was added, and this mixture was extracted with diisopropyl ether. The separated organic layer was dried ($MgSO_4$) and filtered, and the solvent was evaporated. The residue was purified by column chromatography over silica gel (eluent: 20:80 up to 5:95 hexane:$CH_2Cl_2$). The product fractions were collected and the solvent was evaporated to yield the title compound as a residue.

**Example 34**

**Cyclohexyl-[3-(3-dimethoxymethyl-4-nitro-phenoxy)-phenyl]-carbamic acid benzyl ester**

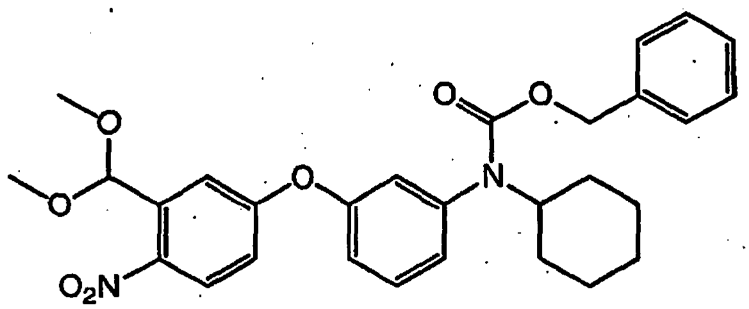

A mixture of cyclohexyl-[3-(3-dimethoxymethyl-4-nitro-phenoxy)-phenyl]-amine (0.00868 mol) and $Na_2CO_3$ (0.02083 mol) in $H_2O$ (15 mL) and THF (75 mL) was stirred at 5˚C. A solution of benzyl chloroformate (0.0104 mol) in THF (25 mL) was added dropwise over 15 min at 5˚C. The resultant reaction mixture was stirred for 21 hours at room temperature. This mixture was extracted with diisopropyl ether. The separated organic layer was dried ($MgSO_4$) and filtered, and the solvent was evaporated. The residue (0.00633 mol A) was reacted again with benzyl chloroformate (1.3 g, 0.0076 mol) and TEA (1.53 g, 0.0152 mol) In DCM (70 mL) under the same conditions as above. The residue was purified by column chromatography over silica gel (eluent: 95:5 $CH_2Cl_2$:hexane). The product fractions were collected and the solvent was evaporated to yield the title compound as a residue.

**Example 35**

**Cyclohexyl-[3-(3-formyl-4-nitro-phenoxy)-phenyl]-carbamic acid benzyl ester**

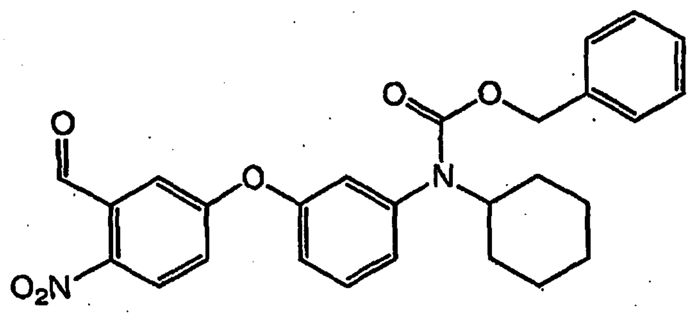

Cyclohexyl-[3-(3-dimethoxymethyl-4-nitro-phenoxy)-phenyl]-carbamic acid benzyl ester (0.0027 mol) was dissolved in THF (12 mL). Water (6 mL) and 12*N* HCl (3 mL) were added and the reaction mixture was stirred for 42 hours at room temperature. Water was added. This mixture was extracted with diisopropyl ether. The organic layer was separated, washed with a 10% aqueous $NaHCO_3$ solution and then with water, dried ($MgSO_4$) and filtered. The solvent was then evaporated to yield a residue that was used in the next reaction step without further purification.

**Example 36**

**Cyclohexyl-[3-(4-nitro-3-propylaminomethyl-phenoxy)-phenyl]-carbamic acid benzyl ester**

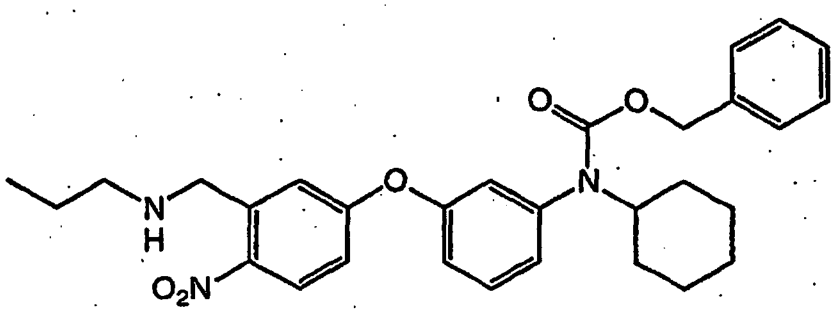

A mixture of propylamine (0.00356 mol) in DCE (70 mL) was stirred at room temperature. Cyclohexyl-[3-(3-formyl-4-nitro-phenoxy)-phenyl]-carbamic acid benzyl ester (0.00274 mol) was added. $NaBH(OAc)_3$ (0.00438 mol) was added. The reaction mixture was stirred overnight at room temperature. A 10% NaOH solution was added and this mixture was extracted with diisopropyl ether. The separated organic layer was dried ($MgSO_4$), filtered and the solvent evaporated. The residue was purified by column chromatography over silica gel (eluent: 100:0 to 96:4 $CH_2Cl_2$:$CH_3OH$). The product fractions were collected and the solvent was evaporated to yield the title compound as a residue.

**Example 37**

**[3-(4-Amino-3-propylaminomethyl-phenoxy)-phenyl]-cyclohexyl-carbamic acid benzyl ester**

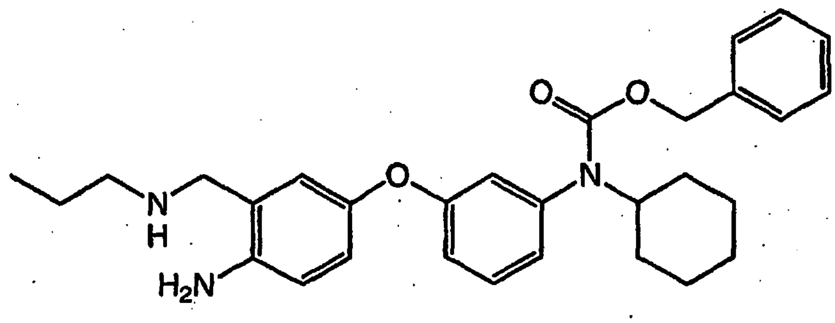

A mixture of cyclohexyl-[3-(4-nitro-3-propylaminomethyl-phenoxy)-phenyl]-carbamic acid benzyl ester (0.00181 mol) in methanol (50 mL) was hydrogenated with 5% Pt/C (0.5 g) as a catalyst in the presence of thiophene solution (1 mL). After uptake of $H_2$ (3 equiv), the catalyst was filtered off and the filtrate was evaporated to yield the title compound as a residue which was used in subsequent reactions without further purification.

**Example 38**

**[3-(2-Amino-3-propyl-3,4-dihydro-guinazolin-6-yloxy)-phenyl]-cyclohexylcarbamic acid benzyl ester**

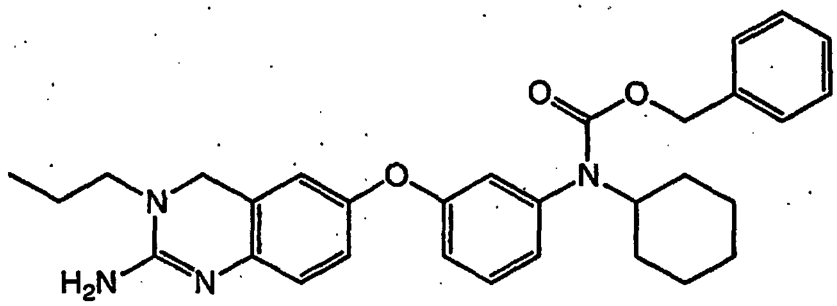

A mixture of [3-(4-amino-3-propylaminomethyl-phenoxy)-phenyl]-cyclohexyl-carbamic acid benzyl ester (0.0018 mol) and cyanogen bromide (0.00234 mol) in ethanol (60 mL) was stirred and refluxed for 3 hours and then was cooled. The ethanol solvent was evaporated. The residue was triturated under diisopropyl ether and ethanol, filtered off, and dried to yield the title compound as a solid.

**Example 39**

**Cyclohexylmethyl[3-(3-dimethoxymethyl-4-nitro-phenoxy)-phenyl]-amine**

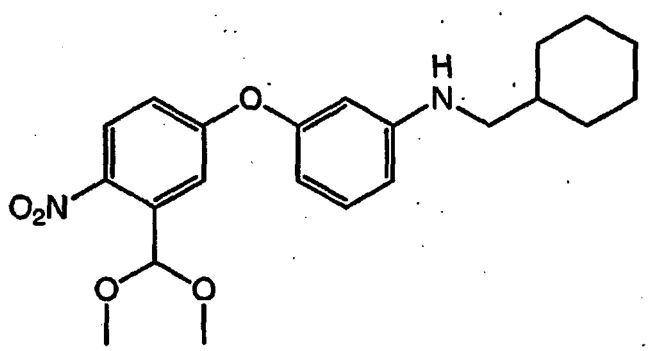

The reaction was completed under $N_2$. Cyclohexanecarboxaldehyde (0.01 mol) and then $NaBH(OAc)_3$ (0.015 mol) were added to a solution of 3-(3-dimethoxymethyl-4-nitro-phenoxy)-phenylamine (0.01 mol) in DCE (130 mL). The reaction mixture was stirred overnight at room temperature and washed with a saturated $NaHCO_3$ solution. The organic layer was separated, dried ($MgSO_4$) and filtered, and the solvent was evaporated to yield the title compound as a residue.

## Example 40

## 5-[3-(Cyclohexylmethyl-amino)-phenoxy]-2-nitro-benzaldehyde

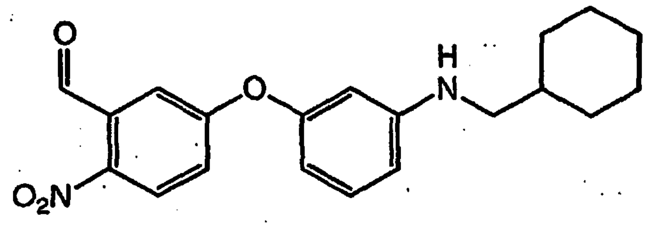

12*N* HCl (2 mL) and $H_2O$ (4 mL) were added to a mixture of cyclohexylmethyl-[3-(3-dimethoxymethyl-4-nitro-phenoxy)-phenyl]-amine (0.0055 mol) in THF (17 mL), and the reaction mixture was stirred overnight at room temperature. $H_2O$ was added, and the mixture was extracted with diisopropyl ether. The organic layer was washed with a 10% $NaHCO_3$ solution, dried ($MgSO_4$), filtered off and the solvent was evaporated. The residue was purified by short column chromatography (eluent: 100% $CH_2Cl_2$). The purest product fractions were collected and the solvent was evaporated to yield the title compound as a residue.

## Example 41

## Cyclohexylmethyl-[3-(4-nitro-3-propylaminomethyl-phenoxy)-phenyl]-amine

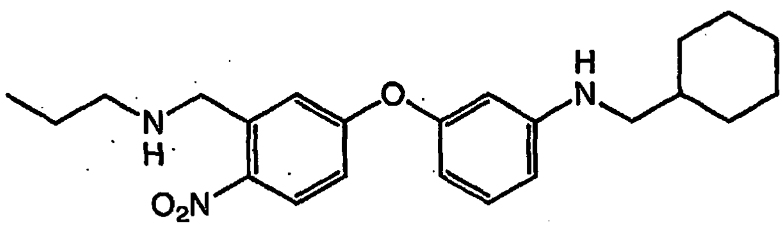

$NaBH(OAc)_3$ (0.0080 mol) was added to a mixture of propylamine (0.0054 mol) and 5-[3-(cyclohexylmethyl-amino)-phenoxy]-2-nitrobenzaldehyde (0.00054 mol) in DCE (100 mL). Then the reaction mixture was stirred overnight at room temperature and washed with a saturated $NaHCO_3$ solution. The organic layer was separated, dried ($MgSO_4$), and filtered, and the solvent was evaporated. The residue was purified by column chromatography (eluent: 100% $CH_2Cl_2$). The purest product fractions were collected and the solvent was evaporated to yield the title compound as a residue.

## Example 42

## 2-Amino-5-[3-[(cyclohexylmethyl)amino]phenoxy]-*N*-propyl-benzenemethamine

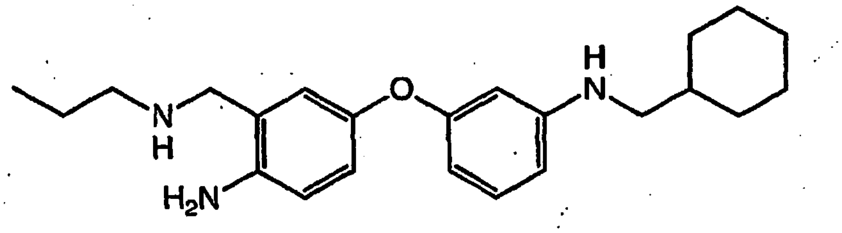

A mixture of cyclohexylmethyl-[3-(4-nitro-3-propylaminomethylphenoxy)-phenyl]-amine (0.0037 mol) in methanol (50 mL) was hydrogenated with 10% Pd/C (0.5 g) as a catalyst in the presence of thiophene solution (0.5 mL). After uptake of $H_2$ (3 equiv.), the reaction mixture was filtered over Dicalite and the filtrate was evaporated. The residue was purified by column chromatography (eluent: 98:2 $CH_2Cl_2$:$CH_3OH$). The purest product fractions were collected and the solvent was evaporated to yield the title compound as a residue.

**Example 43**

**6-[3-(Cyclohexylmethyl-amino)-phenoxy]-3-propyl-3,4-dihvdro-quinazolin-2-ylamine and [3-(2-Amino-3-propyl-3,4-dihydro-quinazolin-6-yloxy)-phenyl]-cyclohexylmethyl-cyanamide**

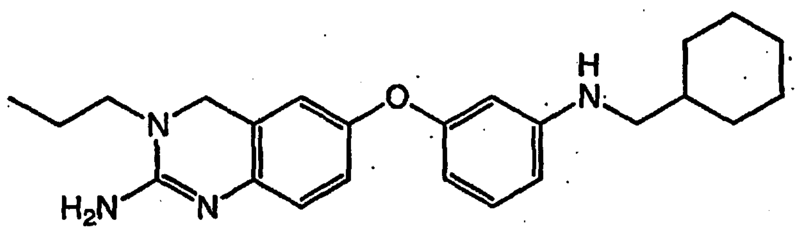

and

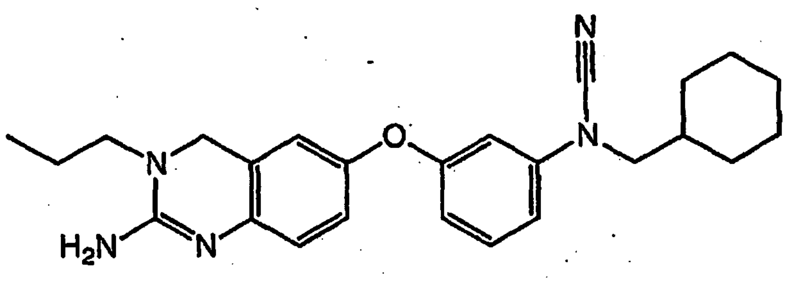

**Example 44**

**3-(3-Dimethoxymethyl-4-nitro-phenoxy)-benzonitrile**

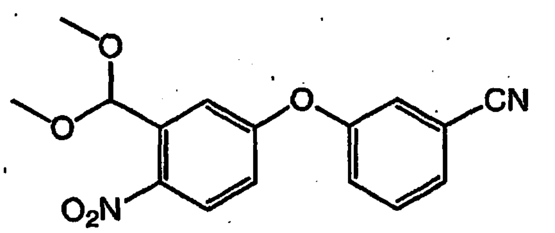

A mixture of 3-cyanophenol (0.38 mol) in ethanol (600 mL) was stirred at room temperature and $K_2CO_3$ (0.29 mol) was added. 5-fluoro-2-nitrobenzaldehyde (0.18 mol) was added, and the reaction mixture was stirred and refluxed for 4 hours. The solvent was evaporated, and the residue was stirred in water and extracted with $CH_2Cl_2$. The organic layer was separated, dried, filtered off and the solvent was evaporated. The residue was triturated under diisopropyl ether, and then the formed solids were filtered off and dried to yield the title compound.

**Example 45**

**3-(3-Formyl-4-nitro-phenoxy)-benzonitrile**

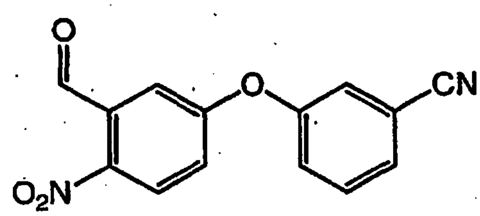

A mixture of 3-(3-dimethoxymethyl-4-nitro-phenoxy)-benzonitrile (0.068 mol) in THF (140 ml) was stirred at room temperature, then 12N HCl (55 ml) and water (55 ml) were added and the reaction mixture was stirred overnight at room temperature. The mixture was stirred in $H_2O$ and extracted with DIPE. The organic layer was separated, washed with a 10% $NaHCO_3$ solution, separated again, dried, filtered off and the solvent was evaporated. The obtained residue was stirred in DIPE, then the desired product was filtered off and dried to yield the title compound as a residue.

**Example 46**

**3-(4-Nitro-3-propylaminomethyl-phenoxy)-benzonitrile**

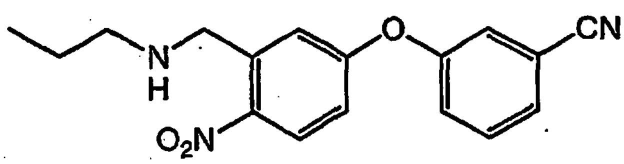

The following reaction was completed under $N_2$. A mixture of 3-(3-formyl-4-nitro-phenoxy)-benzonitrile (0.056 mol) and propylamine (0.059 mol) in DCE (450 ml) was stirred at room temperature, then $NaBH(OAc)_3$ (0.063 mol) was added. The reaction mixture was stirred overnight at room temperature. A saturated $NaHCO_3$ solution was added and the layers were separated. The organic layer was dried, filtered off and the solvent was evaporated. The residue was purified over silica gel filter (eluent: $CH_2Cl_2(CH_3OH$ 99/1). The purest product fractions were collected and the solvent was evaporated to yield the title compound as a residue.

**Example 47**

**3-(4-Amino-3-propylaminomethyl-phenoxy)-benzonitrile**

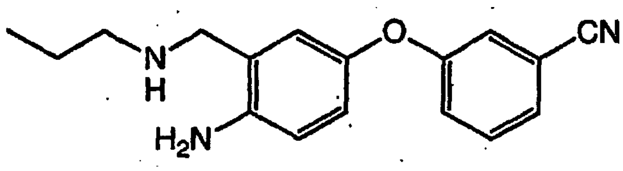

A mixture of 3-(4-nitro-3-propylaminomethyl-phenoxy)-benzonitrile (0.045 mol) in methanol (250 ml) was hydrogenated with 10% Pd/C (2 g) as a catalyst in the presence of thiophene solution (2 ml). After uptake of $H_2$ (3 equiv.), the reaction mixture was filtered over dicalite and the filtrate was evaporated. The residue was purified by column chromatography over silica gel (eluent: $CH_2Cl_2/CH_3OH$ 98/2). The purest product fractions were collected and the solvent was evaporated to yield the title compound as a residue.

## Example 48

## 3-(2-Amino-3-propyl-3,4-dihydro-quinazolin-6-yloxy)-benzonitrile

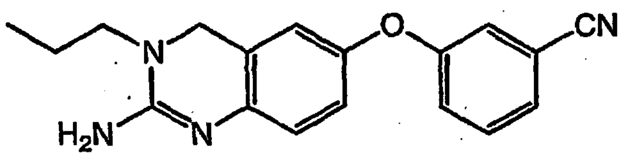

A mixture of 3-(4-Amino-3-propylaminomethyl-phenoxy)-benzonitrile (0.032 mol) in ethanol (400 ml) was stirred at room temperature and cyanogens bromide (0.038 mol) was added. The reaction mixture was stirred and refluxed for 2 hours. After cooling, the precipitate was filtered off, washed with DIPE and dried to yield the title compound.

The filtrate was evaporated and the obtained residue was crystallised from $CH_3CN$/DIPE, then the precipitate was filtered off, washed with DIPE and dried to yield residue.

## Example 49

## 3-(2-Amino-3-propyl-3,4-dihydro-quinazolin-6-yloxy)-benzoic acid

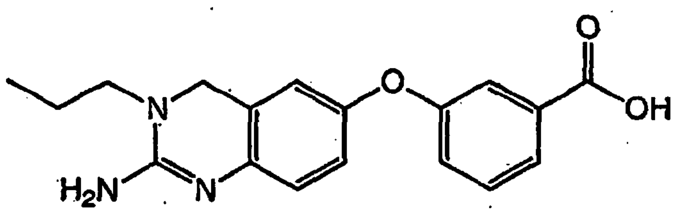

A mixture of 3-(2-amino-3-propyl-3,4-dihydro-quinazolin-6-yloxy)-benzonitrile (0.0013 mol), prepared as described in Example 75, in concentrated $H_2SO_4$ (5 mL) and water (5 mL) was stirred and refluxed for 2 hours. The reaction mixture was then cooled, and the resulting solids were filtered off. The obtained filter residue was washed with ice-water and dried to yield the title compound as a solid.

## Example 50

## 3-(2-Amino-3-propyl-3,4-dihydro-quinazolin-6-yloxy)-benzoyl chloride

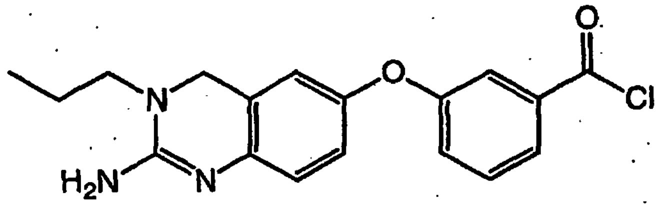

A mixture of 3-(2-amino-3-propyl-3,4-dihydro-quinazolin-6-yloxy)-benzoic acid (0.0009 mol) in $SOCl_2$ (2 mL) and DCM (10 mL) was stirred for 2 hours at room temperature and then the solvent was evaporated. The obtained residue was stirred in toluene and the solvent was evaporated again to yield the title compound as a residue.

**Example 51**

**3-(2-Amino-3-propyl-3,4-dihydro-qulnazolin-6-yloxy)-N-(2,4,6-trimethylbenzyl)-benzamide**

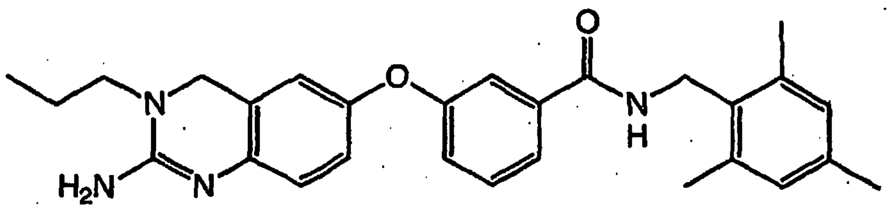

A mixture of 3-(2-amino-3-propyl-3,4-dihydro-quinazolin-6-yloxy)-benzoyl chloride (0.0008 mol) in DCM (20 mL) was stirred at 0˚C and 2,4,6-trimethylbenzylamine (0.0013 mol) was added.Then TEA (q.s.) was added, and the reaction mixture was stirred at room temperature. The mixture was stirred in $H_2O$ and the organic layer was separated. The remaining layer was washed again with $H_2O$, then the organic layer was separated, dried, and filtered, and the solvent was evaporated. The residue was purified by column chromatography over silica gel (gradient eluent: $CH_2Cl_2$:($CH_3OH$:$NH_3$)). The purest product fractions were collected, and the solvent was evaporated. The residue was triturated under diisopropyl ether:$CH_3CN$, and then the resulting solids were filtered off and dried to yield the title compound as a solid.

**Example 52**

**4-Chloro-2,dimethoxymethyl-1-nitro-benzene**

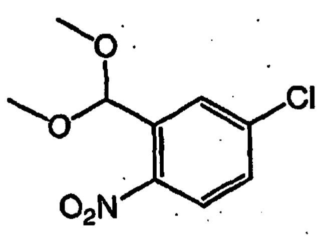

A mixture of 5-chloro-2-nitro-benzaldehyde (0.0792 mol), trimethoxymethane (0.126 mol) and PTSA (0.00079 mol) in methanol (80m) was refluxed until the 5-chloro-2-nitro-benzaldehyde had completely reacted. The mixture was cooled, $Na_2CO_3$ was added and stirred for 5min. The mixture was filtered off and the filtrate was evaporated under reduced pressure to yield the title compound as a residue.

**Example 53**

**3-(3-Dimthoxymethyl-4-nitro-phenylsulfanyl)-phenylamine**

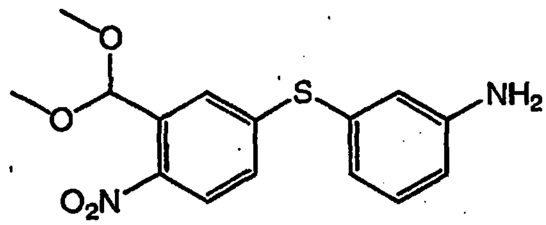

$K_2CO_3$ (0.085 mol) was added to a mixture of 3-aminobenzenethiol (0.075 mol) in DMA (150 mL). Then 4-chloro-2-dimethoxymethyl-1-nitrobenzene (0.050 mol) was added and the reaction mixture was stirred for 2 hours at 80˚C. $H_2O$ was added and the mixture was extracted with diisopropyl ether. The crude product was purified by column chromatography (eluent:' 70:30 $CH_2Cl_2$/hexane up to 99:1 $CH_2Cl_2$:$CH_3OH$). The product fractions were collected, and the solvent was evaporated to yield the title compound as a residue.

**Example 54**

**N-[3-(3-Dimethoxymethyl-4-nitro-phenylsulfanyl)-phenyl]-benzenesulfonamide**

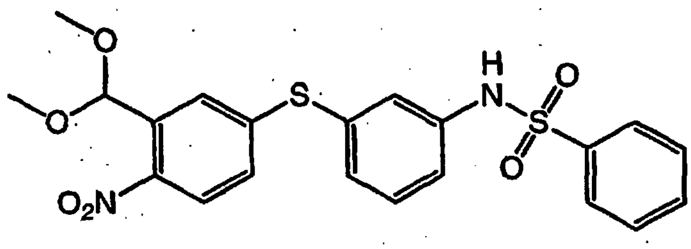

A mixture of 3-(3-dimethoxymethyl-4-nitro-phenylsulfanyl)-phenylamine (0.0384 mol) and TEA (0.0578 mol) in THF (q.s.) was cooled to 5˚C, and phenylsulfonyl chloride (0.0384 mol) was added dropwise at 5˚C. Then, the reaction mixture was reacted at room temperature, and purified by column chromatography over silica gel (eluent: $CH_2Cl_2$). The product fractions were collected and the solvent was evaporated to yield the title compound as a residue.

**Example 55**

**N-[3-(3-Formyl-4-nitro-phenylsulfanyl)-phenyl]-benzenesulfonamide**

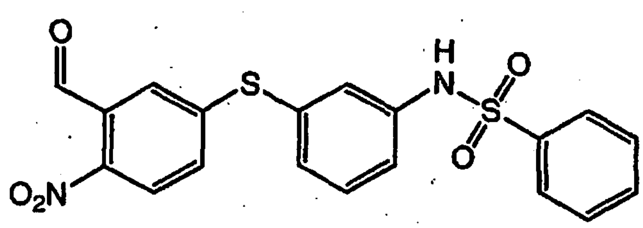

A mixture of N-[3-(3-dimethoxymethyl-4-nitro-phenylsulfanyl)-phenyl]-benzenesulfonamide (0.027 mol) in 12*N*

HCl (25 mL), THF (75 mL), and water (50 mL) was stirred overnight at room temperature, and then the mixture was extracted with EtOAc. The extract was washed with $H_2O$ and then with a saturated $Na_2CO_3$ solution. The solvent was evaporated, and the residue was purified by column chromatography over silica gel (eluent: $CH_2Cl_2$). The product fractions were collected and the solvent was evaporated to yield the title compound as a residue.

**Example 56**

**N-[3-(3-Formyl-4-nitro-benzenesulfinyl)-phenyl]-benzenesulfonamide**

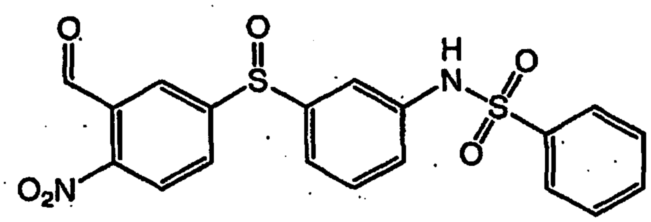

A mixture of N-[3-(3-formyl-4-nitro-phenylsulfanyl)-phenyl]-benzenesulfonamide (0.0034 mol) and 3-Chlorobenzenecarboperoxoic acid (also known as MCPBA or meta-chloroperbenzoic acid) (0.7 g; 75%) in $CHCl_3$ (q.s.) was stirred for 4 hours at room temperature. Then a $NaHCO_3$ solution was added, and the reaction mixture was stirred. The organic layer was separated, dried, and filtered, and the solvent was evaporated. The residue was purified by column chromatography over Biotage (gradient eluent: $CH_2Cl_2$:$CH_3OH$). The purest product fractions were collected, and the solvent was evaporated to yield the title compound as a residue.

**Example 57**

**N-[3-(4-Nitro-3-propylaminomethyl-benzenesulfinyl)-phenyl]-benzenesulfonamide**

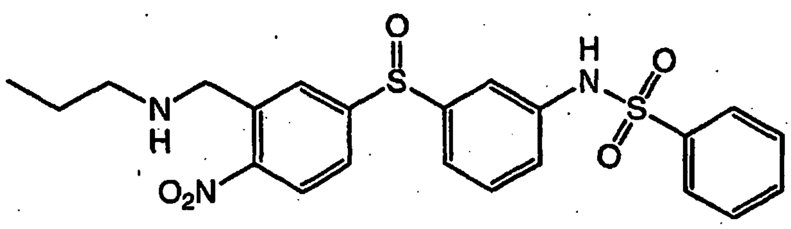

The following reaction was run under $N_2$. A mixture of N-[3-(3-formyl-4-nitro-benzenesulfinyl)-phenyl]-benzenesulfonamide (0.0023 mol) and propylamine (0.0025 mol) in DCE (50 mL) was stirred at room temperature and NaBH$(OAc)_3$ (0.8 g) was added. The reaction mixture was stirred overnight at room temperature, and then a $NaHCO_3$ solution was added. The organic layer was separated, dried, and filtered, and the solvent was evaporated to yield the title compound as a residue.

**Example 58**

**N-[3-(4-Amino-3-propylaminomethyl-benzenesulfinyl)-phenyl]-benzenesulfonamide**

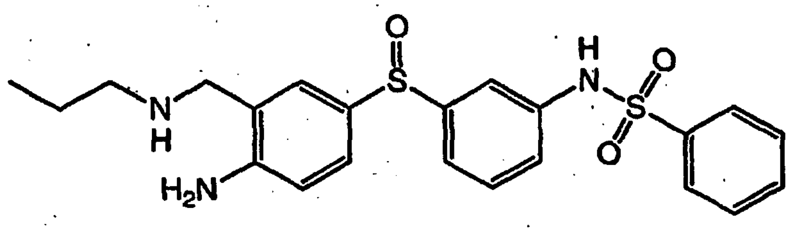

A mixture of N-[3-(4-nitro-3-propylaminomethyl-benzenesulfinyl)-phenyl]-benzenesulfonamide (0.0019 mol) in methanol (100 mL) was hydrogenated with 10%Pd/C (0.5 g) as a catalyst. After uptake of $H_2$ (3 equiv.), the reaction mixture was filtered over Dicalite, and the filtrate was evaporated. The residue was filtered over silica gel (eluent: 95:5 $CH_2Cl_2$:$CH_3OH$),and then the purest product fractions were collected, and the solvent was evaporated to yield the title compound as a residue.

**Example 59**

**N-[3-(2-Amino-3-prooyl-3,4-dihydro-quinazoline-6-sulfinyl)-phenyl]-benzenesulfonamide**

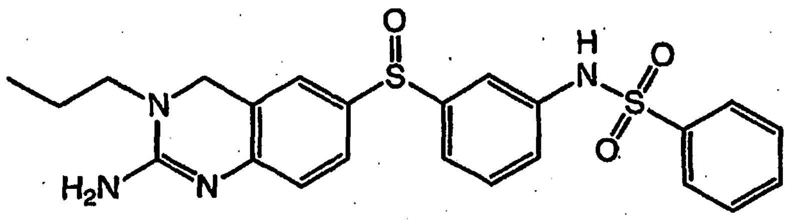

A mixture of N-[3-(4-amino-3-propylaminomethyl-benzenesulfinyl)-phenyl]-benzenesulfonamide (0.00090 mol) and cyanogen bromide (0.00094 mol) In ethanol (30 mL) was stirred overnight at room temperature, and then the solvent was evaporated. The residue was triturated under diisopropyl ether, and the solids were filtered off. The residue was converted into the free base with a NaOH solution. The mixture was extracted with $CH_2Cl_2$, but the product stayed in the aqueous layer so the mixture was desalted with NaCl to yield an oil. The solvent was decanted, and the remaining oil was triturated under $CH_3CN$. The resulting solids were filtered off, washed with diisopropyl ether, and dried to yield the title compound as a solid.

**Example 60**

**BACE ASSAY-1**

The following standards are reagents were used in this assay: Sodium Citrate trisodium salt dihydrate (Sigma), Citric Acid monohydrate (Merck), PEG8000 (Sigma), MCA-substrate (Bachem), β-secretase (BACE) (Panvera), 96-well plate zwart (Costar (Elscolab)), StatVal (Bachem).

The following standard assay buffer solution was prepared and used in this assay: 0.05 M, pH 5.0 mixture of sodium citrate trisodium salt dihydrate (9.56g), citric acid monohydrate (3.68g) and PEG8000 (0.50g).

The MCA-substrate stock solution was prepared by mixing 10 mg MCA-substrate with 5 mL DMSO for a final solution concentration of 2.0 mg/mL. The substrate work solution was prepared by mixing 0.1 mL substrate in 1.90 mL assay buffer for a final concentration of 0.05 mM. The β-secretase (BACE) work solution was prepared by mixing 8μL BACE in 2 mL assay buffer for a final concentration of 10μg/mL.

Test compounds were dissolved in DMSO at various concentrations in the range of $3.3x10^{-3}$ M to $1.5x10^{-6}$ M.

The screen procedure for this assay was as follows. 60 μL of assay buffer was pipetted into each well of a 96-well plate. To each well was then pipetted 1μL of test compound at the selected concentration. To each well was then added 20μL of the β-secretase work solution and 20μL of the MCA-substrate stock solution. Each well was mixed for a few second and the $T_0$ measured with fluoroscan Ex320/Em405. The plates were then incubated for 1 hour at room temperature and the T60 measured with fluoroscan Ex320/Em405.

The procedure for the blank was as follows. 80 μL of assay buffer was pipetted into each well to be used as

a blank control. To each well was then added 1 μL of DMSO and 20 μL of MCA-substrate solution in each well. The To was measured with fluoroscan Ex320/Em405, the plate was incubated for 1 hour at room temperature and the T60 was then measured with fluoroscan Ex320/Em405.

The procedure for the positive control was as follows. 60 μL of assay buffer was pipetted into each well to be used as a positive control. To each well was then added 1 μL of DMSO, 20 μL of BACE work solution and 20 μL of MCA-substrate stock solution. The To was measured with fluoroscan Ex320/Em405, the plate was incubated for 1 hour at room temperature and the T60 was then measured with fluoroscan Ex320/Em405.

For test compounds, measured at multiple concentrations, the measured $T_0$ and $T_{60}$ values were used to calculate an $IC_{50}$ value using Graphpad Software (or PIR).

## Example 61

## BACE ASSAY-2

The following reagents were used in this assay: sodium acetate, PEG8000 (Sigma), DMSO, HEPES, FS1 substrate [R(AedensE)EEVNLDAEFK-(DabcyiK)R], β-secretase (BACE) (Panvera), and 96-well plate (HE microplate, Molecular Devices).

The following assay buffers were prepared and used in this assay: (1) enzyme assay buffer (0.05 M sodium acetate, pH5, 0.1 % PEG8000 (w/v)), (2) substrate assay buffer (0.05 M sodium acetate, pH5), and (3) compound vehicle (30% DMSO in 50 mM HEPES, pH 7.4).

The FS1-substrate stock solution was prepared in DMSO as a 10 mg/mL solution. The FS1-substrate working solution was prepared by diluting the 10 mg/mL stock solution with substrate assay buffer to a final concentration of 300 μg/mL. The β-secretase (BACE) working solution was prepared by diluting a 0.83 mg/mL BACE stock solution with enzyme assay buffer to a final concentration of 4μg/mL.

Test compounds were dissolved in DMSO at 10mM. Compounds were further diluted in compound vehicle to various concentrations in the range of 675 μM to 13.5nM (13.5 x final compound concentration in Ki plate).

The procedure for this assay was as follows: 15 μL of BACE working solution was pipetted into each well of a 96-well plate. To each well was then pipetted 2 μL of test compound at the selected concentration. Test compound and BACE were then mixed with a pipettor and incubated for 20 min at room temperature. To each well was then added 10μL of the FS1 substrate working solution. The fluorescence for each well was then measured on a Polarstar fluorometer (Ex 390nm/Em 520 nm) for 20 min at room temperature, reading fluorescence at 1 min intervals.

The procedure for the positive control was as follows: 15 μL of BACE working solution was pipetted into each well to be used as a positive control. To each well was then pipetted 2 μL of vehicle. Vehicle and BACE were then mixed with a pipettor and incubated for 20 min at room temperature. To each well was then added 10μL of the FS1 substrate working solution. The fluorescence was then measured on a Polarstar fluorometer (Ex 390nm/Em 520 nm) for 20 min at room temperature, reading fluorescence at 1 min intervals.

For test compounds, measured at multiple concentrations, the measured To and $T_{60}$ values were used to calculate an $IC_{50}$ value using Graphpad Software (or PIR). For test compounds, $K_i$ inhibition was determined as follows: For each compound concentration and positive control, rate of cleavage of substrate ($V_i$, where i = compound concentration in μM) was determined as Δ Fluorescence/A time (min). Cleavage rates ($V_i$) were plotted as a function of inhibitor concentration in μM [I]. The $K_i$ was then determined by fitting the following equation to the graph of [I] vs $V_i$

$$Y=aV_{max}/(50+24*(1+X/K_i)),$$

where 50 = substrate concentration (μM) and 24 = $K_m$ of substrate (μM).

Representative compounds of the present invention were tested according to procedures described in Example 158 and 159 above with measured $IC_{50}$ and $K_i$ values as listed in Table 15 below. The procedure used to determine a particular value is defined within the header row in parentheses (for example (158) indicates that the $IC_{50}$ values in that column were determined using the procedure described in Example 60).

**Table 15: BACE *In vitro* Assay Results**

| ID No. | $IC_{50}$ μM (158) | $IC_{50}$ μM (159) | KI μM (159) |
|---|---|---|---|
| 44 | 6.1 | | 3.3 |
| 49 | 0.80 | 2.8 | 0.84 |

(continued)

| ID No. | $IC_{50}$ μM (158) | $IC_{50}$ μM (159) | KI μM (159) |
|---|---|---|---|
| 53 | 1.0 | 6.8 | 0.82 |
| 55 | 0.28 | 2.4 | 0.79 |
| 56 | 0.97 | 5.4 | 0.77 |
| 60 | 5.6 | | |
| 61 | 0.20 | 0.44 | 0.23 |
| 67 | 0.68 | 3.9 | 0.81 |
| 95 | 0.31 | | |
| 126 | 0.24 | | |
| 203 | 4.7 | | |
| 225 | 2,9 | | |
| 237 | >10 | | |
| 238 | 8.9 | | |
| 239 | 0.35 | | |
| 240 | 0.89 | | |
| 241 | 0.49 | | |
| 242 | >10 | | |
| 243 | 0.033 | | |
| 244 | 2.1 | | |
| 245 | 0.17 | | |
| 246 | >10 | | |
| 247 | 1.7 | | |
| 248 | 6.9 | | |
| 249 | 5.2 | | |
| 250 | >10 | | |
| 251 | >10 | | |
| 252 | 1.0 | | |
| 253 | >10 | | |
| 254 | >10 | | |
| 255 | >10 | | |
| 269 | 0.35 | | |
| 270 | 2.37 | | |
| 271 | 0.39 | | |
| 272 | 0.52 | | |
| 273 | 0.17 | | |
| 274 | >10 | | |
| 275 | >10 | | |
| 276 | >1.0 | | |
| 277 | 0.70 | | |

(continued)

| ID No. | $IC_{50}$ μM (158) | $IC_{50}$ μM (159) | KI μM (159) |
|---|---|---|---|
| 278 | >10 | | |
| 279 | >10 | | |
| 280 | 11 | | |
| 281 | 1.6 | | |
| 282 | >10 | | |
| 283 | >10 | | |
| 284 | 0.87 | | |
| 285 | >10 | | |
| 296 | 0.90 | | |
| 305 | 1.4 | | |
| 335 | 0.24 | | |
| 340 | 2.0 | | |
| 344 | 0.058 | | |
| 345 | 0.028 | | |
| 355 | 2.6 | | |
| 380 | 0.71 | | |
| 383 | 0.65 | | |
| 385 | 0.28 | | |
| 387 | 3.3 | | |
| 397 | 3.98 | | |
| 423 | 5.8 | | |
| 430 | 2.8 | | |
| 431 | 0.80 | | |
| 464 | 1.3 | | |
| 497 | 0.011 | | |
| 500 | 0.16 | | |
| 501 | 3.4 | | |
| 502 | 0.18 | | |
| 507 | 0.081 | | |
| 533 | 0.17 | | |
| 729 | | | 0.33 |
| 755 | 0.21 | 1.9 | 0.270 |

**Example 62**

**BACE FS1 % Inhibition Assay**

The following reagents were used in this assay: sodium acetate, PEG8000 (Sigma), DMSO, HEPES, FS1 substrate [R(AedensE)EEVNLDAEFK-(DabcylK)R], β-secretase (BACE) (Panvera), and 96-well plate (HE microplate, Molecular Devices).

The following assay buffers were prepared and used in this assay: (1) enzyme assay buffer (0.05 M sodium

acetate, pH5, 0.1% PEG8000 (w/v)), (2) substrate assay buffer (0.05 M sodium acetate, pH5), and (3) compound vehicle (30% DMSO In 50mM HEPES, pH 7.4).

The FS1-substrate stock solution was prepared in DMSO as a 10 mg/mL solution. The FS1-substrate working solution was prepared by diluting the 10 mg/mL stock solution with substrate assay buffer to a final concentration of 300 μg/mL The β-secretase (BACE) working solution was prepared by diluting a 0.83 mg/mL BACE stock solution with enzyme assay buffer to a final concentration of 4μg/mL.

Test compounds were dissolved in DMSO to 10mM. Compounds were further diluted in vehicle to various concentrations in the range of 405 μM to 4.05 μM (13.5 x final compound concentration in screening plate).

The screening procedure for this assay was as follows: 15 μL of BACE working solution was pipetted into each well of a 96-well plate. To each well was then pipetted 2 μL of test compound at the selected concentration. Test compound and BACE were then mixed with a pipettor and incubated for 20 min at room temperature. To each well was then added 10μL of the FS1 substrate working solution. The plates were then incubated for 1 hour at room temperature. The fluorescence for each well was then measured on an LJL analyst (Ex 360nm /Em 530 nm).

The procedure for the blank was as follows. 15 μL of assay buffer was pipetted Into each well to be used as a blank control. To each well was then added 2 μL of vehicle and 10 μL of FS1-substrate working solution. The plates were then incubated for 1 hour at room temperature. The fluorescence was measured on an LJL analyst (Ex 360nm /Em 530 nm).

The procedure for the positive control was as follows: 15 μL of BACE working solution was pipetted into each well to be used as a positive control. To each well was then pipetted 2 μL of vehicle. Vehicle and BACE were then mixed with a pipettor and incubated for 20 min at room temperature. To each well was then added 10μL of the FS1 substrate working solution. The plates were then incubated for 1 hour at room temperature. The fluorescence (FI) was then measured on an LJL analyst (Ex 360nm /Em 530 nm).

For test compounds, % inhibition was determined at each concentration as follows:

$$\% \text{ Inhibition} = \frac{[\text{FI(compound)} - \text{FI(negative control)}]}{[\text{FI(positive control)} - \text{FI(negative control)}]}$$

% Inhibition values of less than 30% were indistinguishable from control are listed as ≤30% in the Table below. % Inhibition values greater than 100% were indistinguishable from 100% within the error of the measurement.

Representative compounds of the present invention were tested according to procedure described in Example 62 above with results as listed In Table 16 below.

**Table 16: % Inhibition (FS)**

| ID No. | 30 μM | 10μM | 3 μM | 1 μM | 0.3 μM |
|---|---|---|---|---|---|
| 729 | | | 100 | 61 | 33 |
| 755 | | 79 | 59 | | |

Example 63

BACE % Inhibition Assay

The following reagents were used In this assay: sodium acetate, PEG8000 (Sigma), DMSO, HEPES, (Aedens)-EVNLDAEF-(Dabcyl K-amide) substrate, β-secretase (BACE) (Panvera), and 96-well plate (HE microplate, Molecular Devices).

The following assay buffers were prepared and used in this assay: (1) enzyme assay buffer (0.05 M sodium acetate, pH5, 0.1 % PEG8000 (w/v)), (2) substrate assay buffer (0.05 M sodium acetate, pH5), and (3) compound vehicle (30% DMSO in 50mM HEPES, pH 7.4).

The substrate stock solution was prepared in DMSO as a 10 mg/mL solution. The substrate working solution was prepared by diluting the 10 mg/mL stock solution with substrate assay buffer to a final concentration of 300 μg/mL. The β-secretase (BACE) working solution was prepared by diluting a 0.83 mg/mL BACE stock solution with enzyme assay buffer to a final concentration of 4μg/mL.

Test compounds were dissolved in DMSO to 10mum. Compounds where further diluted in vehicle to various concentrations in the range of 405 μM to 4.05 μM (13.5 x final compound concentration in screening plate).

The screening procedure for this assay was as follows: 15 μL of BACE working solution was pipetted into each

well of a 96-well plate. To each well was then pipetted 2 μL of test compound at the selected concentration. Test compound and BACE were then mixed with a pipettor and incubated for 20 min at room temperature. To each well was then added 10μL of the substrate working solution. The plates were then incubated for 1 hour at room temperature: The fluorescence for each well was then measured on an LJL analyst (Ex 360nm /Em 530 nm).

The procedure for the blank was as follows. 15 μL of assay buffer was pipetted into each well to be used as a blank control. To each well was then added 2 μL of vehicle and 10 μL of substrate working solution. The plates were then incubated for 1 hour at room temperature. The fluorescence was measured on an LJL analyst (Ex 360nm /Em 530 nm).

The procedure for the positive control was as follows: 15 μl of BACE working solution was pipetted into each well to be used as a positive control. To each well was then pipetted 2 μL of vehicle. Vehicle and BACE were then mixed with a pipettor and incubated for 20 min at room temperature. To each well was then added 10μL of the substrate working solution. The plates were then incubated for 1 hour at room temperature. The fluorescence (FI) was then measured on an LJL analyst (Ex 360nm/Em 530 nm).

For test compounds, % inhibition was determined at each concentration as follows:

$$\%\ \text{Inhibition} = \frac{[\text{FI(compound)} - \text{FI(negative control)}]}{[\text{FI(positive control)} - \text{FI(negative control)}]}$$

% Inhibition values of less than 30% were indistiriguishable from control are listed as ≤30% in the Table below.

Representative compounds of the present invention were tested according to procedure described in Example 63 above with results as listed in Table 17 below. The % error In the measurements was ±10%.

**TABLE 17: % Inhibition**

| ID No. | 30 μM | 10 μM | 3 μM | 1 μM | 0.3 μM |
|---|---|---|---|---|---|
| 44 | | 31 | <30 | | |
| 49 | | 90 | 56 | | |
| 53 | | 82 | 34 | | |
| 55 | | 95 | 62 | | |
| 56 | | 79 | 35 | | |
| 61 | | 87 | 75 | | |
| 67 | | 71 | 44 | | |

While the foregoing specification teaches the principles of the present Invention, with examples provided for the purpose of illustration, it will be understood that the practice of the invention encompasses all of the usual variations, adaptations and/or modifications as come within the scope of the following claims and their equivalents.

## Claims

**1.** A compound of formula (III)

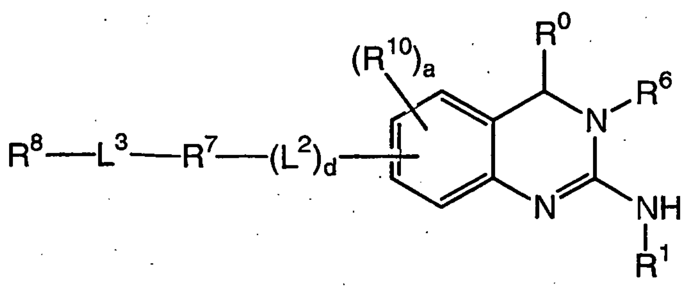

(III).

wherein

$R^0$ is selected from the group consisting of hydrogen, methyl and $CF_3$;
$R^1$ is selected from the group consisting of hydrogen, hydroxy, methyl, ethyl, trifluoromethyl, 2,2,2-trifluoroethyl, methoxy, methoxy and methyl-carbonyl;
$R^6$ is selected from the group consisting of $C_{1-6}$alkyl and hydroxy substituted $C_{1-8}$alkyl;
d is an integer from 0 to 1;
$L^2$ is selected from the group consisting of -O-, -S(O)$_{0-2}$- and -NR$^Q$-; wherein $R^Q$ is selected from the group consisting of hydrogen and $C_{1-4}$alkyl;
$R^7$ is selected from the group consisting of cycloalkyl, cycloalkyl-$C_{1-4}$alkyl, aryl, $C_{1-4}$alkyl-aryl, aryl-$C_{1-4}$alkyl, partially unsaturated carbocyclyl, partially unsaturated carbocyclyl-$C_{1-4}$alkyl, heteroaryl, heteroaryl-$C_{1-4}$alkyl, heterocycloalkyl and heterocycloalkyl-$C_{1-4}$alkyl;
wherein the aryl, cycloalkyl, partially unsaturated carbocyclyl, heteroaryl or heterocycloalkyl group, whether alone or as part of a substituent group, is optionally substituted with one or more substituents independently selected from the group consisting of $C_{1-4}$alkyl, $C_{1-4}$alkoxy, halogen substituted $C_{1-4}$alkyl, halogen substituted $C_{1-4}$alkoxy, hydroxy substituted $C_{1-4}$alkyl, hydroxy, carboxy, cyano, nitro, amino, $C_{1-4}$alkylamino, di($C_{1-4}$alkyl) amino, 5-tetrazolyl and 1-(1,4-dihydro-tetrazol-5-one);
$L^3$ is selected from the group consisting of -O-, -S-, -C(O)-, -C(S)-,-C(O)O-, -OC(O)-, -NR$^A$-, -N(CN)-, -NR$^A$-C(O)-, -C(O)-NR$^A$-, -NR$^A$-C(S)-, -C(S)-NR$^A$-, -NR$^A$-SO$_2$-, -SO$_2$-NR$^A$-, -NR$^A$-SO-, -SO-NR$^A$, -NR$^A$-C(O)O-, -OC(O)-NR$^A$-, -O-SO$_2$-NR$^A$-, -NR$^A$-SO$_2$-O-, -NR$^A$-C(O)-NR$^B$-, -NR$^A$-C(S)-NR$^B$- and -NR$^A$-SO$_2$-NR$^B$-;
wherein each $R^A$ and $R^B$ is independently selected from the group consisting of hydrogen, $C_{1-8}$alkyl, hydroxy substituted $C_{1-4}$alkyl, $C_{1-4}$aralkyloxy substituted $C_{1-4}$alkyl, cycloalkyl, aryl, heteroaryl, heterocycloalkyl, cycloalkyl-$C_{1-4}$alkyl-, $C_{1-4}$aralkyl, heteroaryl-$C_{1-4}$alkyl-, heterocycloalkyl-$C_{1-4}$alkyl- and spiro-heterocyclyl;
wherein the cycloalkyl, aryl, heteroaryl, heterocycloalkyl or spiro-heterocyclyl, whether alone or as part of a substituent group is optionally substituted with one or more substituents independently selected from the group consisting of halogen, hydroxy, oxo, carboxy, $C_{1-4}$alkyl, $C_{1-4}$alkoxy, $C_{1-4}$alkoxy-carbonyl, nitro, cyano, amino, $C_{1-4}$alkylamino, di($C_{1-4}$alkyl)amino,-SO$_2$-N($R^CR^D$), 5-tetrazolyl and 1-(1,4-dihydro-tetrazol-5-one)
wherein each $R^C$ and $R^D$ is independently selected from the group consisting of hydrogen and $C_{1-4}$alkyl;
$R^8$ is selected from the group consisting of $C_{1-10}$alkyl, cycloalkyl, aryl, biphenyl, partially unsaturated carbocyclyl, heteroaryl, heterocycloalkyl, cycloalkyl-$C_{1-4}$alkyl-, $C_{1-4}$aralkyl, partially unsaturated carbocyclyl-$C_{1-4}$alkyl-, heteroaryl-$C_{1-4}$alkyl-, heterocycloalkyl-$C_{1-4}$alkyl- and spiro-heterocyclyl;
wherein the $C_{1-10}$alkyl, cycloalkyl, aryl, partially unsaturated carbocyclyl, heteroaryl, heterocycloalkyl or spiro-heterocyclyl, whether alone or as part of a substituent group is optionally substituted with one or more substituents independently selected from the group consisting of halogen, hydroxy, oxo, carboxy, -C(O)-$C_{1-4}$alkyl, -C(O)-$C_{1-4}$aralkyl, -C(O)O-$C_{1-4}$alkyl, -C(O)O-$C_{1-4}$aralkyl, -$C_{1-4}$alkyl-C(O)O-$C_{1-4}$alkyl, -$C_{1-4}$alkyl-S-$C_{1-4}$alkyl, -C(O)-N($R^LR^M$), -$C_{1-4}$alkyl-C(O)-N($R^LR^M$), -NR$^L$-C(O)-$C_{1-4}$alkyl, -SO$_2$-$C_{1-4}$alkyl, -SO$_2$-aryl, -SO$_2$-N($R^LR^M$), -$C_{1-4}$alkyl-SO$_2$-N($R^LR^M$), $C_{1-4}$alkyl, fluoro substituted $C_{1-4}$alkyl, hydroxy substituted $C_{1-4}$alkyl, carboxy substituted $C_{1-4}$alkyl, $C_{1-4}$alkoxy, fluoro substituted $C_{1-4}$alkoxy, nitro, cyano, amino, $C_{1-4}$alkylamino, di($C_{1-4}$alkyl)amino, phenyl and heteroaryl;
wherein the phenyl or heteroaryl is optionally substituted with one or more substituent independently selected from the group consisting of halogen, hydroxy, oxo, carboxy, C(O)O-$C_{1-4}$alkyl, C(O)-$C_{1-4}$alkyl, $C_{1-4}$alkyl, fluoro substituted $C_{1-4}$alkyl, $C_{1-4}$alkoxy, nitro, cyano, amino, $C_{1-4}$alkylamino and di($C_{1-4}$alkyl)amino;
wherein each $R^L$ and $R^M$ is independently selected from the group consisting of hydrogen, $C_{1-4}$alkyl and cycloalkyl;
a is an integer from 0 to 3;
each $R^{10}$ is independently selected from the group consisting of hydroxy, halogen, $C_{1-4}$alkyl, $C_{1-4}$alkoxy, halogen substituted $C_{1-4}$alkyl, halogen substituted $C_{1-4}$alkoxy, -C(O)-NR$^V$R$^W$, -SO$^2$-NR$^V$R$^W$, -C(O)-$C_{1-4}$alkyl and -SO$_2$-$C_{1-4}$alkyl;
wherein each $R^V$ and $R^W$ is independently selected from the group consisting of hydrogen and $C_{1-4}$alkyl; alternatively $R^V$ and $R^W$ are taken together with the N atom to which they are bound to form a 5 to 6 membered saturated, partially unsaturated or aromatic ring structure;

provided that the halogens on the halogen substituted $C_{1-4}$alkyl or the halogen substituted $C_{1-4}$alkoxy are selected from the group consisting of chloro and fluoro;
or a pharmaceutically acceptable salt thereof.

**2.** A compound as in Claim 1 wherein
$R^0$ is selected from the group consisting of hydrogen, methyl and $CF_3$;

$R^1$ is selected from the group consisting of hydrogen, hydroxy, methyl, trifluoromethyl, methoxy and methyl-carbonyl;
$R^6$ is selected from the group consisting of $C_{1-6}$alkyl and hydroxy substituted $C_{1-6}$alkyl;
d is an integer selected from 0 to 1;
$L^2$ is selected from the group consisting of -O-, -$S(O)_{0-2}$- and -NH-;
$R^7$ is selected from the group consisting of cycloalkyl, cycloalkyl-$C_{1-4}$alkyl, aryl, $C_{1-4}$alkyl-aryl, aryl-$C_{1-4}$alkyl, heteroaryl, heteroaryl-$C_{1-4}$alkyl, heterocycloalkyl and heterocycloalkyl-$C_{1-4}$alkyl;
wherein the aryl, cycloalkyl, partially unsaturated carbocyclyl, heteroaryl or heterocycloalkyl group, whether alone or as part of a substituent group, is optionally substituted with one to two substituents independently selected from the group consisting of $C_{1-4}$alkyl, $C_{1-4}$alkoxy, halogen substituted $C_{1-4}$alkyl, halogen substituted $C_{1-4}$alkoxy, hydroxy substituted $C_{1-4}$alkyl, hydroxy, carboxy, cyano, nitro, amino, $C_{1-4}$alkylamino and di($C_{1-4}$alkyl)amino;
$L^3$ is selected from the group consisting of -C(O)-, -C(O)O-, -OC(O)-, - $NR^A$-, -N(CN)-, -$NR^A$-C(O)-, -C(O)-$NR^A$-, -$NR^A$-$SO_2$-, -$SO_2$-$NR^A$-, -$NR^A$-C(O)O- and -OC(O)-$NR^A$;
wherein $R^A$ is selected from the group consisting of hydrogen, $C_{1-4}$alkyl, hydroxy substituted $C_{1-4}$alkyl, $C_{1-4}$aralkyloxy substituted $C_{1-4}$alkyl, cycloalkyl, aryl, heteroaryl, heterocycloalkyl, cycloalkyl-$C_{1-4}$alkyl-, $C_{1-4}$aralkyl, heteroaryl-$C_{1-4}$alkyl- and heterocycloalkyl-$C_{1-4}$alkyl-;
wherein the cycloalkyl, aryl, heteroaryl or heterocycloalkyl, whether alone or as part of a substituent group is optionally substituted with one to two substituents independently selected from the group consisting of halogen, hydroxy, oxo, carboxy, $C_{1-4}$alkyl, $C_{1-4}$alkoxy, $C_{1-4}$alkoxy-carbonyl, nitro, cyano, amino, $C_{1-4}$alkylamino and di($C_{1-4}$alkyl)amino;
$R^8$ is selected from the group consisting of $C_{1-6}$alkyl, cycloalkyl, aryl, heteroaryl, heterocycloalkyl, cycloalkyl-$C_{1-4}$alkyl-, $C_{1-4}$aralkyl, heteroaryl-$C_{1-4}$alkyl- and heterocycloalkyl-$C_{1-4}$alkyl-;
wherein the $C_{1-6}$alkyl, cycloalkyl, aryl, heteroaryl or heterocycloalkyl, whether alone or as part of a substituent group is optionally substituted with one to two substituents independently selected from the group consisting of halogen, hydroxy, carboxy, -C(O)-$C_{1-4}$alkyl, -C(O)-$C_{1-4}$aralkyl, -C(O)O-$C_{1-4}$alkyl, -C(O)O-$C_{1-4}$aralkyl, -C(O)-N($R^LR^M$), -$NR^L$-C(O)-$C_{1-4}$alkyl, -$SO_2$-$C_{1-4}$alkyl, -$SO_2$-aryl, -$SO_2$-N($R^LR^M$), $C_{1-4}$alkyl, fluoro substituted $C_{1-4}$alkyl, hydroxy substituted $C_{1-4}$alkyl, carboxy substituted $C_{1-4}$alkyl, $C_{1-4}$alkoxy, fluoro substituted $C_{1-4}$alkoxy, nitro, cyano, amino, $C_{1-4}$alkylamino, di($C_{1-4}$alkyl)amino, phenyl and heteroaryl;
wherein the phenyl or heteroaryl is optionally substituted with one or more substituent independently selected from the group consisting of halogen, hydroxy, carboxy, $C_{1-4}$alkyl, fluoro substituted $C_{1-4}$alkyl, $C_{1-4}$alkoxy, nitro, cyano, amino, $C_{1-4}$alkylamino and di($C_{1-4}$alkyl)amino;
wherein each $R^L$ and $R^M$ is independently selected from the group consisting of hydrogen, $C_{1-4}$alkyl and $C_{5-8}$cycloalkyl;
a is an integer from 0 to 1;
$R^{10}$ is selected from the group consisting of hydroxy, halogen, $C_{1-4}$alkyl, $C_{1-4}$alkoxy, halogen substituted $C_{1-4}$alkyl and halogen substituted $C_{1-4}$alkoxy;
provided that the halogens on the halogen substituted $C_{1-4}$alkyl or the halogen substituted $C_{1-4}$alkoxy are selected from the group consisting of chloro and fluoro;
or a pharmaceutically acceptable salt thereof.

**3.** A compound as in Claim 2 wherein
$R^0$ is hydrogen;
$R^1$ is hydrogen;
$R^6$ is selected from the group consisting of $C_{1-6}$alkyl and hydroxy substituted $C_{1-6}$alkyl;
d is an integer selected from 0 to 1;
$L^2$ is selected from the group consisting of -O-, -S-, -SO- and -SO-;
$R^7$ is selected from the group consisting of aryl, $C_{1-4}$alkyl-aryl and aryl-$C_{1-4}$alkyl;
$L^3$ is selected from the group consisting of -NH-, -N(CN)-, -N($C_{1-4}$alkyl)-, -NH-C(O)-, -C(O)-NH-, -NH-$SO_2$-, -N($C_{1-4}$alkyl)-C(O)O- and -N(cycloalkyl)-C(O)O-;
$R^8$ is selected from the group consisting of $C_{1-4}$alkyl, cycloalkyl, cycloalkyl-$C_{1-4}$alkyl, aryl, $C_{1-4}$aralkyl, heteroaryl and heterocycloalkyl;
wherein the aryl or heteroaryl, whether alone or as part of a substituent group is optionally substituted with one to three substituents independently selected from the group consisting of halogen, hydroxy, carboxy, $C_{1-4}$alkyl, $C_{1-4}$alkoxy, fluoro substituted $C_{1-4}$alkyl, fluoro substituted $C_{1-4}$alkoxy, cyano, amino, $C_{1-4}$alkylamino, di($C_{1-4}$alkyl) amino, -C(O)O-$C_{1-4}$alkyl, -C(O)-N($R^LR^M$)-$SO_2$-$C_{1-4}$alkyl, -$SO_2$-aryl, -NH-C(O)-$C_{1-4}$alkyl, phenyl and heteroaryl;
wherein the phenyl or heteroaryl is optionally substituted with a substituent selected from the group consisting of fluoro substituted $C_{1-4}$alkyl;
wherein each $R^L$ and $R^M$ is independently selected from the group consisting of hydrogen, $C_{1-4}$alkyl and $C_{5-6}$cydoalkyl;
a is 0;

or a pharmaceutically acceptable salt thereof.

**4.** A compound as in Claim 3 wherein
$R^0$ is hydrogen;
$R^1$ is hydrogen;
$R^6$ is selected from the group consisting of n-propyl, 4-hydroxy-n-butyl and 5-hydroxy-n-perityl;
d is an integer from 0 to 1;
$L^2$ is selected from the group consisting of -O-, -S- and -SO-;
$R^7$ is selected from the group consisting of phenyl, $-CH_2$-phenyl-,-phenyl-3-$CH_2$- and -phenyl-2-$CH_2$-$CH_2$-;
$L^3$ is selected from the group consisting of -NH-, -N(CN)-, -N($CH_3$)-, - NH-C(O)-, -C(O)-NH-, -NH-$SO_2$- and -N (cyclohexyl)-C(O)O-;
wherein the $L^3$ group is bound at the 3-position of the $R^7$ group;
$R^8$ is selected from the group consisting of methyl, isopropyl, n-butyl, cyclohexyl, cyclohexyl-methyl, phenyl, phenyl-ethyl, phenyl-n-propyl, 3-(N-methyl-N-cyclohexyl-amino-carbonyl)-n-propyl, 3-bromo-phenyl, 3-methoxy-phenyl, 4-methoxy-phenyl, 2-methoxy-4-methyl-phenyl, 2,4,6-trimethyl-phenyl, 2,5-dimethoxy-phenyl, 3,4-dimethoxy-phenyl, 3-trifluoromethoxy-phenyl, 4-trifluoromethoxy-phenyl, 3-cyano-phenyl, 2-(methyl-sulfonyl)-phenyl, 4-(methyl-carbonyl-amino)-phenyl, 5-carboxy-2-methoxy-phenyl, benzyl, 3-hydroxy-benzyl, 4-methyl-benzyl, 2-methoxy-benzyl, 4-methoxy-benzyl, 2,6-dimethoxy-benzyl, 2,4,6-trimethyl-benzyl, 1-nahthyl, 2-naphthyl, 1-naphthyl-methyl, 1-(5-dimethylamino)-naphthyl, 4-biphenyl, 2-thienyl, 3-thienyl, 4-(3,5-dimethyl-isoxazolyl), 3-benzothienyl, 4-benzo [2,3,1]thiadiazolyl, 2-(5-(2-pyridyl)-thienyl), 2-(5-(3-(2-methyl-thiazolyl)-thienyl)), 2-(5-(3-(5-trifluoromethyl)-isoxazolyl)-thienyl), 6-(2,3-dihydro-benzo[1,4]dioxanyl), 3-(2-methoxy-carbonyl)-thienyl, 2-(5-(5-isoxazolyl)-thienyl)), 2-(5-bromo-thienyl) and 2-(4-phenyl-sulfonyl)-thienyl;
a is 0;
or a pharmaceutically acceptable salt thereof.

**5.** A compound as in Claim 4 wherein
$R^0$ is hydrogen;
$R^1$ is hydrogen;
$R^6$ is selected from the group consisting of n-propyl, 4-hydroxy-n-butyl and 5-hydroxy-n-pentyl;
d is an integer from 0 to 1;
$L^2$ is selected from the group consisting of -O- and -S-;
$R^7$ is selected from the group consisting of -phenyl-, -phenyl-3-$CH_2$- and phenyl-2-$CH_2$-$CH_2$-;
$L^3$ is selected from the group consisting of -NH-, -N(CN)-, -NH-C(O)-, - NH-C(O)O-, -N(cyclohexyl)-C(O)O- and -NH-$SO_2$-; wherein the $L^3$ is bound to the $R^7$ phenyl at the 3-position;
$R^8$ is selected from the group consisting of n-butyl, 3-(N-methyl-N-cyclohexyl-amino-carbonyl)-n-propyl, cyclohexyl, cyclohexyl-methyl-, phenyl, 2-trifluoromethylphenyl, 3-trifluoromethoxyphenyl, 3-cyanophenyl, 3-methoxyphenyl, 4-methoxyphenyl, 2-methoxy-4-methylphenyl, 2,4,6-trimethylphenyl, benzyl, 2-methoxybenzyl, 4-methoxbenzyl, 2,4,6-trimethylbenzyl, phenylethyl-, 2-thienyl, 3-thienyl, 2-(5-bromo-thienyl) and 3-benzothienyl;
a is 0;
or a pharmaceutically acceptable salt thereof.

**6.** A compound as in Claim 5 wherein
$R^0$ is hydrogen;
$R^1$ is hydrogen;
$R^6$ is selected from the group consisting of n-propyl and 4-hydroxy-n-butyl;
d is 1;
$L^2$ is selected from the group consisting of -O- and -S-;
$R^7$ is selected from the group consisting of -phenyl- and -phenyl-3-$CH_2$-; $L^3$ is selected from the group consisting of -NH-, -NH-C(O)- and -NH-$SO_2$-; wherein the $L^3$ is bound to the $R^7$ phenyl at the 3-position;
$R^8$ is selected from the group consisting of 3-(N-methyl-N-cyclohexyl-amino-carbonyl)-n-propyl, phenyl, 2-methoxy-4-methylphenyl, 2,4,6-trimethylphenyl, benzyl, 2-methoxybenzyl, 4-methoxybenzyl, 2,4,6-trimethylbenzyl and 3-benzothienyl;
a is 0;
or a pharmaceutically acceptable salt thereof.

**7.** A compound as in Claim 6 wherein
$R^0$ is hydrogen; $R^1$ is hydrogen; $R^6$ is n-propyl; d is 1; $L^2$ is selected from the group consisting of -O- and -S-; $R^7$ is -phenyl-; $L^3$ is -NH-$SO_2$-; wherein the $L^3$ is bound to the $R^7$ phenyl at the 3-position; $R^8$ is 2,4,6-trimethylphenyl; a

is 0; or a pharmaceutically acceptable salt thereof.

**8.** A compound as in Claim 4 wherein
$R^0$ is hydrogen; $R^1$ is hydrogen; $R^6$ is n-propyl; d is 1; $L^2$ is -0-; $R^7$ is phenyl; $L^3$ is selected from the group consisting of -NH-C(0)0-, -N(cyclohexyl)-C(0)0-, -NH-, -N(CN)- and -NH-$SO_2$; wherein the $L^3$ is bound to $R^7$ phenyl at the 3-position; $R^8$ is selected from the group consisting of 3-(N-methyl-N-cyclohexyl-amino-carbonyl)-n-propyl, cyclohexyl, cyclohexyl-methyl-, phenyl and benzyl; a is 0; or a pharmaceutically acceptable salt thereof.

**9.** A pharmaceutical composition comprising a pharmaceutically acceptable carrier and a compound of Claim 1.

**10.** A pharmaceutical composition made by mixing a compound of Claim 1 and a pharmaceutically acceptable carrier.

**11.** A process for making a pharmaceutical composition comprising mixing a compound of Claim 1 and a pharmaceutically acceptable carrier.

**12.** A compound as in Claim 1 for treating a disorder mediated by β-secretase by administering to a subject in need thereof a therapeutically effective amount of the compound.

**13.** A compound as in Claim 12, wherein the disorder mediated by β-secretase is selected from the group consisting of Alzheimer's disease (AD), mild cognitive impairment, senility, dementia, dementia with Lewy bodies, Down's syndrome, dementia associated with Parkinson's disease and dementia associated with beta-amyloid.

**14.** A composition as in Claim 9 for treating a disorder mediated by β-secretase by administering to a subject in need thereof a therapeutically effective amount of the composition.

**15.** A compound as in Claim 1 for treating a condition selected from the group consisting of Alzheimer's disease (AD), mild cognitive impairment, senility, dementia, dementia with Lewy bodies, Down's syndrome, dementia associated with Parkinson's disease and dementia associated with beta-amyloid by administering to a subject in need thereof a therapeutically effective amount of the compound.

**16.** The use of a compound as in Claim 1 for the preparation of a medicament for treating: (a) Alzheimer's disease (AD), (b) mild cognitive impairment, (c) senility, (d) dementia, (e) dementia with Lewy bodies, (f) Down's syndrome, (g) dementia associated with Parkinson's disease or (h) dementia associated with beta-amyloid, in a subject in need thereof.

**17.** A compound of formula (CIII)

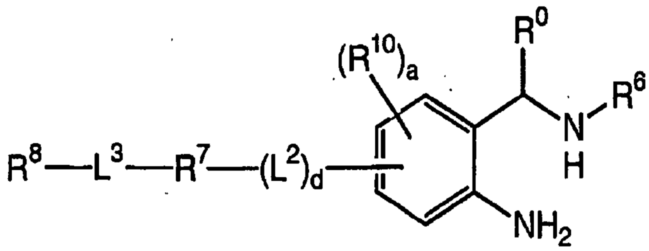

(CIII)

wherein

$R^0$ is selected from the group consisting of hydrogen, methyl and $CF_3$;
$R^6$ is selected from the group consisting of $C_{1-6}$alkyl and hydroxy substituted $C_{1-8}$alkyl;
d is an integer from 0 to 1;
$L^2$ is selected from the group consisting of -O-, -$S(O)_{0-2}$- and -$NR^Q$-;
wherein $R^Q$ is selected from the group consisting of hydrogen and $C_{1-4}$alkyl;
$R^7$ is selected from the group consisting of cycloalkyl, cycloalkyl-$C_{1-4}$alkyl, aryl, $C_{1-4}$alkyl-aryl, aryl-$C_{1-4}$alkyl, partially unsaturated carbocyclyl, partially unsaturated carbocyclyl-$C_{1-4}$alkyl, heteroaryl, heteroaryl-$C_{1-4}$alkyl, heterocycloalkyl and heterocycloalkyl-$C_{1-4}$alkyl;

wherein the aryl, cycloalkyl, partially unsaturated carbocyclyl, heteroaryl or heterocycloalkyl group, whether alone or as part of a substituent group, is optionally substituted with one or more substituents independently selected from the group consisting of $C_{1-4}$alkyl, $C_{1-4}$alkoxy, halogen substituted $C_{1-4}$alkyl, halogen substituted $C_{1-4}$alkoxy, hydroxy substituted $C_{1-4}$alkyl, hydroxy, carboxy, cyano, nitro, amino, $C_{1-4}$alkylamino, di($C_{1-4}$alkyl) amino, 5-tetrazolyl and 1-(1,4-dihydro-tetrazol-5-one);

$L^3$ is selected from the group consisting of -O-, -S-, -C(O)-, -C(S)-, - C(O)O-, -OC(O)-, -$NR^A$-, -N(CN)-, -$NR^A$-C (O)-, -C(O)-$NR^A$-, -$NR^A$-C(S)-, -C(S)- . $NR^A$-, -$NR^A$-$SO_2$-, -$SO_2$-$NR^A$-, -$NR^A$-SO-, -SO-$NR^A$, -$NR^A$-C(O)O-, -OC (O)-$NR^A$-,-O-$SO_2$-$NR^A$-, -$NR^A$-$SO_2$-O-, -$NR^A$-C(O)-$NR^B$-, -$NR^A$-C(S)-$NR^B$- and -$NR^A$-$SO_2$-$NR^B$-;

wherein each $R^A$ and $R^B$ is independently selected from the group consisting of hydrogen, $C_{1-8}$alkyl, hydroxy substituted $C_{1-4}$alkyl, $C_{1-4}$aralkyloxy substituted $C_{1-4}$alkyl, cycloalkyl, aryl, heteroaryl, heterocycloalkyl, cycloalkyl-$C_{1-4}$alkyl-, $C_{1-4}$aralkyl, heteroaryl-$C_{1-4}$alkyl-, heterocycloalkyl-$C_{1-4}$alkyl- and spiroheterocyclyl;

wherein the cycloalkyl, aryl, heteroaryl, heterocycloalkyl or spiroheterocyclyl, whether alone or as part of a substituent group is optionally substituted with one or more substituents independently selected from the group consisting of halogen, hydroxy, oxo, carboxy, $C_{1-4}$alkyl, $C_{1-4}$alkoxy, $C_{1-4}$alkoxy-carbonyl, nitro, cyano, amino, $C_{1-4}$alkylamino, di($C_{1-4}$alkyl)amino,-$SO_2$-N($R^CR^D$), 5-tetrazolyl and 1-(1,4-dihydro-tetrazol-5-one)

wherein each $R^C$ and $R^D$ is independently selected from the group consisting of hydrogen and $C_{1-4}$alkyl;

$R^8$ is selected from the group consisting of $C_{1-10}$alkyl, cycloalkyl, aryl, biphenyl, partially unsaturated carbocyclyl, heteroaryl, heterocycloalkyl, cycloalkyl-$C_{1-4}$alkyl-, $C_{1-4}$aralkyl, partially unsaturated carbocyclyl-$C_{1-4}$alkyl-, heteroaryl-$C_{1-4}$alkyl-, heterocycloalkyl-$C_{1-4}$alkyl- and spiro-heterocyclyl;

wherein the $C_{1-10}$alkyl, cycloalkyl, aryl, partially unsaturated carbocyclyl, heteroaryl, heterocycloalkyl or spiroheterocyclyl, whether alone or as part of a substituent group is optionally substituted with one or more substituents independently selected from the group consisting of halogen, hydroxy, oxo, carboxy, -C(O)-$C_{1-4}$alkyl, -C (O)-$C_{1-4}$aralkyl, -C(O)O-$C_{1-4}$alkyl, -C(O)O-$C_{1-4}$aralkyl, -$C_{1-4}$alkyl-C(O)O-$C_{1-4}$alkyl, -$C_{1-4}$alkyl-S-$C_{1-4}$alkyl, -C (O)-N($R^LR^M$), -$C_{1-4}$alkyl-C(O)-N($R^LR^M$), -$NR^L$-C(O)-$C_{1-4}$alkyl, -$SO_2$-$C_{1-4}$alkyl, -$SO_2$-aryl, -$SO_2$-N($R^LR^M$), -$C_{1-4}$alkyl-SO2-N($R^LR^M$), $C_{1-4}$alkyl, fluoro substituted $C_{1-4}$alkyl, hydroxy substituted $C_{1-4}$alkyl, carboxy substituted $C_{1-4}$alkyl, $C_{1-4}$alkoxy, fluoro substituted $C_{1-4}$alkoxy, nitro, cyano, amino, $C_{1-4}$alkylamino, di($C_{1-4}$alkyl)amino, phenyl and heteroaryl;

wherein the phenyl or heteroaryl is optionally substituted with one or more substituent independently selected from the group consisting of halogen, hydroxy, oxo, carboxy, C(O)O-$C_{1-4}$alkyl, C(O)-$C_{1-4}$alkyl, $C_{1-4}$alkyl, fluoro substituted $C_{1-4}$alkyl, $C_{1-4}$alkoxy, nitro, cyano, amino, $C_{1-4}$alkylamino and di($C_{1-4}$alkyl)amino;

wherein each $R^L$ and $R^M$ is independently selected from the group consisting of hydrogen and $C_{1-4}$alkyl;

a is an integer from 0 to 3;

each $R^{10}$ is independently selected from the group consisting of hydroxy, halogen, $C_{1-4}$alkyl, $C_{1-4}$alkoxy, halogen substituted $C_{1-4}$alkyl, halogen substituted $C_{1-4}$alkoxy, -C(O)-$NR^VR^W$, -$SO_2$-$NR^VR^W$, -C(O)-$C_{1-4}$alkyl and -$SO_2$-$C_{1-4}$alkyl;

wherein each $R^V$ and $R^W$ is independently selected from the group consisting of hydrogen and $C_{1-4}$alkyl; alternatively $R^V$ and $R^W$ are taken together with the N atom to which they are bound to form a 5 to 6 membered saturated, partially unsaturated or aromatic ring structure;

provided that the halogens on the halogen substituted $C_{1-4}$alkyl or the halogen substituted $C_{1-4}$alkoxy are selected from the group consisting of chloro and fluoro;
or a pharmaceutically acceptable salt thereof.

## Patentansprüche

1. Eine Verbindung mit der Formel (III)

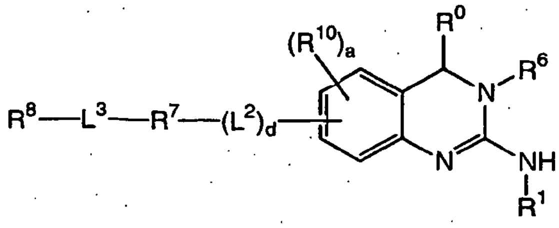

(III).

bei der

R° aus der Gruppe bestehend aus Wasserstoff, Methyl und CF3 ausgewählt wird;
$R^1$ aus der gruppe bestehend aus Wasserstoff, Hydroxy, Methyl, Ethyl, Trifluormethyl, 2,2,2-Trifluorethyl, Methoxy, Ethoxy und Methylcarbonyl ausgewählt wird;
$R^6$ aus der Gruppe bestehend aus $C_{1-6}$-alkyl durch Hydroxy-substituiertem $C_{1-8}$-alkyl ausgewählt wird;
d eine Ganzzahl von 0 bis 1 ist;
$L^2$ aus der Gruppe bestehend aus -O-, 5 -$S(O)_{0-2}$- und -$NR^Q$- ausgewählt wird;
wobei $R^Q$ aus der Gruppe bestehend aus Wasserstoff und $C_{1-4}$-alkyl ausgewählt wird;
$R^7$ aus der Gruppe bestehend aus Cycloalkyl, Cycloalkyl-$C_{1-4}$-alkyl, Aryl, $C_{1-4}$-alkylaryl, Aryl-$C_{1-4}$-alkyl, teilweise ungesättigtem Carbocyclyl, teilweise ungesättigtem Carbocyclyl-$C_{1-4}$-alkyl, Heteroaryl, Heteroaryl-$C_{1-4}$-alkyl, Heterocycloalkyl und Heterocycloalkyl-$C_{1-4}$-alkyl ausgewählt wird;
wobei die Aryl-, Cycloalkyl-, teilweise ungesättigte Carbocyclyl-, Heteroaryl- oder Heterocycloalkyl-Gruppe, weder allein oder als Teil einer Substituentengruppe, optionsweise durch einen oder mehrere Substituenten substituiert wird, die unabhängig aus der Gruppe bestehend aus folgenden ausgewählt werden, nämlich $C_{1-4}$-alkyl, $C_{1-4}$-alkoxy, Halogen substituiertem $C_{1-4}$-alkyl, Halogen substituiertem $C_{1-4}$-alkoxy, Hydroxy substituiertem $C_{1-4}$-alkyl, Hydroxy, Carboxy, Cyan, Nitro, Amino, $C_{1-4}$-alkylamino, di($C_{1-4}$-alkyl)amino, 5-tetrazolyl und 1-(1,4-dihydro-tetrazol-5-on);
$L^3$ aus der Gruppe bestehend aus folgenden ausgewählt wird, nämlich -O-, -S-, - C(O)-, -C(S)-, -C(O)O-, -OC (O)-, -$NR^A$-, -N(CN)-, -$NR^A$-C(O)-, -C(O)-$NR^A$-, -$NR^A$-C(S)-, -C(S)-, NRA-, -NRA-$SO_2$-, -$SO_2$-$NR^A$-, -$NR^A$-SO-, -SO-$NR^A$, -$NR^A$-C(O)O-, - OC(O)-$NR^A$-, -O-$SO_2$-$NR^A$-, -$NR^A$-$SO_2$-O-, -$NR^A$-C(O)-$NR^B$-, -$NR^A$-C(S)-$NR^B$- und - $NR^A$-$SO_2$-$NR^B$-;
wobei jedes $R^A$ und $R^B$ unabhängig aus der Gruppe bestehend aus Wasserstoff, $C_{1-8}$-alkyl, Hydroxy substituiertem $C_{1-4}$-alkyl, $C_{1-4}$aralkyloxy substituiertem $C_{1-4}$-alkyl, Cycloalkyl, Aryl, Heteroaryl, Heterocycloalkyl, Cycloalkyl- $C_{1-4}$-alkyl-, $C_{1-4}$-aralkyl, Heteroaryl- $C_{1-4}$-alkyl-, Heterocycloalkyl- $C_{1-4}$-alkyl- und Spiro-heterocyclyl;
wobei das Cycloalkyl, Aryl, Heteroaryl, Heterocycloalkyl oder Spiro-heterocyclyl, egal ob allein oder als Teil einer Substituentengruppe optional mit einem oder mehreren Substituenten, die unabhängig aus der folgenden Gruppe ausgewählt werden, substituiert wird, nämlich Halogen, Hydroxy, Oxo, Carboxy, $C_{1-4}$-alkyl, $C_{1-4}$-alkoxy, $C_{1-4}$-alkoxycarbonyl, Nitro, Cyan, Amino, $C_{1-4}$-alkylamino, di($C_{1-4}$-alkyl)amino, -$SO_2$-N($R^CR^D$), 5-tetrazolyl und 1 -(1,4-dihydro-tetrazol-5-on)
wobei jedes $R^C$ und $R^D$ unabhängig aus der gruppe bestehend aus Wasserstoff und $C_{1-4}$-alkyl ausgewählt wird,
$R^8$ aus der Gruppe bestehend aus $C_{1-10}$-alkyl, Cycloalkyl, Aryl, Biphenyl, teilweise ungesättigtem Carbocyclyl, Heteroaryl, Heterocycloalkyl, Cycloalkyl- $C_{1-4}$-alkyl-, $C_{1-4}$-aralkyl, teilweise ungesättigtem Carbocyclyl- $C_{1-4}$-alkyl-, heteroaryl- $C_{1-4}$-alkyl-, heterocycloalkyl- $C_{1-4}$-alkyl- und Spiro-heterocyclyl ausgewählt wird;
wobei das $C_{1-10}$alkyl, Cycloalkyl, Aryl, das teilweise ungesättigte Carbocyclyl, Heteroaryl, Heterocycloalkyl oder Spiro-heterocyclyl, entweder allein oder als Teil einer Substituentengruppe optional durch einen oder mehrere Substituenten substituiert wird, die unabhängig aus der Gruppe bestehend aus Folgenden ausgewählt werden, nämlich Halogen, Hydroxy, Oxo, Carboxy, -C(O)-$C_{1-4}$alkyl, - C(O)- $C_{1-4}$aralkyl, -C(O)O-$C_{1-4}$alkyl, -C(O)O-$C_{1-4}$aralkyl, - $C_{1-4}$alkyl-C(O)O-$C_{1-4}$-alkyl, - $C_{1-4}$alkyl-S- $C_{1-4}$alkyl, -C(O)-N($R^LR^M$), - $C_{1-4}$alkyl-C(O)-N($R^LR^M$), -$NR^L$-C(O)- $C_{1-4}$alkyl,- $SO_2$- $C_{1-4}$alkyl, -$SO_2$-aryl, -$SO_2$-N($R^LR^M$), - $C_{1-4}$alkyl-$SO_2$-N($R^LR^M$), $C_{1-4}$alkyl, Fluor substituiertes $C_{1-4}$alkyl, Hydroxy substituiertes $C_{1-4}$alkyl, Carboxy substituiertes $C_{1-4}$alkyl, $C_{1-4}$alkoxy, Fluor substituiertes $C_{1-4}$alkoxy, Nitro, Cyan, Amino, $C_{1-4}$alkylamino, di($C_{1-4}$alkyl)amino, Phenyl und Heteroaryl;
wobei das Phenyl oder Heteroaryl optional durch einen oder mehrere Substituenten substituiert wird, die unabhängig aus der gruppe bestehen aus Halogen, Hydroxy, Oxo, Carboxy, C(O)O- $C_{1-4}$alkyl, C(O)- $C_{1-4}$alkyl, $C_{1-4}$alkyl, Fluor substituiertes $C_{1-4}$alkyl, $C_{1-4}$alkoxy, Nitro, Cyan, Amino, $C_{1-4}$alkylamino und di($C_{1-4}$alkyl)amino ausgewählt werden;

wobei jedes $R^L$ und $R^M$ unabhängig aus der Gruppe bestehend aus Wasserstoff und $C_{1-4}$alkyl ausgewählt wird;
a eine Ganzzahl von 0 bis 3 ist;
jedes $R^{10}$ unabhängig aus der Gruppe bestehend aus Hydroxy, Halogen, $C_{1-4}$alkyl, $C_{1-4}$alkoxy, Halogen substituiertem $C_{1-4}$alkyl, Halogen substituiertem $C_{1-4}$alkoxy, - C(O)-$NR^VR^W$, -$SO_2$-$NR^VR^W$, -C(O)- $C_{1-4}$alkyl und -$SO_2$- $C_{1-4}$alkyl ausgewählt wird;

wobei jedes $R^V$ und $R^W$ unabhängig aus der Gruppe bestehend aus Wasserstoff und $C_{1-4}$alkyl ausgewählt werden; alternativ werden $R^V$ und $R^W$ zusammen mit dem N-Atom, mit dem sie verbunden sind, genommen, um eine gesättigte, teilweise ungesättigte oder aromatische Ringstruktur mit 5 bis 6 Gliedern zu bilden; y
vorausgesetzt, dass die Halogen am Halogen substituierten $C_{1-4}$alkyl oder Halogen substituierten $C_{1-4}$alkoxy aus der Gruppe bestehend aus Chlor oder Fluor oder einem pharmazeutisch verträglichen Salz dieser ausgewählt werden.

**2.** Eine Verbindung gemäß Anspruch 1, wobei
R° aus der Gruppe bestehend aus Wasserstoff, Methyl und $CF_3$ ausgewählt wird;
$R^1$ aus der Gruppe bestehend aus Wasserstoff, Hydroxy, Methyl, Trifluormethyl, Methoxy, Ethoxy und Methylcarbonyl ausgewählt wird;
$R^6$ aus der Gruppe bestehend aus $C_{1-6}$alkyl und Hydroxy substituiertem $C_{1-6}$alkyl ausgewählt wird;
d eine Ganzzahl ist, die von 0 bis ausgewählt wird;
$L^2$ aus der Gruppe bestehend aus -O-, $S(O)_{0-2}$ und -NH- ausgewählt wird;
$R^7$ aus der Gruppe bestehend aus Cycloalkyl, Cycloalkyl- $C_{1-4}$alkyl, Aryl, $C_{1-4}$alkylaryl, Aryl-$C_{1-4}$alkyl, Heteroaryl, Heteroaryl- $C_{1-4}$alkyl, Heterocycloalkyl und Heterocycloalkyl-$C_{1-4}$alkyl ausgewählt wird;
wobei die Aryl-, Cycloalkyl-, teilweise ungesättigte Carbocyclyl-, Heteroaryl- oder Heterocycloalkyl-Gruppe entweder allein oder als Teil einer Substituentengruppe optional durch einen bis zwei Substituenten substituiert werden, die unabhängig aus der Gruppe bestehend aus Folgenden ausgewählt werden, nämlich $C_{1-5}$alkyl, $C_{1-4}$alkoxy, Halogen substituiertes $C_{1-4}$alkyl, Halogen substituiertes $C_{1-4}$alkoxy, Hydrogen substituiertes $C_{1-4}$alkyl, Hydroxy, Carboxy, Cyan, Nitro, Amino, $C_{1-4}$alkylamino und di($C_{1-4}$ -alkyl(amino;
wobei L3 aus der Gruppe bestehend aus -C(O)-, -C(O)O-, -OC(O)-, -$NR^A$-, -N(CN)-, -$NR^A$-C(O)-, -C(O)-$NR^A$-, $NR^A$-$SO_2$-, -$SO_2$-$NR^A$, -$NR^A$-C(O)O-, und -OC(O)$NR^A$ ausgewählt wird;
wobei $R^A$ aus der Gruppe bestehend aus Folgenden ausgewählt wird, nämlich Wasserstoff, $C_{1-4}$alkyl, Hydroxy substituiertes $C_{1-4}$alkyl, $C_{1-4}$aralkyloxy substituiertes $C_{1-4}$alkyl, Cycloalkyl, Aryl, Heteroaryl, Heterocycloalkyl, Cycloalkyl- $C_{1-4}$alkyl-, $C_{1-4}$aralkyl, Heteroaryl-$C_{1-4}$alkyl und Heterocycloalkyl-$C_{1-4}$alkyl_;
wobei das Cycloalkyl, Aryl, Heteroaryl oder Heterocycloalkyl, entweder allein oder als Teil einer Substituentengruppe optional durch einen oder zwei Substituenten substituiert werden, die unabhängig aus der Gruppe bestehend aus Halogen, Hydroxy, Oxo, Carboxy, $C_{1-4}$alkyl, $C_{1-4}$alkoxy, $C_{1-4}$alkoxy-carbonyl, Nitro, Cyan, Amino, $C_{1-4}$alkylamino und di($C_{1-4}$alkyl)amino ausgewählt werden;
wobei $R^8$ aus der Gruppe bestehend aus $C_{1-6}$alkyl, Cycloalkyl, Aryl, Heteroaryl, Heterocycloalkyl, Cycloalkyl-$C_{1-4}$alkyl, $C_{1-4}$aralkyl, Heteroaryl- $C_{1-4}$alkyl und heterocycloalkyl-$C_{1-4}$alkyl ausgewählt wird;
wobei das $C_{1-4}$alkyl, Cycloalkyl, Aryl, Heteroaryl oder Heterocycloalkyl, entweder allein oder als Teil einer Substituentengruppe, optional durch ein oder zwei Substituenten substituiert wird, die unabhängig aus der Gruppe bestehen aus Folgenden ausgewählt wird, nämlich Halogen, Hydroxy, Carboxy, C(O)-$C_{1-4}$alkyl, - C(O)O-$C_{1-2}$aralkyl, C(O)O- $C_{1-4}$alkyl, -C(O)O- $C_{1-4}$aralkyl, C(O)-N($R^LR^M$), NRL-C(O)-$C_{1-4}$alkyl, $SO_2$- $C_{1-4}$alkyl, -$SO_2$-aryl, - $SO_2$-N($R^L$-$R^M$), $C_{1-4}$alkyl, Fluor subsituiertes $C_{1-4}$alkyl, Hydroxy substituiertes $C_{1-4}$alkyl, Carboxy substituiertes $C_{1-4}$alkyl, $C_{1-4}$alkoxy, Fluor substituiertes $C_{1-4}$alkoxy, Nitro, Cyan, Amino, $C_{1-4}$alkylamino, di($C_{1-4}$alkyl)amino, Phenyl und heteroaryl;
Wobei das Phenyl oder Heteroaryl optional durch einen oder mehrere Substituenten substituiert wird, die aus der Gruppe bestehend aus Halogen, Hydroxy, Carboxy, $C_{1-4}$alkyl, Fluor substituiertes $C_{1-4}$alkyl, $C_{1-4}$alkoxy, Nitro, Cyan, Amino, $C_{1-4}$alkylamino und di($C_{1-4}$alkyl)amino ausgewählt werden;
Wobei jedes $R^L$ und $R^M$ unabhängig aus der Gruppe bestehend aus Wasserstoff, $C_{1-4}$alkyl und $C_{5-8}$cydoalkyl ausgewählt wird; Phenyl und heteroaryl; 5-tetrazolyl und 1-(1,4-dihydro-tetrazol-5-on);
a eine Ganzzahl von 0 bis 1 ist;
$R^{10}$ aus der Gruppe bestehend aus Hydroxy, Halogen, $C_{1-4}$alkyl, $C_{1-4}$alkoxy, Halogen substituiertem $C_{1-4}$alkyl und Halogen substituiertem $C_{1-4}$alkoxy ausgewählt wird;
vorausgesetzt, dass die Halogene am Halogen substituierten $C_{1-4}$alkyl oder Halogen substituiertem $C_{1-4}$alkoxy aus der Gruppe bestehend aus Chlor und Fluor oder einem pharmazeutisch verträglichen Salz dieser ausgewählt werden.

**3.** Eine Verbindung gemäß Anspruch 2, wobei

$R^0$ Wasserstoff ist.
$R^1$ Wasserstoff ist
$R^6$ aus der Gruppe bestehend aus $C_{1-4}$alkyl und Hydroxy substituiertes $C_{1-6}$alkyl ausgewählt wird;
d eine Ganzzahl ist, die von 0 bis 1 ausgewählt wird;
$L^2$ aus der Gruppe bestehend aus -O-; -S-, -SO- und -SO- ausgewählt wird;
$R^7$ aus der Gruppe bestehend aus Aryl, $C_{1-4}$alkyl-aryl und Aryl-$C_{1-4}$alkyl ausgewählt wird;
$L^3$ aus der Gruppe bestehend aus -NH-, -N(CN)-, -N($C_{1-4}$alkyl), -NH-C(O)-, -C(O)-NH-, NH-$SO_2$-, N($C_{1-4}$alkyl)-C(O)O- und N(cycloalkyl)-C(O)O ausgewählt wird;
$R^8$ aus der Gruppe bestehend aus $C_{1-4}$alkyl, Cycloalkyl, Cycloalkyl- $C_{1-4}$alkyl, Aryl, $C_{1-4}$aralkyl, Heteroaryl und Heterocycloalkyl ausgewählt wird;
wobei das Aryl oder Heteroaryl, entweder allein oder als Teil einer Substituentengruppe optional durch ein bis drei Substituenten substituiert wird, die unabhängig aus der Gruppe bestehend aus Halogen, Hydroxy, Carboxy, $C_{1-4}$alkyl, $C_{1-4}$alkoxy, Fluor substituiertem $C_{1-2}$alkyl, Fluor substituiertem $C_{1-4}$alkoxy, Cyan, Amino, $C_{1-4}$alkylamino, di($C_{1-4}$alkyl)amino, -C(O)O- $C_{1-4}$alkyl, -C(O)-N($R^LR^M$)-$SO_2$- $C_{1-4}$alkyl, -$SO_2$-aryl, -NH-C(O)- $C_{1-4}$alkyl, Phenyl und Heteroaryl ausgewählt wird;
wobei das Phenyl oder Heteroaryl optional durch einen Substituenten substituiert wird, der aus der Gruppe bestehend aus Fluor substituiertem $C_{1-4}$alkyl ausgewählt wird;
wobei $R^L$ und $R^M$ jeweils unabhängig aus der Gruppe bestehend aus Wasserstoff, $C_{1-4}$alkyl und $C_{5-6}$cycloalkyl ausgewählt werden;
a 0 ist;
oder ein pharmazeutisch verträgliches Salz dieser.

**4.** Eine Verbindung gemäß Anspruch 3, wobei
$R^0$ Wasserstoff ist.
$R^1$ Wasserstoff ist
$R^6$ aus der Gruppe bestehend aus n-propyl, 4-hydroxy-n-butyl und 5-hydroxy-n-pentyl ausgewählt wird;
d eine Ganzzahl von 0 bis 1 ist;
$L^2$ aus der Gruppe bestehend aus -O-, -S- und -SO ausgewählt wird;
$R^7$ aus der Gruppe bestehend aus Phenyl, -$CH_2$-phenyl-, phenyl-3-$CH_2$ und phenyl-2-$CH_2$-$CH_2$ ausgewählt wird;
$L^3$ aus der Gruppe bestehend aus -NH-, -N(CN)-, -N($CH_3$)-, NH-C(O)-, -C(O)-NH, - NH-$SO_2$- und N(cyclohexyl)-C(O)O- ausgewählt wird;
wobei die $L^3$-Gruppe an der 3-Position der $R^7$-Gruppe gebunden ist;
$R^8$ aus der Gruppe bestehend aus Folgenden ausgewählt wird, nämlich Methyl, Isopropyl, n-butyl, cyclohexyl, cyclohexyl-methyl, phenyl, phenyl-ethyl, phenyl-n-propyl, 3-(N-methyl-N-cyclohexyl-amino-carbonyl)-n-propyl, 3-brom-phenyl, 3-methoxy-phenyl, 4-methoxy-phenyl, 3,4-dimethoxy-phenyl" 3-trifluormethoxy-phenyl, 4-trifluormethoxy-phenyl, 3-cyan-phenyl, 2-(methyl-sulfonyl)-phenyl, 4-(methylcarbonyl-amino)-phenyl, 5-carboxy-2-methoxy-phenyl, benzyl, 3-hydroxy-benzyl, 4-methyl-benzyl, 2-methoxy-benzyl, 4-methoxy-benzyl, 2,6-dimethoxy-benzyl, 2,4,6-trimethyl-benzyl, 1-naphthyl, 2-naphthyl, 1-naphthtyl-methyl, 1-(5-dimethylamino(-naphthyl, 4-biphenyl, 2-thienyl, 3-thienyl, 4-((3,5,-dimethyl-isoxazolyl), 3-benzothienyl, 4-benzo[2,3,1]thiadiazolyl, 2-(5-(2-pyridyl)-thienyl), 2-(5-(3-)2-methylthiazolyl)thienyl, 2-(5-(3-(5-trifluormethyl)isoxazolyl)-thienyl), 6-(2,3-dihydrobenzo[1,4]dioxanyl), 3-(2-methoxy-carbonyl)-thienyl, 2-(5-(5-isoxazolyl)-thienyl), 2-(5-brom-thienyl) und 2,(4-phenyl-sulfonyl)-thienyl;
a 0 ist;
oder ein pharmazeutisch verträgliches Salz dieser.

**5.** Eine Verbindung gemäß Anspruch 4, wobei
$R^0$ Wasserstoff ist.
$R^1$ Wasserstoff ist
$R^6$ aus der Gruppe bestehend aus n-propyl, 4-hydroxy-n-butyl und 5-hydroxy-n-pentyl ausgewählt wird;
d eine Ganzzahl von 0 bis 1 ist;
$L^2$ aus der Gruppe bestehend aus -O- und -S- ausgewählt wird;
$R^7$ aus der Gruppe bestehend aus -phenyl, -phenyl-3-$CH_2$ und phenyl-2-$CH_2$-$CH_2$ ausgewählt wird;
$L^3$ aus der Gruppe bestehend aus -NH-, -N(CN)-, NH-C(O)-, NH-C(O)O-, und N(cyclohexyl)-C(O)O- und -NH-$SO_2$-ausgewählt wird; wobei die $L^3$-Gruppe an der 3-Position der $R^7$-Gruppe gebunden ist;
$R^8$ aus der Gruppe bestehend aus Folgenden ausgewählt wird, nämlich n-butyl, 3-(N-methyl-N-cyclohexyl-amino-carbonyl)-n-propyl, Cyclohexyl, Cyclohexyl-methyl-, 2-trifluormethylphenyl, 3-trifluormethoxyphenyl, 3-cyanphenyl, 3-methoxyphenyl, 4-methoxyphenyl, 2-methoxy-4-methylphenyl, 2,4,6-trimethyl-phenyl, Benzyl, 2-methoxybenzyl, 4-methoxybenzyl, 2,4,6-trimethylbenzyl, phenylethyl-, 2-thienyl, 3-thienyl, 2-(5-brom-thienyl) und 3-benzothienyl;
a 0 ist;

oder ein pharmazeutisch verträgliches Salz dieser.

6. Eine Verbindung gemäß Anspruch 5, wobei
$R^0$ Wasserstoff ist.
$R^1$ Wasserstoff ist
$R^6$ aus der Gruppe bestehend aus n-propyl und 4-hydroxy-n-butyl ausgewählt wird;
d 1 ist;
$L^2$ aus der Gruppe bestehend aus -O- und -S- ausgewählt wird;
$R^7$ aus der Gruppe bestehend aus -phenyl und -phenyl-3-$CH_2$ ausgewählt wird; $L^3$ aus der Gruppe bestehend aus -NH-, NH-C(O)- und -NH-$SO_2$- ausgewählt wird; wobei die $L^3$-Gruppe an der 3-Position der $R^7$-Gruppe gebunden ist;
$R^8$ aus der Gruppe bestehend aus Folgenden ausgewählt wird, nämlich 3-(N-methyl-N-cyclohexyl-amino-carbonyl)-n-propyl, Phenyl, 2-methoxy-4-methylphenyl, 2,4,6-trimethylphenyl, Benzyl, 2-methoxybenzyl, 4-methoxybenzyl, 2,4,6-trimethylbenzyl und 3-benzothienyl;
a 0 ist;
oder ein pharmazeutisch verträgliches Salz dieser.

7. Eine Verbindung gemäß Anspruch 6, wobei
$R^0$ Wasserstoff ist.
$R^1$ Wasserstoff ist
$R^6$ n-propyl ist;
d 1 ist;
$L^2$ aus der Gruppe bestehend aus -O- und -S- ausgewählt wird;
$R^7$ -phenyl ist;
$L^3$ -NH-$SO_2$- ist; wobei die $L^3$-Gruppe an der 3-Position der $R^7$-Gruppe gebunden ist;
$R^8$ 2,4,6-trimethylphenyl ist;
a 0 ist;
oder ein pharmazeutisch verträgliches Salz dieser.

8. Eine Verbindung gemäß Anspruch 4, wobei
$R^0$ Wasserstoff ist.
$R^1$ Wasserstoff ist
$R^6$ n-propyl ist;
d 1 ist;
$L^2$-O- ist;
$R^7$-phenyl ist;
$L^3$ aus der Gruppe bestehend aus -NH-C(O)O-, N(cyclohexyl)-C(O)O, - NH-, - N(CN)- und NH-$SO_2$- ausgewählt wird; wobei die $L^3$-Gruppe an der 3-Position der $R^7$-Gruppe gebunden ist;
$R^8$ aus der Gruppe bestehend aus Folgenden ausgewählt wird, nämlich 3-(N-methyl-N-cyclohexyl-amino-carbonyl)-n-propyl, Cyclohexyl, Cyclohexyl-methyl, Phenyl und Benzyl;
a 0 ist;
oder ein pharmazeutisch verträgliches Salz dieser.

9. Eine pharmazeutische Zusammensetzung bestehend aus einem pharmazeutisch verträglichen Trägerstoff und einer Verbindung gemäß Anspruch 1.

10. Eine pharmazeutische Zusammensetzung, die durch Mischen einer Verbindung gemäß Anspruch 1 und einem pharmazeutische verträglichen Trägerstoff erstellt wird.

11. Ein Prozess zur Erstellung einer pharmazeutischen Zusammensetzung bestehend aus Mischen einer Verbindung gemäß Anspruch 1 und einem pharmazeutisch verträglichen Trägerstoff.

12. Eine Verbindung gemäß Anspruch 1 zur Behandlung einer durch β-Sekretase vermittelten Krankheit, indem dem Betroffenen eine therapeutisch wirksame Menge der Verbindung verabreicht wird.

13. Die Verbindung gemäß Anspruch 12, wobei die durch β-Sekretase vermittelte Krankheit zur folgenden Krankheitsgruppe gehört, nämlich Alzheimer-Krankheit (AD), milde kognitive Störung, Senilität, Demenz, Lewy-Körper-Demenz, Down-Syndrom, mit Parkinson-Krankheit assoziierte Demenz und mit Beta-Amyloid assoziierte Demenz.

**14.** Eine Verbindung gemäß Anspruch 9 zur Behandlung einer durch β-Sekretase vermittelten Krankheit, indem dem Betroffenen eine therapeutisch wirksame Menge der Verbindung verabreicht wird.

**15.** Eine Verbindung gemäß Anspruch 1, zur Behandlung einer Krankheit, die zur folgenden Krankheitsgruppe gehört, nämlich Alzheimer-Krankheit (AD), milde kognitive Störung, Senilität, Demenz, Lewy-Körper-Demenz, Down-Syndrom, mit Parkinson-Krankheit assoziierte Demenz und mit Beta-Amyloid assoziierte Demenz, indem dem Betroffenen eine therapeutisch wirksame Menge der Verbindung verabreicht wird.

**16.** Die Verwendung einer Verbindung gemäß Anspruch 1 zur Herstellung eines Medikaments zur Behandlung eine mit einer der folgenden Krankheiten Betroffenen: (a) Alzheimer-Krankheit (AD), (b) milde kognitive Störung, (c) Senilität, (d) Demenz, (e) Lewy-Körper-Demenz, (f)
Down-Syndrom, (g) mit Parkinson-Krankheit assoziierte Demenz und (h) mit Beta-Amyloid assoziierte Demenz.

**17.** Eine Verbindung mit der Formel (CIII)

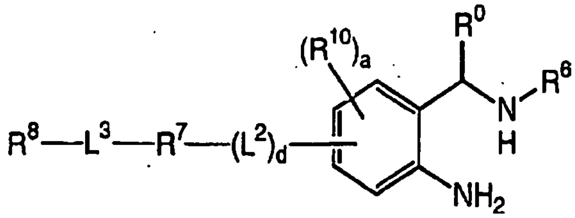

(CIII)

wobei

$R^{\circ}$ aus der Gruppe bestehend aus Wasserstoff, Methyl und $CF_3$ ausgewählt wird;
$R^6$ aus der Gruppe bestehend aus $C_{1-6}$ und Hydroxy substituiertem $C_{1-8}$alkyl ausgewählt wird;
d eine Ganzzahl von 0 bis 1 ist;
$L^1$ aus der Gruppe bestehend aus -O-, $-S(O)_{0-2}$- und $-NR^Q$ ausgewählt wird; wobei $R^Q$ aus der Gruppe bestehend aus Wasserstoff und $C_{1-4}$alkyl ausgewählt wird;
$R^7$ aus der Gruppe bestehend aus Folgenden ausgewählt wird, nämlich Cycloalkyl, Cycloalkyl- $C_{1-4}$alkyl,, Aryl, $C_{1-4}$alkyl-aryl, aryl-$C_{1-4}$alkyl, teilweise ungesättigtem Carbocyclyl, teilweise ungesättigtem Carbocyclyl-$C_{1-4}$alkyl, Heteroaryl, Heteroaryl-$C_{1-4}$alkyl-, Heterocycloalkyl und Heterocyclolalkyl-$C_{1-4}$alkyl;
wobei die Aryl-, Cycloalkyl-, teilweise ungesättigte Carbocyclyl-, Heteroaryl- oder, Heterocycloalkyl-Gruppe, entweder allein oder als Teil einer Substituentengruppe optional durch einen oder mehrere Substituenten substituiert wird, die unabhängig aus der Gruppe bestehend aus Folgenden ausgewählt werden, nämlich $C_{1-4}$alkyl, $C_{1-4}$alkoxy, Halogen substituiertes $C_{1-4}$alkyl, Halogen substituiertes $C_{1-4}$alkoxy, Hydroxy substituiertes $C_{1-4}$alkyl, Hydroxy, Carboxy, Cyan, Nitro, Amino, $C_{1-4}$alkylamino, di($C_{1-4}$alkyl(amino, 5-tetrazol und 1-(1,4-dihydro-tetrazol-5-on);
$L^3$ aus der Gruppe bestehend aus -O-, -S-, -C(O)-, -C(S)-, -C(O)O-, -OC(O)-, - $NR^A$-, N(CN), $-NR^A$-C(O)-, -C (O)-$NR^A$-, $-NR^A$-C(S)-, -C(S)-$NR^A$-, $NR^A$-$SO_2$, $-SO_2$-$NR^A$-, $-NR^A$-SO-, -SO-$NR^A$, $-NR^A$-C(O),O-, -OC(O)-$NR^A$, -O-$SO_2$-$NR^A$-, $-NR^A$-$SO_2$-O-, $-NR^A$-C(O)-$NR^B$-, $-NR^A$-C(S)-$NR^B$- und $-NR^A$-$SO_2$-$NR^B$-;
wobei jedes $R^A$ und $R^B$ jeweils unabhängig aus der Gruppe bestehend aus Wasserstoff, $C_{1-8}$-alkyl, Hydroxy substituiertem $C_{1-4}$-alkyl, $C_{1-4}$-aralkyloxy substituiertem $C_{1-4}$-alkyl, Cycloalkyl, Aryl, Heteroaryl, Heterocycloalkyl, Cycloalkyl-$C_{1-4}$-alkyl-, $C_{1-4}$-aralkyl, Heteroaryl- $C_{1-4}$-alkyl-, Heterocycloalkyl-$C_{1-4}$-alkyl und Spiro-heterocyclyl ausgewählt wird;
wobei das Cycloalkyl, Aryl, Heteroaryl, Heterocycloalkyl oder Spiro-heterocyclyl, entweder allein oder als Teil einer Substituentengruppe optional durch einen oder mehrere Substituenten substituiert wird, die unabhängig aus der Gruppe bestehend aus Folgenden ausgewählt werden, nämlich Halogen, Hydroxy, Oxo, Carboxy, $C_{1-4}$-alkyl, $C_{1-4}$-alkoxy, $C_{1-4}$-alkoxy-carbonyl, Nitro, Cyan, Amino, $C_{1-4}$-alkylamino, di($C_{1-4}$alkyl)amino,-$SO_2$-N ($R^CR^D$), 5-tetrazolyl und 1-(1,4-dihydro-tetrazol-5-on);
wobei $R^C$ und $R^D$ jeweils unabhängig aus der Gruppe bestehend aus Wasserstoff, und $C_{1-4}$-alkyl ausgewählt werden;
$R^8$ aus der Gruppe bestehend aus $C_{1-10}$alkyl, Cycloalkyl, Aryl, Biphenyl, teilweise ungesättigtem Carbocyclyl, Heteroaryl, Heterocycloalkyl, Cycloalkyl-$C_{1-4}$alkyl-, $C_{1-4}$aralkyl, teilweise ungesättigtem Carbocyclyl-$C_{1-4}$alkyl-, Heteroaryl-$C_{1-4}$alkyl-, Heterocycloalkyl-$C_{1-4}$alkyl- und Spiro-heterocyclyl ausgewählt wird;

wobei das $C_{1-10}$alkyl, Cycloalkyl, Aryl, teilweise ungesättigte Carbocyclyl, Heteroaryl, Heterocycloalkyl oder Spiro-heterocyclyl, entweder allein oder als Teil einer Substituentengruppe optional durch einen oder mehrere Substituenten substituiert wird, die unabhängig aus einer Gruppe bestehend aus Folgenden ausgewählt werden, nämlich Halogen, Hydroxy, Oxo, Carboxy, -C(O)-$C_{1-4}$alkyl, - C(O)-$C_{1-4}$aralkyl, -C(O)O-$C_{1-4}$alkyl, -C(O)O-$C_{1-4}$aralkyl, $C_{1-4}$alkyl-C(O)O-$C_{1-4}$alkyl, $C_{1-4}$alkyl-S-$C_{1-4}$alkyl, -C(O)-N($R^LR^M$), - $C_{1-4}$alkyl-C(O)-N($R^LR^M$), -$NR^L$-C(O)-$C_{1-4}$alkyl, $SO_2$-$C_{1-4}$alkyl, $SO_2$-aryl, -$SO_2$- N($R^LR^M$), $C_{1-4}$alkyl- $SO_2$- N($R^LR^M$), Fluor substituiertes $C_{1-4}$alkyl, Hydroxy substituiertes $C_{1-4}$alkyl, Carboxy substituiertes $C_{1-4}$alkyl, $C_{1-4}$alkoxy, Fluor substituiertes $C_{1-4}$alkoxy, Nitro, Cyan, Amino, $C_{1-4}$alkylamino, di($C_{1-4}$alkyl)amino, Phenyl und Heteroaryl;
wobei das Phenyl oder Heteroaryl optional durch einen oder mehrere Substituenten substituiert wird, die unabhängig aus der Gruppe bestehend aus Halogen, Hydroxy, Oxo, Carboxy, C(O)O-$C_{1-4}$alkyl, C(O)-$C_{1-4}$alkyl, $C_{1-4}$alkyl, Fluor substituiertem $C_{1-4}$alkyl, $C_{1-4}$alkoxy, Nitro, Cyan, Amino, $C_{1-4}$alkylamino und di($C_{1-4}$alkyl)amino ausgewählt werden;
wobei jedes $R^L$ und $R^M$ unabhängig aus der Gruppe bestehend aus Wasserstoff und $C_{1-4}$alkyl ausgewählt wird;
a eine Ganzzahl von 0 bis 3 ist;
jedes $R^{10}$ unabhängig aus der Gruppe bestehend aus Hydroxy, Halogen, $C_{1-4}$alkyl, $C_{1-4}$alkoxy, Halogen substituiertem $C_{1-4}$alkyl, Halogen substituiertem $C_{1-4}$alkoxy, - C(O)-$NR^VR^W$, -$SO_2$-$NR^VR^W$, -C(O)-$C_{1-4}$alkyl und -$SO_2$- $C_{1-4}$alkyl ausgewählt wird;
wobei jedes $R^V$ und $R^W$ unabhängig aus der Gruppe bestehend aus Wasserstoff und $C_{1-4}$alkyl ausgewählt werden; alternativ werden $R^V$ und $R^W$ zusammen mit dem N-Atom, mit dem sie verbunden sind, genommen, um eine gesättigte, teilweise ungesättigte oder aromatische Ringstruktur mit 5 bis 6 Gliedern zu bilden;
vorausgesetzt, dass die Halogen am Halogen substituierten $C_{1-4}$alkyl oder Halogen substituierten $C_{1-4}$alkoxy aus der Gruppe bestehend aus Chlor oder Fluor oder einem pharmazeutisch verträglichen Salz dieser ausgewählt werden.

## Revendications

**1.** Un composé représenté par la formule générale (III)

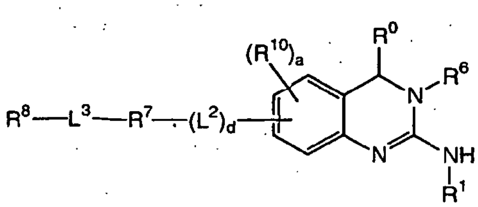
(III).

dans lequel

$R^0$ est choisi dans le groupe constitué par hydrogène, méthyle et $CF_3$ ;
$R^1$ est choisi dans le groupe constitué par hydrogène, hydroxy, méthyle, éthyle, trifluorométhyle, 2,2,2-trifluoréthyle, méthoxy, éthoxy et méthyle-carbonyle ;
$R^6$ est choisi dans le groupe constitué par $C_{1-6}$-alkyle et $C_{1-8}$-alkyle substitué par hydroxy ;
d désigne un entier de 0 à 1 ;
$L^2$ est choisi dans le groupe constitué par -O-, 5 -$S(O)_{0-2}$- et -$NR^Q$- ;
dans lequel $R^Q$ est choisi dans le groupe constitué par hydrogène et $C_{1-4}$-alkyle ;
$R^7$ est choisi dans le groupe constitué par cycloalkyle, cycloalkyle-$C_{1-4}$-alkyle, aryle, $C_{1-4}$-alkyle-aryle, aryle-$C_{1-4}$-alkyle, carbocyclyle partiellement insaturé, carbocyclyle-$C_{1-4}$-alkyle partiellement insaturé, hétéroaryle, hétéroaryle-$C_{1-4}$-alkyle, hétérocycloalkyle et hétérocycloalkyle-$C_{1-4}$-alkyle ;
dans lequel l'aryle, le cycloalkyle, le carbocyclyle partiellement insaturé, le groupe hétéroaryle ou hétérocycloalkyle, que ce soit seul ou en tant que partie d'un groupe substituant, est substitué facultativement par un ou plusieurs substituant(s) choisi(s) indépendamment dans le groupe constitué par $C_{1-4}$-alkyle, $C_{1-4}$-alkoxy, $C_{1-4}$-alkyle substitué par halogène, $C_{1-4}$-alkoxy substitué par halogène, $C_{1-4}$-alkyle substitué par hydroxy, hydroxy, carboxy, cyano, nitro, amino, $C_{1-4}$-alkylamino, di($C_{1-4}$-alkyle)amino, 5-tétrazolyle et 1-(1,4-dihydro-tétrazol-5-one) ;

$L^3$ est choisi dans le groupe constitué par -O-, -S-, -C(O)-, -C(S)-, -C(O)O-, -OC(O)-, - $NR^A$-, -N(CN), -$NR^A$-C(O)-, -C(O)-$NR^A$-, -$NR^A$-C(S)-, -C(S)-, $NR^A$-, -$NR^A$-$SO_2$-, -$SO_2$-$NR^A$-, - $NR^A$-SO-, -SO-$NR^A$, -$NR^A$-C(O)O-, -OC(O)-$NR^A$-, -O-$SO_2$-$NR^A$-, -$NR^A$-$SO_2$-O-, -$NR^A$-C(O)-$NR^B$-, -$NR^A$-C(S)-$NR^B$- et -$NR^A$-$SO_2$-$NR^B$- ;
dans lequel chaque $R^A$ et $R^B$ est choisi indépendamment dans le groupe constitué par hydrogène, $C_{1-8}$-alkyle, $C_{1-4}$-alkyle substitué par hydroxy, $C_{1-4}$-alkyle substitué par $C_{1-4}$aralkyloxy, cycloalkyle, aryle, hétéroaryle, hétérocycloalkyle, cycloalkyle- $C_{1-4}$-alkyle-, $C_{1-4}$-aralkyle, hétéroaryle- $C_{1-4}$-alkyle-, hétérocycloalkyle- $C_{1-4}$-alkyle- et spiro-hétérocyclyle ;
dans lequel le cycloalkyle, l'aryle, l'hétéroaryle, l'hétérocycloalkyle ou le spiro-hétérocyclyle, que ce soit seul ou en tant que partie d'un groupe substituant, est substitué facultativement par un ou plusieurs substituant(s) choisi(s) indépendamment dans le groupe constitué par halogène, hydroxy, oxo, carboxy, $C_{1-4}$-alkyle, $C_{1-4}$-alkoxy, $C_{1-4}$-alkoxy-carbonyle, nitro, cyano, amino, $C_{1-4}$-alkylamino, di($C_{1-4}$-alkyle)amino, -$SO_2$-N($R^CR^D$), 5-tétrazolyle et 1 -(1,4-dihydro-tétrazol-5-one)
dans lequel chaque $R^C$ et $R^D$ est choisi indépendamment dans le groupe constitué par hydrogène et $C_{1-4}$-alkyle ;
$R^8$ est choisi dans le groupe constitué par $C_{1-10}$-alkyle, cycloalkyle, aryle, biphényle, carbocyclyle partiellement insaturé, hétéroaryle, hétérocycloalkyle, cycloalkyle-$C_{1-4}$-alkyle-, $C_{1-4}$-aralkyle, carbocyclyle- $C_{1-4}$-alkyle- partiellement insaturé, hétéroaryle-$C_{1-4}$-alkyle-, hétérocycloalkyle- $C_{1-4}$-alkyle- et spiro-hétérocyclyle ;
dans lequel le $C_{1-10}$alkyle, le cycloalkyle, l'aryle, le carbocyclyle partiellement insaturé, l'hétéroaryle, l'hétérocycloalkyle ou le spiro-hétérocyclyle, que ce soit seul ou en tant que partie d'un groupe substituant, est substitué facultativement par un ou plusieurs substituant(s) choisi(s) indépendamment dans le groupe constitué par halogène, hydroxy, oxo, carboxy, -C(O)-$C_{1-4}$alkyle, -C(O)- $C_{1-4}$aralkyle, -C(O)O-$C_{1-4}$alkyle, -C(O)O-$C_{1-4}$aralkyle, - $C_{1-4}$alkyle-C(O)O-$C_{1-4}$-alkyle, - $C_{1-4}$alkyle-S- $C_{1-4}$alkyle, -C(O)-N($R^LR^M$), - $C_{1-4}$alkyle-C(O)-N($R^LR^M$), -$NR^L$-C(O)- $C_{1-4}$alkyle, -$SO_2$- $C_{1-4}$alkyle, -$SO_2$-aryle, -$SO_2$-N($R^LR^M$), - $C_{1-4}$alkyle-$SO_2$-N($R^LR^M$), $C_{1-4}$alkyle, $C_{1-4}$alkyle substitué par fluor, $C_{1-4}$alkyle substitué par hydroxy, $C_{1-4}$alkyle substitué par carboxy, $C_{1-4}$alkoxy, $C_{1-4}$alkoxy substitué par fluor, nitro, cyano, amino, $C_{1-4}$alkylamino, di($C_{1-4}$alkyl)amino, phényle et hétéroaryle ;
dans lequel le phényle ou l'hétéroaryle est substitué facultativement par un ou plusieurs substituant(s) choisi(s) indépendamment dans le groupe constitué par halogène, hydroxy, oxo, carboxy, C(O)O-$C_{1-4}$alkyle, C(O)-$C_{1-4}$alkyle, $C_{1-4}$alkyle, $C_{1-4}$alkyle substitué par fluor, $C_{1-4}$alkoxy, nitro, cyano, amino, $C_{1-4}$alkylamino et di($C_{1-4}$alkyle)amino ;
dans lequel chaque $R^L$ et $R^M$ est choisi indépendamment dans le groupe constitué par hydrogène, $C_{1-4}$alkyle et cycloalkyle ;
a désigne un entier compris entre 0 et 3 ;
chaque $R^{10}$ est choisi indépendamment dans le groupe constitué par hydroxy, halogène, $C_{1-4}$alkyle, $C_{1-4}$alkoxy, $C_{1-4}$alkyle substitué par halogène, $C_{1-4}$alkoxy substitué par halogène, -C(O)-$NR^VR^W$, -$SO_2$-$NR^VR^W$, -C(O)-$C_{1-4}$alkyle et -$SO_2$-$C_{1-4}$alkyle ;
dans lequel chaque $R^V$ et $R^W$ est choisi indépendamment dans le groupe constitué par hydrogène et $C_{1-4}$alkyle ;
en variante, $R^V$ et $R^W$ sont fusionnés à l'atome N auquel ils sont rattachés pour former une structure à noyau saturé, partiellement insaturé ou aromatique à 5 ou 6 éléments ;
pour autant que les halogènes sur le $C_{1-4}$alkyle substitué par halogène ou le $C_{1-4}$alkoxy substitué par halogène sont choisis dans le groupe constitué par clore et fluor ;

ou un sel pharmaceutiquement acceptable de ces composés.

**2.** Composé selon la Revendication 1 dans lequel
$R^0$ est choisi dans le groupe constitué par hydrogène, méthyle et $CF_3$ ;
$R^1$ est choisi dans le groupe constitué par hydrogène, hydroxy, méthyle, trifluorométhyle, méthoxy, éthoxy et méthylecarbonyle ;
$R^6$ est choisi dans le groupe constitué par $C_{1-6}$alkyle et $C_{1-6}$alkyle substitué par hydroxy ;
d désigne un entier sélectionné de 0 à 1 ;
$L^2$ est choisi dans le groupe constitué par -O-, $S(O)_{0-2}$ et -NH- ;
$R^7$ est choisi dans le groupe constitué par cycloalkyle, cycloalkyl-$C_{1-4}$alkyle, aryle, $C_{1-4}$alkyle-aryle, aryle-$C_{1-4}$alkyle, hétéroaryle, hétéroaryle-$C_{1-4}$alkyle, hétérocycloalkyle et hétérocycloalkyle-$C_{1-4}$alkyle ;
dans lequel l'aryle, le cycloalkyle, le carbocyclyle partiellement insaturé, le groupe hétéroaryle ou hétérocycloalkyle, que ce soit seul ou en tant que partie d'un groupe substituant, est substitué facultativement par un à deux substituant(s) choisi(s) indépendamment dans le groupe constitué par $C_{1-5}$alkyle, $C_{1-4}$alkoxy, $C_{1-4}$alkyle substitué par halogène, $C_{1-4}$alkoxy substitué par halogène, $C_{1-4}$alkyle substitué par hydroxy, hydroxy, carboxy, cyano, nitro, amino, $C_{1-4}$alkylamino et di($C_{1-4}$ -alkyle)amino ;
$L^3$ est choisi dans le groupe constitué par -C(O)-, -C(O)O-, -OC(O)-, -$NR^4$-, -N(CN)-, -$NR^A$-C(O)-, -C(O)-$NR^A$,

$-NR^A-SO_2-$ $-SO_2-NR^A$, $NR^A-C(O)O-$ et $-OC(O)-NR^4$,
dans lequel $R^A$ est choisi dans le groupe constitué par hydrogène, $C_{1-4}$alkyle, $C_{1-4}$alkyle substitué par hydroxy, $C_{1-4}$alkyle substitué par $C_{1-4}$aralkyloxy, cycloalkyle, aryle, hétéroaryle, hétérocycloalkyle, cycloalkyle-$C_{1-4}$alkyle, $C_{1-4}$aralkyle, hétéroaryle-$C_{1-4}$alkyle et hétérocycloalkyle-$C_{1-4}$alkyle- ;
dans lequel le cycloalkyle, l'aryle, l'hétéroaryle ou l'hétérocycloalkyle, que ce soit seul ou en tant que partie d'un groupe substituant, est substitué facultativement par un ou deux substituant(s) choisi(s) indépendamment dans le groupe constitué par halogène, hydroxy, oxo, carboxy, $C_{1-4}$alkyle, $C_{1-4}$alkoxy-carbonyle, nitro, cyano, amino, $C_{1-4}$alkylamino et di($C_{1-4}$alkyle)amino ;
$R^8$ est choisi dans le groupe constitué par $C_{1-6}$alkyle, cycloalkyle, aryle, hétéroaryle, hétérocycloalkyle, cycloalkyl-$C_{1-4}$alkyle, $C_{1-4}$aralkyle, hétéroaryle-$C_{1-4}$alkyle et hétérocycloalkyle-$C_{1-4}$alkyle ;
dans lequel le $C_{1-4}$alkyle, le cycloalkyle, l'aryle, l'hétéroaryle ou l'hétérocycloalkyle, que ce soit seul ou en tant que partie d'un groupe substituant, est substitué facultativement par un ou deux substituant(s) choisi(s) indépendamment dans le groupe constitué par halogène, hydroxy, carboxy,
$-C(O)-C_{1-4}$alkyle, $-C(O)O-C_{1-2}$aralkyle, $-C(O)O-C_{1-4}$alkyle, $-C(O)O-C_{1-4}$aralkyle, $-C(O)-N(R^LR^M)$, $-NR^L-C(O)-C_{1-4}$alkyle, $-SO_2-C_{1-4}$alkyl-, $-SO_2$-aryle-, $-SO_2-N(R^LR^M)$, $C_{1-4}$alkyle, $C_{1-4}$alkyle substitué par le fluor, $C_{1-4}$alkyle substitué par hydroxy, $C_{1-4}$alkyle substitué par carboxy, $C_{1-4}$alkoxy, $C_{1-4}$alkoxy substitué par fluor, nitro, cyano, amino, $C_{1-4}$alkylamino, di($C_{1-4}$alkyle)amino, phényle et hétéroaryle ;
dans lequel le phényle ou l'hétéroaryle est substitué facultativement par un ou plusieurs substituant(s) choisi(s) indépendamment dans le groupe constitué par halogène, hydroxy, carboxy, $C_{1-4}$alkyle, $C_{1-4}$alkyle substitué par fluor, $C_{1-4}$alkoxy, nitro, cyano, amino, $C_{1-4}$alkylamino et di($C_{1-4}$alkyle)amino ;
dans lequel chaque $R^L$ et $R^M$ est choisi indépendamment dans le groupe constitué par hydrogène, $C_{1-4}$alkyle et $C_{5-8}$cycloalkyle ;
a désigne un entier de 0 à 1 ;
$R^{10}$ est choisi dans le groupe constitué par hydroxy, halogène, $C_{1-4}$alkyle, $C_{1-4}$alkoxy, $C_{1-4}$alkyle substitué par halogène et $C_{1-4}$alkoxy substitué par halogène ;
pour autant que les halogènes sur le $C_{1-4}$alkyle substitué par halogène ou le $C_{1-4}$alkoxy substitué par halogène sont choisis dans le groupe constitué par chlore et fluor ;
ou un sel pharmaceutiquement acceptable de ces composés.

**3.** Composé selon la Revendication 2 dans lequel
$R^0$ désigne un atome d'hydrogène ;
$R^1$ désigne un atome d'hydrogène
$R^6$ est choisi dans le groupe constitué par $C_{1-4}$alkyle et $C_{1-4}$alkyle substitué par hydroxy
d est un entier sélectionné de 0 à 1 ;
$L^2$ est choisi dans le groupe constitué par -O-. -S-, -SO- et -SO- ;
$R^7$ est choisi dans le groupe constitué par aryle, $C_{1-4}$alkyle-aryle et aryle-$C_{1-4}$alkyle;
$L^3$ est choisi dans le groupe constitué par -NH-, -N(CN)-, $-N(C_{1-4}$alkyle), -NH-C(O)-, - C(O)-NH-, $NH-SO_2-$, N($C_{1-4}$alkyle)-C(O)O- et N(cycloalkyle)-C(O)O- ;
$R^8$ est choisi dans le groupe constitué par $C_{1-4}$alkyle, cycloalkyle, cycloalkyl-$C_{1-4}$alkyle, aryle, $C_{1-4}$aralkyle, hétéroaryle et hétérocycloalkyle ;
dans lequel l'aryle ou l'hétéroaryle, que ce soit seul ou en tant que faisant partie d'un groupe substituant, est substitué facultativement par un ou trois substituant(s) choisi(s) indépendamment dans le groupe constitué par halogène, hydroxy, carboxy, $C_{1-4}$alkyle, $C_{1-4}$alkoxy, $C_{1-2}$alkyle substitué par fluor, $C_{1-4}$alkoxy substitué par fluor, cyane, amino, $C_{1-4}$alkylamino, di($C_{1-4}$alkyle)amino, $-C(O)O-C_{1-4}$alkyle, $-C(O)-N(R^LR^M)-SO_2-C_{1-4}$alkyle, $-SO_2$-aryle, $-NH-C(O)-C_{1-4}$alkyle, phényle et hétéroaryle ;
dans lequel le phényle ou l'hétéroaryle est substitué facultativement par un substituant choisi dans le groupe constitué par hydrogène, $C_{1-4}$alkyle et $C_{5-6}$cyloalkyle ;
dans lequel chaque $R^L$ et $R^M$ est choisi indépendamment dans le groupe constitué par hydrogène, $C_{1-4}$alkyle et $C_{5-6}$cycloalkyle ;
a désigne 0 ;
ou un sel pharmaceutiquement acceptable de ces composés.

**4.** Composé selon la revendication 3 dans lequel
$R^0$ est un atome d'hydrogène ;
$R^1$ est un atome d'hydrogène ;
$R^6$ est sélectionné dans le groupe constitué par n-propyle, 4-hydroxy-n-butyle et 5-hydroxy-n-pentyle ;
d désigne un entier de 0 à 1 ;
$L^2$ est choisi dans le groupe constitué par -O-, -S- et -SO ;

$R^7$ est choisi dans le groupe constitué par phényle, -$CH_2$-phényle-, phényle-3-$CH_2$ et phényle-2-$CH_2$-$CH_2$- ;
$L^3$ est choisi dans le groupe constitué par -NH-, -N(CN)-, -N($CH_3$)-, NH-C(O)-, -C(O)-NH, -NH-$SO_2$- et N(cyclohexyle)-C(O)O- ;
dans lequel I groupe $L^3$ est rattaché à la position 3 du groupe $R^7$ ;
$R^8$ est choisi dans le groupe constitué par méthyle, isopropyle, n-butyle, cyclohexyle, cyclohexyle-méthyle, phényle, phényle-éthyle, phényle-n-propyle, 3-(N-méthyle-N-cyclohexyle-amino-carbonyle)-n-propyle, 3-brom-phényle, 3-méthoxy-phényle, 4-méthoxyphényle, 3,4-diméthoxy-phényle, 3-trifluorométhoxy-phényle, 4-trifluoreméthoxy-phényle, 3-cyano-phényle, 2-(méthyle-sulfonyle)-phényle, 4-(méthyle-carbonyle-amino)-phényle, 5-carboxy-2-méthoxy-phényle, benzyle, 3-hydroxy-benzyle, 4-méthyle-benzyle, 2-méthoxybenzyle, 4-méthoxy-benzyle, 2,6-diméthoxy-benzyle, 2,4,6-triméthyle-benzyle, 1-naphthyle, 2-naphthyle, 1-naphthtyle-méthyle, 1-(5-diméthylamino(-naphthyle, 4-biphényle, 2-thiényle, 3-thiényle, 4-((3,5,-diméthyle-isoxazolyle), 3-benzothiényle, 4-benzo[2,3,1]thiadiazolyle, 2-(5-(2-pyridyle)-thiényle), 2-(5-(3-)2-méthyle-thiazolyle)thiényle, 2-(5-(3-(5-trifluorométhyle)isoxazolyle)-thiényle), 6-(2,3-dihydro-benzo[1,4]dioxanyle), 3-(2-méthoxycarbonyle)-thiényle, 2-(5-(5-isoxazolyle)-thiényle), 2-(5-brom-thiényle) et 2,(4-phénylesulfonyle)-thiényle ;
a désigne 0 ;
ou un sel pharmaceutiquement acceptable de ces composés.

**5.** Composé selon la Revendication 4 dans lequel
$R^0$ désigne un atome d'hydrogène ;
$R^1$ désigne un atome d'hydrogène ;
$R^6$ est choisi dans le groupe constitué par n-propyle, 4-hydroxy-n-butyle et 5-hydroxy-n-pentyle ;
d désigne un entier de 0 à 1 ;
$L^2$ est choisi dans le groupe constitué par -O- et -S- ;
$R^7$ est choisi dans le groupe constitué par -phényle, -phényle-3-$CH_2$ et phényle-2-$CH_2$-$CH_2$;
$L^3$ est choisi dans le groupe constitué par -NH-, -N(CN)-, NH-C(O)-, NH-C(O)O- et N(cyclohexyle)-C(O)O- et -NH-$SO_2$- ; dans lequel le groupe $L^3$ est rattaché au phényle à la position 3 du groupe $R^7$ ;
$R^8$ est sélectionné dans le groupe constitué par 3-(N-méthyle-N-cyclohexyle-amino-carbonyle)-n-propyle, cyclohexyle, cyclohexyle-méthyle-, 2-trifluorométhyle-phényle, 3-trifluorométhoxyphényle, 3-cyanophényle, 3-méthoxyphényle, 4-méthoxyphényle, 2-méthoxy-4-méthyle-phényle, 2,4,6-triméthyle-phényle, benzyle, 2-méthoxybenzyle, 4-méthoxybenzyle, 2,4,6-triméthyle-benzyle, phényl-éthyle-, 2-thiényle, 3-thiényle, 2-(5-bromothiényle) et 3-benzothiényle ;
a désigne 0 ;
ou un sel pharmaceutiquement acceptable de ces composés.

**6.** Composé de la Revendication 5 dans lequel
$R^0$ désigne un atome d'hydrogène ;
$R^1$ désigne un atome d'hydrogène
$R^6$ est choisi dans le groupe constitué par n-propyle et 4-hydroxy-n-butyle ;
d désigne 1 ;
$L^2$ est choisi dans le groupe constitué par -O- et -S- ;
$R^7$ est choisi dans le groupe constitué par -phényle et -phényle-3-$CH_2$- ;
$L^3$ est choisi dans le groupe constitué par -NH-, -NH-C(O)- et -NH-$SO_2$- ;
dans lequel le groupe $L^3$ est rattaché au phényle à la position 3 du groupe $R^7$;
$R^8$ est choisi dans le groupe constitué par 3-(N-méthyle-N-cyclohexyle-amino-carbonyle)-n-propyle, phényle, 2-méthoxy-4-méthyle-phényle, 2,4,6-triméthyle-phényle, benzyle, 2-méthoxybenzyle, 4-méthoxybenzyle, 2,4,6-triméthyle-benzyle et 3-benzothiényle ;
a désigne 0 ;
ou un sel pharmaceutiquement acceptable de ces composés.

**7.** Composé selon la Revendication 6, dans lequel
$R^0$ désigne un atome d'hydrogène ; $R^1$ désigne un atome d'hydrogène ; R6 désigne un radical n-propyle ; d désigne 1 ; $L^2$ est choisi dans le groupe constitué par -O- et -S-; $R^7$ désigne un radical -phényle-; $L^3$ désigne un radical -NH-$SO_2$- ; dans lequel $L^3$ est rattaché au phényle à la position 3 du groupe $R^7$; $R^8$ désigne un radical 2,4,6-triméthylphényle ; a désigne 0 ; ou un sel pharmaceutiquement acceptable de ces composés.

**8.** Composé selon la Revendication 4 dans lequel
$R^0$ désigne un atome d'hydrogène ; $R^1$ désigne un atome d'hydrogène ; $R^6$ désigne un radical n-propyle ; d désigne 1 ; $L^2$ désigne -O- ; $R^7$ désigne un radical phényle ; $L^3$ est choisi dans le groupe constitué par -NH-C(O)O-, -N(cyclohexyle)-C(O)O-, -NH-,-N(CN)- et - $NH\text{-}SO_2$; dans lequel $L^3$ est rattaché au phényle à la position 3 du groupe $R^7$; $R^8$ est choisi dans le groupe constitué par 3-(N-méthyle-N-cyclohexyle-amino-carbonyle)-n-propyle, cyclohexyle, cyclohexyle-méthyle-, phényle et benzyle ; a désigne 0 ; ou un sel pharmaceutiquement acceptable de ces composés.

**9.** Composition pharmaceutique contenant un vecteur pharmaceutiquement acceptable et un composé de la Revendication 1.

**10.** Composition pharmaceutique réalisée par le mélange d'un composé de la Revendication 1 et d'un vecteur pharmaceutiquement acceptable.

**11.** Procédé de préparation d'une composition pharmaceutique consistant en le mélange d'un composant de la Revendication 1 et d'un vecteur pharmaceutiquement acceptable.

**12.** Composé selon la Revendication 1 destiné au traitement d'un trouble médié par la β-secrétase par l'administration à un sujet en ayant besoin d'une quantité thérapeutiquement efficace du composé.

**13.** Composé selon la Revendication 12, pour lequel le trouble médié par la β-secrétase est choisi dans le groupe constitué par la maladie d'Alzheimer (MA), la déficience cognitive légère, l'état confusionnel associé à la démence sénile, la démence, la démence à corps de Lewy, le syndrome de Down, la démence associée à la maladie de Parkinson et la démence associée aux bêta-amyloïdes.

**14.** Composition selon la Revendication 9 destinée au traitement d'un trouble médié par la β-secrétase par l'administration à un sujet en ayant besoin d'une quantité thérapeutiquement efficace de la composition.

**15.** Composé selon la Revendication 1 destiné au traitement d'une condition choisie dans le groupe constitué par la maladie d'Alzheimer (MA) , la déficience cognitive légère, l'état confusionnel associé à la démence sénile, la démence, la démence à corps de Lewy, le syndrome de Down, la démence associée à la maladie de Parkinson et la démence associée aux bêta-amyloïdes par l'administration à un sujet en ayant besoin d'une quantité thérapeutiquement efficace du composé.

**16.** Utilisation d'un composé selon la Revendication 1 en vue de la préparation d'un médicament pour le traitement : (a) de la maladie d'Alzheimer (AD), (b) de la déficience cognitive légère, (c) de l'état confusionnel associé à la démence sénile, (d) de la démence, (e) de la démence à corps de Lewy, (f) du syndrome de Down, (g) de la démence associée à la maladie de Parkinson ou (h) de la démence associée aux bêta-amyloïdes chez un sujet en ayant besoin.

**17.** Composé représenté par la formule générale (CIII)

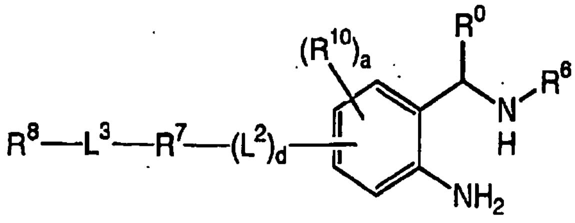

(CIII)

dans lequel

$R^0$ est choisi dans le groupe constitué par hydrogène, méthyle et $CF_3$ ;
R6 est choisi dans le groupe constitué par $C_{1-6}$alkyle et hydroxy substitué par $C_{1-8}$alkyle ;
d désigne un entier de 0 à 1 ;
$L^2$ est choisi dans le groupe constitué par -O-, $-S(O)_{0-2}-$ et $-NR^0-$ ;
dans lequel $R^0$ est choisi dans le groupe constitué par hydrogène et $C_{1-4}$alkyle ;
$R^7$ est choisi dans le groupe constitué par cycloalkyle, cycloalkyle-$C_{1-4}$ alkyle, aryle, $_{c1-4}$alkyle-aryle, aryle-$C_{1-4}$alkyle, carbocyclyle partiellement insaturé, carbocyclyle-$_{C1-4}$alkyle partiellement insaturé, hétéroaryle, hétéroaryle-$C_{1-4}$alkyle, hétérocycloalkyle et hétérocycloalkyle-$_{C1-4}$alkyle ;

dans lequel l'aryle, le cycloalkyle, le carbocyclyle partiellement insaturé, l'hétéroaryle ou le groupe hétérocycloalkyle, que ce soit seul ou en tant que partie d'un groupe substituant, est substitué facultativement par un ou plusieurs substituant(s) choisi(s) indépendamment dans le groupe constitué par $C_{1-4}$alkyle, $C_{1-4}$alkoxy, $C_{1-4}$alkyle substitué par halogène, $C_{1-4}$alkoxy substitué par halogène, $C_{1-4}$alkyle substitué par hydroxy, hydroxy, carboxy, cyano, nitro, amino, $C_{1-4}$alkylamino, di(C-$_{1-4}$alkyle)amino, 5-tétrazolyle et 1-(1,4-dihydro-tétrazol-5-one) ;

$L^3$ est choisi dans le groupe constitué par -O-, -S-, -C(O)-, -C(S)-, -C(O)O-, -OC(O)-, - $NR^A$-, -N(CN)-, -$NR^A$-C(O)-, -C(O)-$NR^A$-, -$NR^A$-C(S)-, -C(S)-, $NR^A$-, -$NR^A$-$SO_2$-, -$SO_2$-$NR^A$-, - $NR^A$-SO-, -SO-$NR^A$, -$NR^A$-C(O)O-, -OC(O)-$NR^A$-, -O-$SO_2$-$NR^A$-, -$NR^A$-$SO_2$-O-, -$NR^A$-C(O)-$NR^B$-, -$NR^A$-C(S)-$NR^B$- et -$NR^A$-$SO_2$-$NR^B$-;

dans lequel chaque $R^A$ et $R^B$ est choisi indépendamment dans le groupe constitué par hydrogène, $C_{1-8}$alkyle, $C_{1-4}$alkyle substitué par hydroxy, $C_{1-4}$-alkyle substitué par $C_{1-4}$aralkyloxy, cycloalkyle, aryle, hétéroaryle, hétérocycloalkyle, cycloalkyle-$C_{1-4}$alkyle-, $C_{1-4}$aralkyle, hétéroaryle-$C_{1-4}$alkyle-, hétérocycloalkyle-$C_{1-4}$alkyle- et spiro-hétérocyclyle ;

dans lequel le cycloalkyle, aryle, hétéroaryle, hétérocycloalkyle ou spiro-hétérocyclyle, que ce soit seul ou en tant que partie d'un groupe substituant, est substitué facultativement par un ou plusieurs substituant(s) choisi(s) indépendamment dans le groupe constitué par halogène, hydroxy, oxo, carboxy, $C_{1-4}$alkyle, $C_{1-4}$alkoxy, $C_{1-4}$alkoxy-carbonyle, nitro, cyano, amino, $C_{1-4}$alkylamino, di($C_{1-4}$alkyle)amino,-$SO_2$-N($R^CR^D$), 5-tétrazolyle et 1 - (1,4-dihydro-tétrazol-5-one)

dans lequel chaque $R^C$ et $R^D$ est choisi indépendamment dans le groupe constitué par hydrogène et $C_{1-4}$alkyle ;

$R^8$ est choisi dans le groupe constitué par $C_{1-10}$alkyle, cycloalkyle, aryle, biphényle, carbocyclyle partiellement insaturé, hétéroaryle, hétérocycloalkyle, cycloalkyle-$C_{1-4}$alkyle-, $C_{1-4}$aralkyle, carbocyclyle-$C_{1-4}$alkyle- partiellement insaturé, hétéroaryle- $C_{1-4}$alkyle-, hétérocycloalkyle- $C_{1-4}$alkyle- et spiro-hétérocyclyle ;

dans lequel $C_{1-10}$alkyle, cycloalkyle, aryle, carbocyclyle partiellement insaturé, hétéroaryle, hétérocycloalkyle ou spiro-hétérocyclyle, que ce soit seul ou en tant que partie d'un groupe substituant, est substitué facultativement par un ou plusieurs substituant(s) choisi(s) indépendamment dans le groupe constitué par halogène, hydroxy, oxo, carboxy, - C(O)-$C_{1-4}$alkyle, -C(O)-$C_{1-4}$aralkyle, -C(O)O-$C_{1-4}$alkyle, -C(O)O-$C_{1-4}$aralkyl, -$C_{1-4}$alkyle-C(O)O$C_{1-4}$alkyle, -$C_{1-4}$alkyle-S- $C_{1-4}$alkyle, -C(O)-N($R^LR^M$), -$C_{1-4}$alkyle-C(O)-N($R^LR^M$), -$NR^L$-C(O)-$C_{1-4}$alkyle, -$SO_2$- $C_{1-4}$alkyle, -$SO_2$-aryle, -$SO_2$-N($R^LR^M$), -$C_{1-4}$alkyle-$SO_2$-N($R^LR^M$), $C_{1-4}$alkyle, $C_{1-4}$alkyle substitué par fluor, $C_{1-4}$alkyle substitué par hydroxy, $C_{1-4}$alkyle substitué par carboxy, $C_{1-4}$alkoxy, $C_{1-4}$alkoxy substitué par fluor, nitro, cyano, amino, $C_{1-4}$alkylamino, di($C_{1-4}$alkyle)amino, phényle et hétéroaryle ;

dans lequel le phényle ou l'hétéroaryle est substitué facultativement par un ou plusieurs substituant(s) choisi(s) indépendamment dans le groupe constitué par halogène, hydroxy, oxo, carboxy, C(O)O-$C_{1-4}$alkyle, C(O)-$C_{1-4}$alkyle, $C_{1-4}$alkyle, $C_{1-4}$alkyle substitué par fluor, $C_{1-4}$alkoxy, nitro, cyano, amino, $C_{1-4}$alkylamino et di($C_{1-4}$alkyle)amino ;

dans lequel chaque $R^L$ et $R^M$ est choisi indépendamment dans le groupe constitué par hydrogène et $C_{1-4}$alkyle ;

a désigne un nombre entier compris entre 0 et 3 ;

chaque $R^{10}$ est choisi indépendamment dans le groupe constitué par hydroxy, halogène, $C_{1-4}$alkyle, $C_{1-4}$alkoxy, $C_{1-4}$alkyle substitué par halogène, $C_{1-4}$alkoxy substitué par halogène, -C(O)-$NR^VR^W$, -$SO_2$-$NR^VR^W$, -C(O)-$C_{1-4}$alkyle et -$SO_2$- $C_{1-4}$alkyle;

dans lequel chaque $R^V$ et $R^W$ est choisi indépendamment dans le groupe constitué par hydrogène et $C_{1-4}$alkyle ;

en alternative, $R^V$ et $R^W$ sont fusionnés à l'atome N auquel ils sont rattachés pour former une structure à noyau saturé, partiellement insaturé ou aromatique à 5 ou 6 éléments ;

pour autant que les halogènes sur $C_{1-4}$alkyle substitué par halogène ou $C_{1-4}$alkoxy substitué par halogène sont choisis dans le groupe constitué par chlore et fluor ;

ou un sel pharmaceutiquement acceptable de ces composés.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 200422523 A

### Non-patent literature cited in the description


- **T.W. Greene ; P.G.M. Wuts.** Protective Groups in Organic Synthesis. John Wiley & Sons, 1999
- **T.W. Greene ; P.G.M. Wuts.** Protective Groups in Organic Synthesis. John Wiley & Sons, 1991
- Protective Groups in Organic Chemistry. Plenum Press, 1973
- Design of Prodrugs. Elsevier, 1985
- **MIyaura, N. ; Suzuki, A.** *Chem. Rev.,* 1995, vol. 95, 2457
- **Suzuki, A.** *J. Organomet. Chem.,* 1999, vol. 576, 147